# EUROPÄISCHE PATENTSCHRIFT

(11) **EP 0 588 166 B1**
(45) Veröffentlichungstag und Bekanntmachung des Hinweises auf die Patenterteilung: **05.06.1996**
(21) Anmeldenummer: 93114052.9
(22) Anmeldetag: 02.09.1993
(51) Int. Cl.: C07D 401/04, C07D 498/06, A61K 31/435, C07D 471/04, C07D 209/44

(54) **Antibakterielle 7-Isoindolinyl-chinolon- und naphthyridon-Derivate**
Antibacterial 7-isoindolinyl-quinolone and naphthyridone derivatives
Dérivés antibactériens de 7-isoindolinyl-quinolone et naphthyridone

(30) Priorität: 15.09.1992 DE 4230804
(43) Veröffentlichungstag der Anmeldung: 23.03.1994
(73) Patentinhaber: BAYER AG, 51368 Leverkusen (DE)
(72) Erfinder: Philipps, Thomas, Dr., D-51065 Köln (DE); Bartel, Stephan, Dr., D-51465 Bergisch Gladbach (DE); Krebs, Andreas, Dr., D-51519 Odenthal (DE); Petersen, Uwe, Dr., D-51375 Leverkusen (DE); Schenke, Thomas, Dr., D-51469 Bergisch Gladbach (DE); Bremm, Klaus-Dieter, Dr., D-42109 Wuppertal (DE); Endermann, Rainer, Dr.r., D-42113 Wuppertal (DE); Metzger, Karl Georg, Dr., D-42115 Wuppertal (DE); Mielke, Burkhard, D-51375 Leverkusen (DE)

(56) Entgegenhaltungen:
- EP-A- 0 343 524
- EP-A- 0 343 560
- EP-A- 0 429 304
- EP-A- 0 516 861
- CHEMICAL ABSTRACTS, vol. 88, no. 5, 30. Januar 1978, Columbus, Ohio, US; abstract no. 37558f, R.S. MKRTCHYAN ET AL. 'Studies on amines and ammonium compounds. CXXXII. Intramolecular thermal cyclization of benzenesulfonic acid allyl-2,4-pentadienyl(furfuryl)amides.' Seite 480 ;

## Beschreibung

Die Erfindung betrifft neue Chinalon- und Naphthyridon-Derivate, die in 7-Stellung durch einen partiell hydrierten Isoindolinylring substituiert sind, Verfahren zu ihrer Herstellung sowie diese enthaltende antibakterielle Mittel und Futterzusatzstoffe.

Es sind bereits aus der EP-A-343 560 Chinalon- und Naphthyridon-Derivate bekannt geworden, die in 7-Stellung durch einen Isoindolinylring substituiert sind, wie z.B. 7-(2-Isoindolinyl)-1-cyclopropyl-6,8-difluor-1,4-dihydro-4-oxo-3-chinolincarbonsäure. Diese zeichnen sich jedoch nur durch eine geringe antibakterielle Wirksamkeit aus.

Weiterhin bekannt sind aus der EP-A-343 524 Chinolon- und Naphthyridoncarbonsäuren, die in 7-Stellung durch ein perhydrierten Isoindolinylring substituiert sind, wie z.B. 7-[(1-RS, 2RS, 6SR)-2-Amino-8-azabicyclo[4.3.0]non-8-yl]-1-cyclopropyl-6,8-difluor-1,4-dihydro-4-oxo-3-chinolincarbonsäure.

Aus der WO-A-9212146 sind Verbindungen bekannt geworden, die in 7- Stellung durch einen 8-Azabicyclo[4.3.0]non-3-en-8-yl-Ring substituiert sind. Diese, mit dem isomeren Isoindolinyl-Ring substituierten Chinolin- und Naphthyridoncarbonsäure-Derivate sind zwar ausgezeichnet antibakteriell wirksam. Die Verbindungen sind jedoch derart toxisch, daß die zur Verfügung stehende therapeutische Breite für ihre Anwendung als Pharmazeutikum zu gering ist.

Weiterhin bekannt ist (-)-7-[(1R,2R,6S)-2-Amino-8-azabicyclo[4.3.0]non-3-en-8-yl)-1-cyclopropyl-6,8-difluor-1,4-dihydro-4-oxo-3-chinolincarbonsäure (T. Yoshinari et al., Postervortrag auf dem Kongress: "Topoisomerases in Chemotherapy", Nagoya (Japan), November 1991).

Es wurde gefunden, daß die Verbindungen der Formel (I) 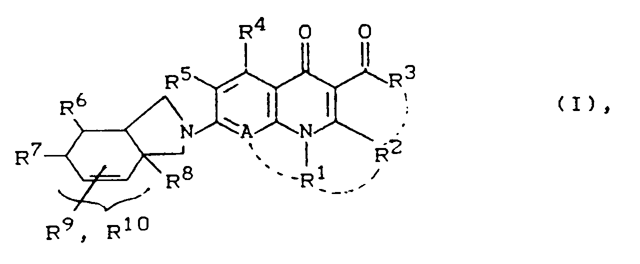
in welcher
- R¹: für gegebenenfalls durch 1-3 Fluoratome substituiertes Alkyl mit 1 bis 4 Kohlenstoffatomen, Alkenyl mit 2 bis 4 Kohlenstoffatomen, gegebenenfalls durch 1 bis 2 Fluoratome substituiertes Cycloalkyl mit 3 bis 6 Kohlenstoffatomen, 2-Hydroxyethyl, Methoxy, Amino, Methylamino, Ethylamino, Dimethylamino, gegebenenfalls durch 1 oder 2 Fluoratome substituiertes Phenyl, 3-Oxetanyl, Bicyclo[1.1.1]pentyl,
- R²: für Wasserstoff steht oder auch gemeinsam mit R¹ eine Brücke bilden kann, so daß ein 4-oder 5-gliedriger Ring entsteht,
- R³: für Hydroxy oder O-R¹¹ steht, worin
- R¹¹: für Alkyl mit 1-4 C-Atomen steht oder
- R³: gemeinsam mit R² eine Brücke bilden kann, so daß ein 5- oder 6-gliedriger Ring entsteht,
- R⁴: für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Halogen, Methyl, Ethyl oder Vinyl,
- R⁵: für Wasserstoff, Halogen oder Methyl,
- R⁶: für Wasserstoff, Alkyloxycarbonyl mit 1 bis 4 Kohlenstoffatomen, Hydroxymethyl, Kohlenstoffatomen, Hydroxymethyl, 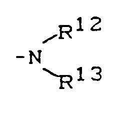 oder 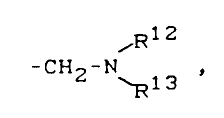 wobei
- R¹²: Wasserstoff, gegebenenfalls durch Hydroxy substituiertes Alkyl mit 1 bis 3 Kohlenstoffatomen, Alkyloxycarbonyl mit 1 bis 4 Kohlenstoffatomen im Alkylteil oder Acyl mit 1 bis 3 Kohlenstoffatomen und
- R¹³: Wasserstoff oder Methyl bedeutet,
- R⁷, R⁸, R⁹ und R¹⁰: für Wasserstoff oder Methyl steht oder R⁹ und R¹⁰ für Wasserstoff oder Methyl steht und R⁷ gemeinsam mit R⁸ eine Brücke der Struktur -O-, -CH₂- oder -CH₂-CH₂- bilden kann und
- A: für N oder C-R¹⁴ steht, worin
- R¹⁴: für Wasserstoff, Halogen, gegebenenfalls durch 1 bis 3 Fluoratome substituiertes Methyl, Ethinyl, Vinyl, Hydroxy oder Methoxy steht, oder auch gemeinsam mit R¹ eine Brücke bilden kann, so daß ein 5- oder 6-gliedriger Ring entsteht, oder auch gemeinsam mit R¹ und R² eine Brücke bilden kann, so daß zwei 5-oder 6-gliedrige Ringe entstehen,
und deren Tautomere sowie deren pharmazeutisch verwendbare Hydrate und Saureadditionssalze sowie die Alkali-, Erdalkali-, Silber- und Guanidiniumsalze der zugrundeliegenden Carbonsäuren gegenüber dem Stand der Technik eine höhere antibakterielle Wirkung insbesondere im grampositiven Bereich aufweisen.

Bevorzugt sind die Verbindungen der Formel (I), in welcher
- R¹: für Cyclopropyl, 2-Fluorcyclopropyl, Ethyl, 2-Fluorethyl, gegebenenfalls durch 1, 2 oder 3 Fluoratome substituiertes tert.-Butyl oder gegebenenfalls durch 1 oder 2 Fluoratome substituiertes Phenyl, 3-Oxetanyl, Bicyclo[1.1.1]pentyl,
- R²: für Wasserstoff steht oder auch gemeinsam mit R¹ eine Brücke der Struktur 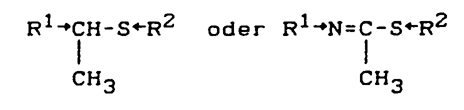 bilden kann*.
- (*:: Bilden zwei oder mehrere Reste gemeinsam eine Brücke, so wird durch Pfeile angegeben, mit welchen Zentren der allgemeinen Formel die Brückenatome verknüpft sind, Beispielsweise bedeutet 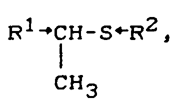 daß CH mit dem durch R¹ substituierten Zentrum der allgemeinen Formel (I) verknüpft ist und daß S mit dem durch R² substituierten Zentrum der allgemeinen Formel (I) verknüpft ist.)
- R³: für Hydroxy oder O-R¹¹ steht, worin
- R¹¹: für Alkyl mit 1-4 C-Atomen steht, oder
- R³: gemeinsam mit R² eine Brücke der Struktur 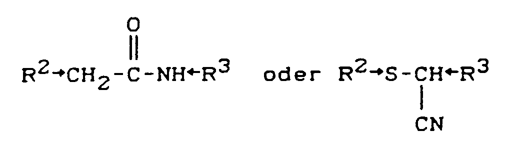 bilden kann,
- R⁴: für Wasserstoff, Fluor, Chlor, Amino, Hydroxy, Methyl oder Vinyl,
- R⁵: für Wasserstoff, Fluor, Chlor, Brom oder Methyl,
- R⁶: für Amino, Methylamino, Ethoxycarbonylamino, Aminomethyl, Ethoxycarbonylaminomethyl, Hydroxymethyl, Ethoxycarbonyl oder Wasserstoff,
- R⁷, R⁸, R⁹ und R¹⁰: für Wasserstoff oder Methyl steht oder R⁹ und R¹⁰ für Wasserstoff oder Methyl steht und R⁷ gemeinsam mit R⁸ eine Brücke der Struktur -O-, -CH₂ oder -CH₂-CH₂- bilden kann und
- A: für N oder C-R¹⁴ steht, worin
- R¹⁴: für Wasserstoff, Fluor, Chlor, Brom, gegebenenfalls durch 1 bis 3 Fluoratome substituiertes Methyl, Ethinyl, Vinyl oder Methoxy steht oder auch gemeinsam mit R¹ eine Brücke der Struktur 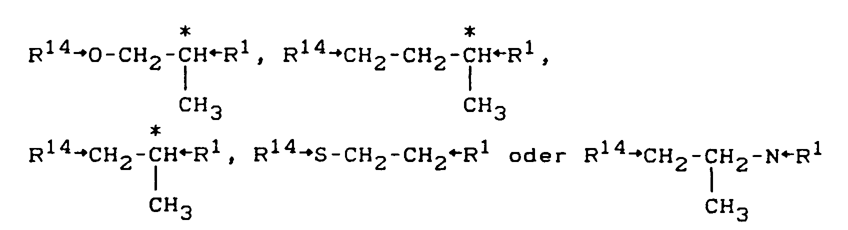 oder auch gemeinsam mit R¹ und R² eine Brücke der Struktur 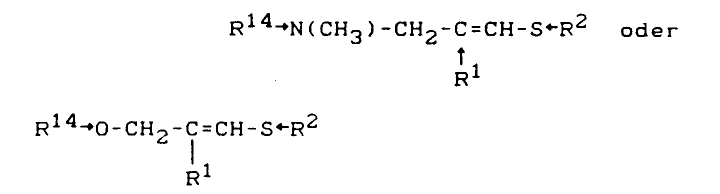 bilden kann
und deren Tautomere sowie deren pharmazeutisch verwendbare Hydrate und Saureadditionssalze sowie die Alkali-, Erdalkali-, Silber- und Guanidinumsalze der zugrundeliegenden Carbonsäuren.

Besonders bevorzugt sind die Verbindungen der Formel (I), in welcher
- R¹: für Cyclopropyl, cis-2-Fluorcyclopropyl, Ethyl, tert.-Butyl oder 2,4-Difluorphenyl,
- R²: für Wasserstoff,
- R³: für Hydroxy oder Ethoxy,
- R⁴: für Wasserstoff, Fluor oder Amino,
- R⁵: für Wasserstoff oder Fluor,
- R⁶: für Amino, Methylamino, Ethoxycarbonylamino, Aminomethyl, Ethoxycarbonylaminomethyl, Hydroxymethyl, Ethoxycarbonyl oder Wasserstoff,
- R⁷, R⁸, R⁹ und R¹⁰: für Wasserstoff oder Methyl und
- A: für N oder C-R¹⁴ steht, worin
- R¹⁴: für Wasserstoff, Fluor oder Chlor steht oder auch gemeinsam mit R¹ eine Brücke der Struktur 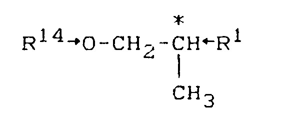 bilden kann
und deren Tautomere sowie deren pharmazeutisch verwendbare Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindung der Formel (I) erhalt, wenn man eine Verbindung der Formel (II) 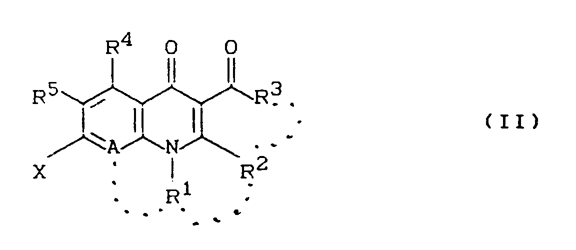
in welcher
- R¹, R², R³, R⁴, R⁵ und A: die oben angegebene Bedeutung haben und
- X: für Halogen, insbesondere Fluor oder Chlor steht,
mit Verbindungen der Formel (III) 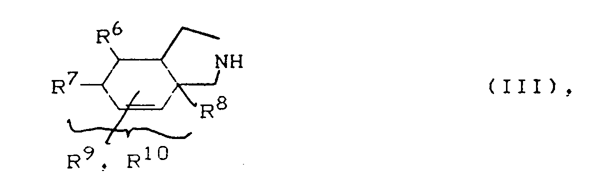
in welcher
R⁶, R⁷, R⁸, R⁹ und R¹⁰ die oben angegebene Bedeutung haben,
gegebenenfalls in Gegenwart von Säurebindern umsetzt und gegebenenfalls vorhandene Schutzgruppen durch alkalische oder saure Hydrolyse abspaltet und die erhaltenen Verbindungen gegebenenfalls in ihre Alkali-, Erdalkali-, Silber- oder Guanidiniumsalze überführt.

Verwendet man beispielsweise 1-Cyclopropyl-6,7,8-trifluor-1,4-dihydro-4-oxo-chinolincarbonsäure und (1SR, 2RS, 6SR)-2-Ethoxycarbonylamino-8-azabicyclo[4.3.0]non-4-en als Ausgangsstoffe, so kann der Reaktionsverlauf durch folgendes Formelschema wiedergegeben werden: 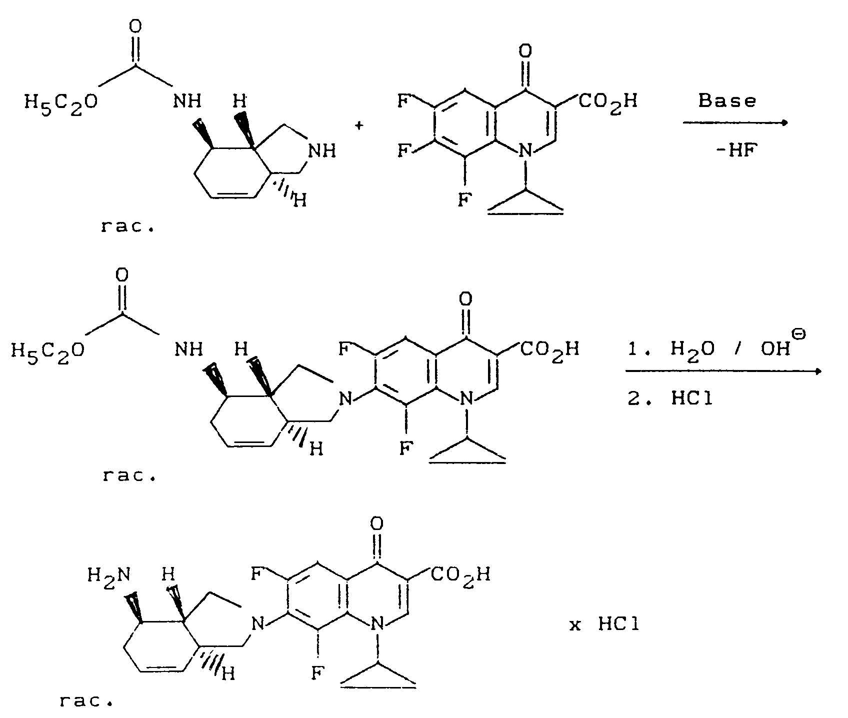
Verwendet man beispielsweise 1-Cyclopropyl-5,6,8-trifluor-1,4-dihydro-7[(1SR, 2RS, 6SR)-2-methylamino-8-azabicyclo[4.3.0]non-4-en-8-yl]-4-oxo-3-chinolincarbonsäure und Ammoniak als Ausgangsstoffe, so kann der Reaktionsverlauf durch folgendes Schema wiedergegeben werden: 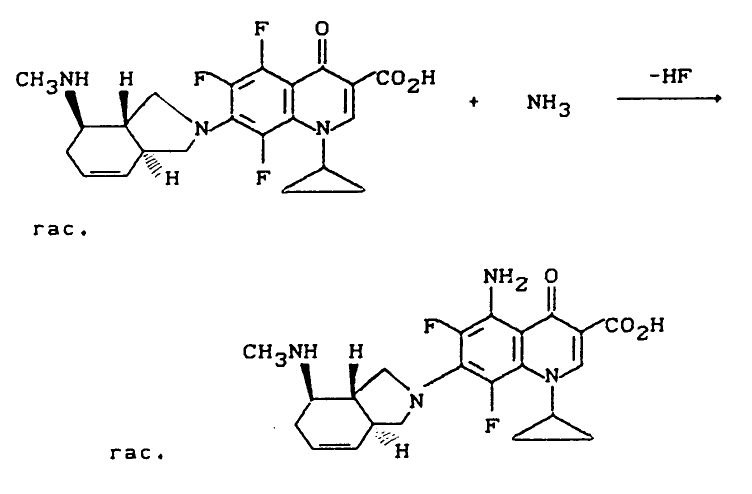
Verwendet man beispielsweise 7-Chlor-1-cyclopropyl-6-fluor-1,4-dihydro-4-oxo-1,8-naphthyridin-3-carbonsäure-ethylester und (1SR, 2RS, 6SR)-2-methylamino-8-azabicyclo[4.3.0]non-4-en als Ausgangsstoffe, so kann der Reaktionsverlauf durch folgendes Formelschema wiedergegeben werden: 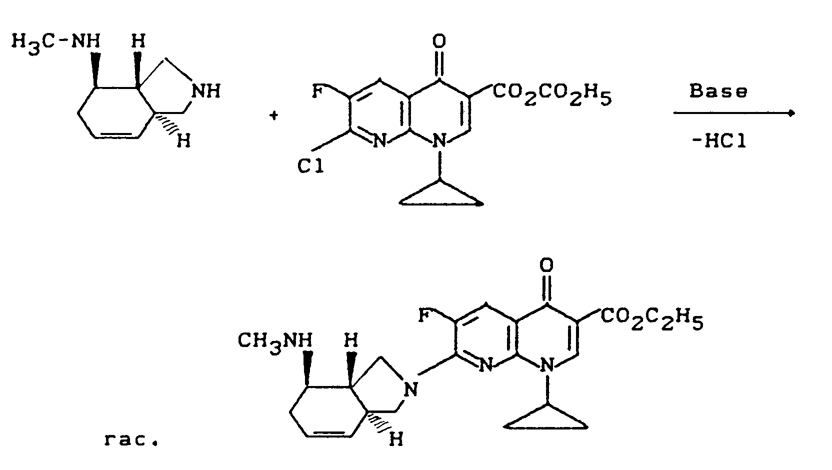
Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethylphosphorsäuretriamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 40 und 100°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol, der Verbindung (III) ein.

Freie Aminogruppen können wahrend der Umsetzung durch eine geeignete Aminschutzgruppe, z.B. tert.-Butyloxycarbonyl oder Ethoxycarbonyl oder als Azomethingruppe, geschützt und nach Beendigung der Reaktion durch Behandlung mit einer geeigneten Säure wie Chlorwasserstoffsäure oder Trifluoressigsäure oder einer geeigneten Base wie Natron- oder Kalilauge wieder freigesetzt werden (T.W. Greene, Protective Groups in Organic Synthesis, Seite 218-288, John Wiley & Sons, 1981).

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton oder Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die meisten der als Ausgangsstoffe verwendeten Verbindungen der Formel (II) sind bekannt oder können nach bekannten Methoden hergestellt werden. Als Beispiele seien genannt:
7-Chlor-1-cyclopropyl-6-fluor-1,4-dihydro-4-oxo-3-chinolincarbonsäure,
1-Cyclopropyl-6,7-difluor-1,4-dihydro-4-oxo-3-chinolincarbonsäure,
6-Chlor-1-cyclopropyl-7,8-difluor-1,4-dihydro-4-oxo-3-chinolincarbonsäure,
8-Chlor-1-cyclopropyl-6,7-difluor-1,4-dihydro-4-oxo-3-chinolincarbonsäure,
1-Cyclopropyl-6,7,8-trifluor-1,4-dihydro-4-oxo-3-chinolincarbonsäure,
5-Brom-1-cyclopropyl-6,7,8-trifluor-1,4-dihydro-4-oxo-3-chinolincarbonsäure,
5-Brom-1-(2,4-difluorphenyl)-6,7,8-trifluor-1,4-dihydro-4-oxo-3-chinolincarbonsäure,
1-Cyclopropyl-6,7-difluor-1,4-dihydro-8-methyl-4-oxo-3-chinolincarbonsäure,
6,7-Difluor-1-ethyl-1,4-dihydro-4-oxo-3-chinolincarbonsäure,
6,7,8-Trifluor-1-ethyl-1,4-dihydro-4-oxo-3-chinolincarbonsäure,
7-Chlor-6-fluor-1,4-dihydro-1-(2-hydroxyethyl)-4-oxo-3-chinolincarbonsäure,
6,7-Difluor-1-(2-fluorethyl)-1,4-dihydro-4-oxo-3-chinolincarbonsäure,
7-Chlor-6-fluor-1,4-dihydro-1-methoxy-4-oxo-3-chinolincarbonsäure,
7-Chlor-6-fluor-1,4-dihydro-1-methylamino-4-oxo-3-chinolincarbonsäure,
6,7-Difluor-1,4-dihydro-4-oxo-1-phenyl-3-chinolincarbonsäure,
7-Chlor-1-cyclopropyl-6-fluor-1,4-dihydro-4-oxo-1,8-naphthyridin-3-carbonsäure,
7-Chlor-1-cyclopropyl-6-fluor-1,4-dihydro-4-oxo-1,8-naphthyridin-3-carbonsäureethylester,
1-Cyclopropyl-6,7,8-trifluor-1,4-dihydro-4-oxo-3-chinolincarbonsäureethylester,
9,10-Difluor-2,3-dihydro-3-methyl-7-oxo-7H-pyrido[1,2,3-de][1,4]benzoxacin-6-carbonsäure,
8,9-Difluor-6,7-dihydro-5-methyl-1-oxo-1H,5H-benzo[i,j]chinolicin-2-carbonsäure,
7-Chlor-6-fluor-1-phenyl-1,4-dihydro-4-oxo-1,8-naphthyridin-3-carbonsäure,
7-Chlor-6-fluor-1-(4-fluorphenyl)-1,4-dihydro-4-oxo-1,8-naphthydrin-3-carbonsäureethylester,
6,7,8-Trifluor-1,4-dihydro-1-methylamino-4-oxo-3-chinolincarbonsäure,
1-Amino-6,7,8-trifluor-1,4-dihydro-4-oxo-3-chinolincarbonsäure,
6,7,8-Trifluor-1,4-dihydro-1-dimethylamino-4-oxo-3-chinolincarbonsäure,
6,7-Difluor-1-(4-fluorphenyl)-1,4-dihydro-8-methyl-4-oxo-3-chinolincarbonsäure,
7-Chlor-6-fluor-1-(4-fluorphenyl)-1,4-dihydro-4-oxo-3-chinolincarbonsäure,
7-Chlor-6-fluor-1-(2,4-difluorphenyl)-1,4-dihydro-4-oxo-3-chinolincarbonsäure,
6,7,8-Trifluor-1-(4-fluorphenyl)-1,4-dihydro-4-oxo-3-chinolincarbonsäure,
6,7,8-Trifluor-1-(2,4-difluorphenyl)-1,4-dihydro-4-oxo-3-chinolincarbonsäure,
6,7,8-Trifluor-1,4-dihydro-4-oxo-1-phenyl-3-chinolincarbonsäure,
7-Chlor-1-ethyl-6-fluor-1,4-dihydro-4-oxo-1,8-naphthyridin-3-carbonsäure,
6,7-Difluor-1,4-dihydro-4-oxo-1-vinyl-3-chinolincarbonsäure,
1-Cyclopropyl-5,6,7,8-tetrafluor-1,4-dihydro-4-oxo-3-chinolincarbonsäure,
5-Amino-1-cyclopropyl-6,7,8-trifluor-1,4-dihydro-4-oxo-3-chinolincarbonsäure,
1-Cyclopropyl-6,7,8-trifluor-1,4-dihydro-5-hydroxy-4-oxo-3-chinolincarbonsäure,
1-Cyclopropyl-6,7-difluor-1,4-dihydro-8-methoxy-4-oxo-3-chinolincarbonsäure,
7-Chlor-1-(2,4-difluorphenyl)-6-fluor-1,4-dihydro-4-oxo-1,8-naphthyridin-3-carbonsäure,
7-Chlor-1-(2,4-difluorphenyl)-6-fluor-1,4-dihydro-4-oxo-1,8-naphthyridin-3-carbonsäureethylester,
9,1-Epoxymethano-7,8-difluor-5-oxo-5H-thiazolo[3,2-a]chinolin-4-carbonsäure,
1-Cyclopropyl-6,7-difluor-1,4-dihydro-4-oxo-8-trifluormethyl-3-chinolincarbonsäure,
9,10-Difluor-2,3-dihydro-7-oxo-7H-pyrido[1,2,3-de][1,4]benzothiazin-6-carbonsäure,
6,7,8-Trifluor-1-methyl-4-oxo-1H,4H-[1,3]thiazeto[3,2-a]-3-chinolincarbonsäure,
1-Cyclopropyl-6,7,8-trifluor-1,4-dihydro-5-methyl-4-oxo-3-chinolincarbonsäure,
9-Cyclopropyl-6,7-difluor-2,3,4,9-tetrahydro-5-methylisothiazolo[5,4-b]chinolin-3,4-dion,
6,7-Difluor-1-(1,2-cis-2-fluorcyclopropyl)-1,4-dihydro-4-oxo-3-chinolincarbonsäure,
6,7,8-Trifluor-1-(1,2-cis-2-fluorcyclopropyl)-1,4-dihydro-4-oxo-3-chinolincarbonsäure,
8-Chlor-6,7-difluor-1-(1,2-cis-2-fluorcyclopropyl)-1,4-dihydro-4-oxo-3-chinolincarbonsäure,
7-Chlor-6-fluor-1-(1,2-cis-2-fluorcyclopropyl)-1,4-dihydro-4-oxo-1,8-naphthyridin-3-carbonsäure,
1-Cyclopropyl-8-difluormethoxy-6,7-difluor-1,4-dihydro-4-oxo-3-chinolincarbonsäure (EP-A-352 123),
8,9-Difluor-1,2-dihydro-2-methyl-6-oxo-6H-pyrrolo[3,2,1-ij)chinolin-5-carbonsäure,
8-Chlor-6,7-difluor-1,4-dihydro-1-(oxetan-3-yl)-4-oxo-3-chinolincarbonsäure,
9,1-(Methylimino)methano-7,8-difluor-5-oxo-5H-thiazolo[3,2-a]chinolin-4-carbonsäure,
1-Cyclopropyl-6,7-difluor-1,4-dihydro-4-oxo-8-vinyl-3-chinolincarbonsäure,
7,8-Difluor-2-mthyl-5-oxo-5H-[1,3,4]thiadiazolo[3,2-a]chinolin-4-carbonsäure,
5-Cyclopropyl-6,7,8-trifluor-1-hydroxy-2,3,5,10-tetrahydro-3,10-dioxobenzo[b]-1,6-naphthyridin,
9-Cyclopropyl-6,7-difluor-3-hydroxy-4,9-dihydro-4-oxo-thieno[2,3-b]chinolin-2-carbonitril,
4,5-Difluor-2,3-dihydro-1-methyl-7-oxo-1H,7H-pyrido[3,2,1-ij]cinnolin-8-carbonsäure,
1-Bicyclo[1.1.1]pentyl-6,7-difluor-1,4-dihydro-4-oxo-3-chinolincarbonsäure,
1-tert.-Butyl-6,7-difluor-1,4-dihydro-4-oxo-3-chinolincarbonsäure,
7-Chlor-6-fluor-1-(1-fluormethyl-1-methyl-ethyl)-1,4-dihydro-4-oxo-1,8-naphthyridin-3-carbonsäure,
5,8-Dichlor-1-cyclopropyl-6,7-difluor-1,4-dihydro-4-oxo-3-chinolincarbonsäure.

Die als Ausgangsstoffe verwendeten Verbindungen der Formel (III) sind neu. Es wurde ein Verfahren zur Herstellung der erfindungsgemäßen Verbindungen der allgemeinen Formel (III) 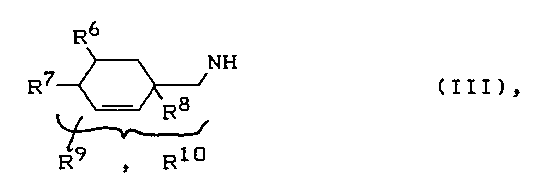
in welcher
R⁶, R⁷, R⁸, R⁹ und R¹⁰ die oben angegebene Bedeutung haben, gefunden, das dadurch gekennzeichnet ist, daß man geeignete Diene mit geeigneten Dienophilen in einer Diels-Alder-Reaktion umsgesetzt, die intermolekular oder intramolekular durchgeführt werden kann und gegegebenenfalls anschließend weitere chemische Reaktionen durchführt, um gegebenenfalls den Pyrrolidinring aufzubauen und um für die biologische Wirkung gewünschte Substituenten einzuführen und als letzten Schritt die Schutzgruppe am Pyrrolidinstickstoff abspaltet.

Bei intramolekularer Durchführung der Diels-Alder-Reaktion werden Verbindungen der Formel (1) oder (2) 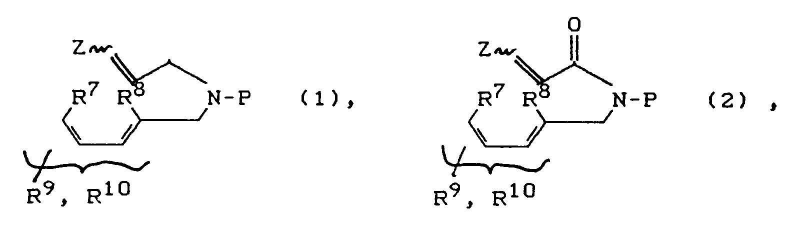
in denen
- R⁷, R⁸, R⁹ und R¹⁰: die oben angegebene Bedeutung haben und
- P: für eine Schutzgruppe (beispielsweise Allyl, Acyl, Carbamoyl oder Trityl),
- Z: für Wasserstoff, eine Carboxyl-, Carbonester- oder Carbonamidgruppe, CN oder NO₂ steht,
zur Verbindungen der Formel (3) [ausgehend von (1)] oder (4) [ausgehend von (2)] 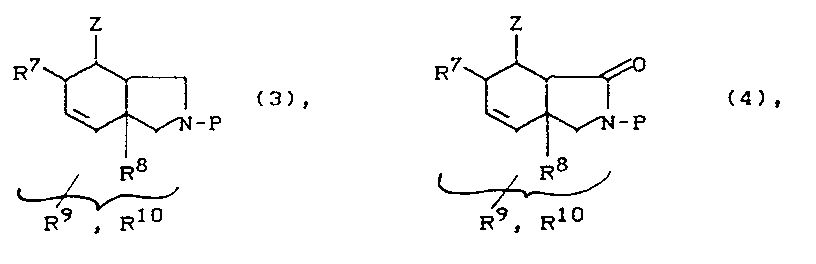
in denen
R⁷, R⁸, R⁹, R¹⁰, P und Z die oben angegebene Bedeutung haben, umgesetzt. Intramolekulare Diels-Alder-Reaktionen ähnlicher Art sind teilweise bekannt: J.M. Mellor, A.M. Wagland; J. Chem. Soc. Perkin I, 997-1005 (1989); W.R. Roush, S.E. Hall; J. Am. Chem. Soc. 103, 5200 (1980). E. Ciganek; Organic Reactions 32, 1-374 (1984). In diesen Arbeiten fehlen jedoch Hinweise auf Schutzgruppen, die gleichzeitig für die Reaktion geeignet und anschließend problemlos abspaltbar sind.

Bei intermolekularer Durchführung der Diels-Alder-Reaktion werden Diene der Formel(5) mit Dienophilen der Formel (6) zu Verbindungen der Formel (7) umgesetzt, und gegebenenfalls nach Modifizierung der Gruppen Z¹ und Z², beispielsweise Überführung eines cyclischen Carbonsäureanhydrids in einen Diester unter Abspaltung der Schutzgruppen P¹ oder P¹ und P², unter Cyclisierung zu den Laktamen der Formel (8) umgesetzt. 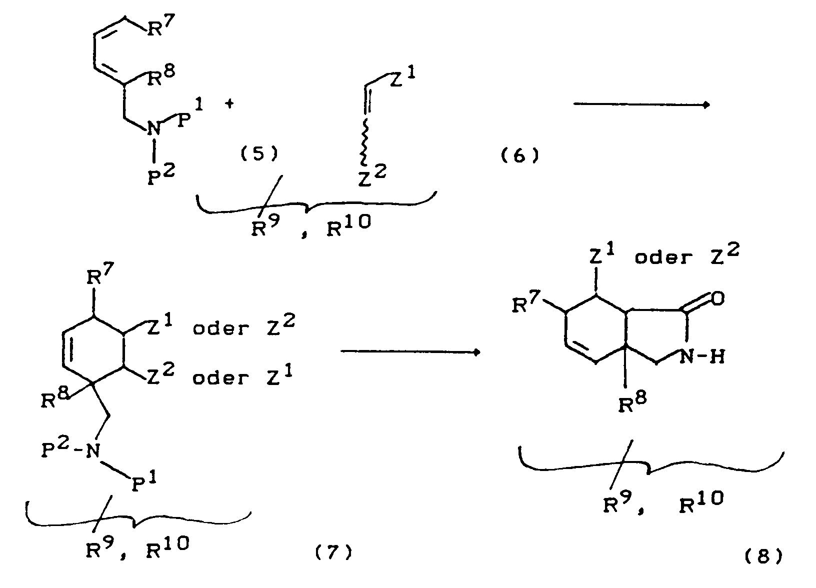
In der Formel (5), (6), (7) und (8) haben R⁷, R⁸, R⁹ und R¹⁰ die oben angegebene Bedeutung,
- P¹: steht für eine Acyl- oder Carbamoylschutzgruppe, wenn
- P²: für Wasserstoff steht oder
- P¹: bildet gemeinsam mit P² ein Imid,
- Z¹ und Z²: stehen für Wasserstoff, Carboxyl, Carbonester- oder Carbonamidgruppen, CN oder NO₂, wobei mindestens eine der beiden Gruppen Z¹ oder Z² eine Carbonestergruppe oder eine Carbonamidgruppe oder CN sein muß oder Z¹ und Z² bilden gemeinsam eine Brücke, so daß ein cyclisches Carbonsäureanhydrid gebildet wird.

Bevorzugte Schutzgruppen P, P¹, P² sind solche Schutzgruppen, bei denen unter den Bedingungen, die zu ihrer Abspaltung verwendet werden, die Cyclisierung zum Laktam und gegebenenfalls eine Veresterung einer zweiten, noch freien Carboxylfunktion mit dem als Lösungsmittel verwendeten Alkohol stattfindet, derart daß alle Reaktionsschritte in einer Eintopfreaktion ausgeführt werden können, und eine unkontrollierte Umwandlung gegebenenfalls diastereomeren- und enantiomerenreiner Ausgangsstoffe in nicht oder schwer trennbare Isomerengemische nicht stattfindet.

Als Beispiele seien genannt:
1. die tert.-Butyloxycarbonylschutzgruppe
   (Abspaltung mit wäßrigen oder alkoholischen Säuren)
2. die Phthalimidoschutzgruppe
   (Aminolyse mit primären Aminen in wäßrigen oder wasserfreien Alkoholen als Lösungsmittel)
Reaktivität und Selektivität, insbesondere Diastereoselektivität sowohl der intramolekularen als auch der intermolekularen Diels-Alder-Reaktion werden durch die Art des Diens und des Dienophils (z.B. Art und Anzahl aktivierender Gruppen, Art und Anzahl zusätzlichen Substituenten, cis- oder trans-Konfiguration an der Doppelbindung) sowie durch die Art der Reaktionsführung (intra- oder intermolekular) und durch die Art der Schutzgruppe (P, P¹, P²) beeinflußt.

Die im Rahmen des erfindungsgemäßen Verfahrens durchzuführenden Diels-Alder- und Cyclisierungsreaktionen können beispielsweise durch die nachfolgenden Formelschemata erläutert werden:

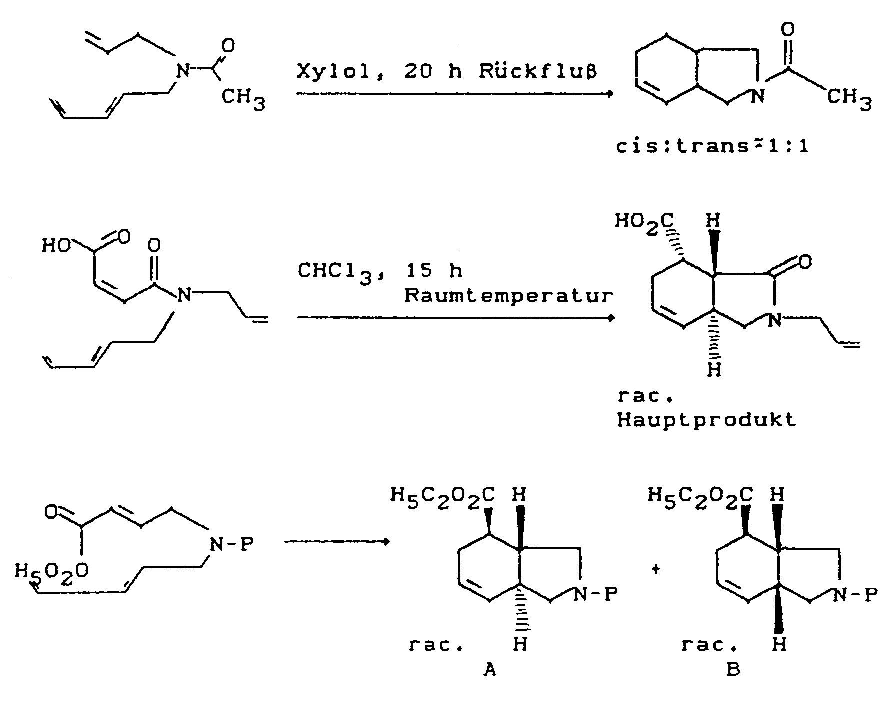

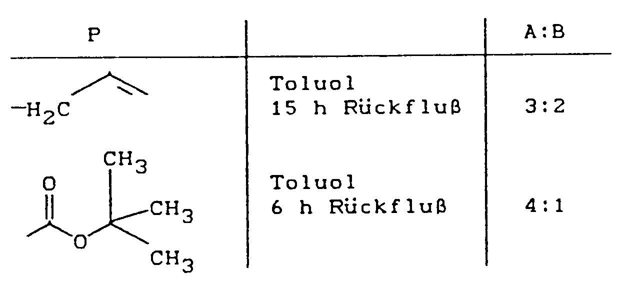

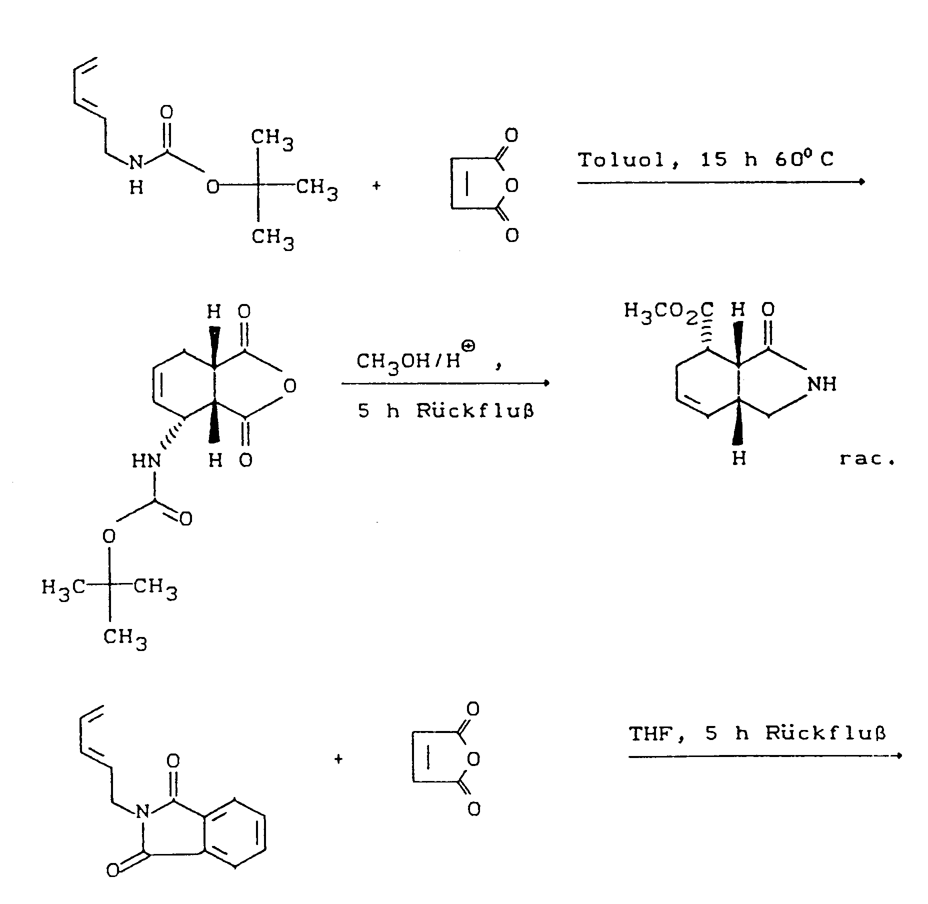

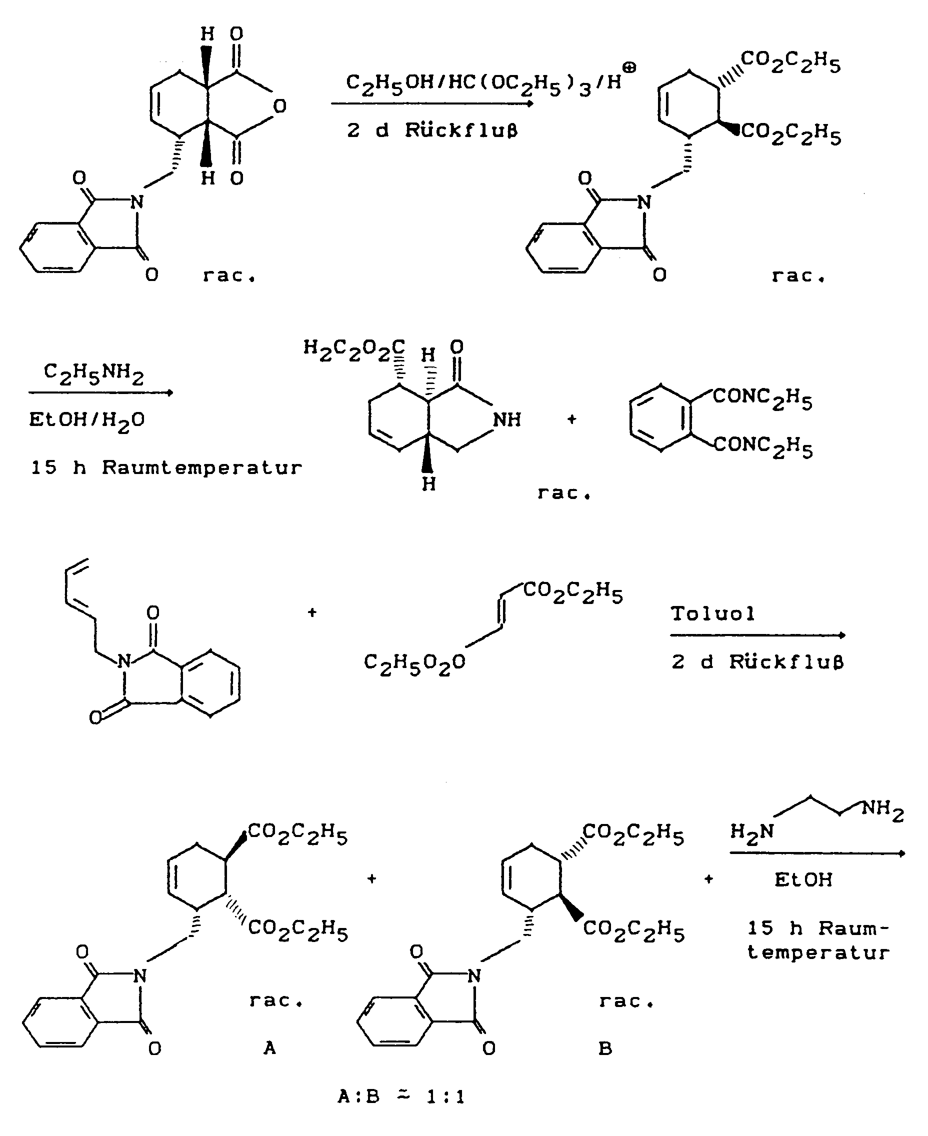

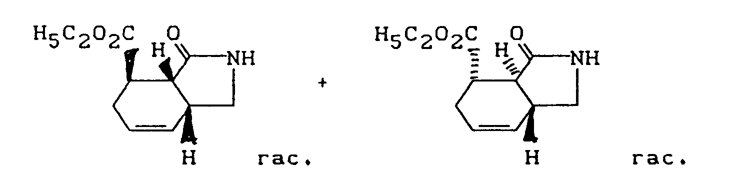

Für die Diels-Alder-Reaktion kommen als Verdünnungsmittel alle inerten organischen Lösungsmittel in Frage. Hierzu gehören vorzugsweise Ether, wie Diisopropylether, Di-n-butylether, Dimethoxyethan, Tetrahydrofuran und Anisol, Kohlenwasserstoffe, wie z.B. Hexan, Methylcyclohexan, Toluol, Xylol und Mesitylen und halogenierte Kohlenwasserstoffe, wie z.B. Chloroform, 1,2-Dichlorethan und Chlorbenzol. Die Diels-Alder-Reaktion kann aber auch ohne Lösungsmittel durchgeführt werden.

Die Reaktionstemperaturen können in einem größeren Bereich variiert werden. Im allgemeinen arbeitet man zwischen etwa -20°C und +200°C, vorzugsweise zwischen -20°C und +150°C. Die Diels-Alder-Reaktion wird normalerweise bei Normaldruck durchgeführt. Zur Beschleunigung der Reaktion können aber auch Drucke bis zu 1,5 GPa eingesetzt werden.

Die weitere Umsetzung der Verbindungen der Formel (7) zu den Verbindungen der Formel (8) erfolgt wie in den Beispielen beschrieben oder nach bekannten Methoden der organischen Chemie.

Um ausgehend von den Verbindungen der Formel (3), (4) oder (8) zu den Verbindungen der Formel (III) zu gelangen, sind weitere Reaktionen erforderlich.

Beispielsweise genannt seien die Hydrolyse eines Esters zur Carbonsäure, die Reduktion von Carbonylgruppen zum Beispiel von Estern zu Aldehyden oder Alkoholen oder von Laktamgruppen zu den Pyrrolidinen, die Überführung einer Hydroxyfunktion in eine Aminofunktion, die Überführung einer Carboxylfunktion oder eines ihrer Derivate unter Abbau um ein Kohlenstoffatom in eine Aminfunktion, die reduktive Aminierung eines Aldehyds mit einer im Molekül vorhandenen Aminfunktion, die reduktive Aminierung eines im Molekül vorhandenen Aldehydfunktion mit einen Amin, die Einführung von Schutzgruppen, die Abspaltung der Schutzgruppe am Pyrrolidinstickstoff derart, daß im Molekül eventuell vorhandene weitere Schutzgruppen erhalten bleiben.

Diese Umsetzungen erfolgen wie in den Beispielen beschrieben oder nach in der organischen Chemie üblichen Methoden.

Die weitere Umsetzung der Verbindungen der Formel (3), (4) oder (8) zu den Verbindungen der Formel (III) kann beispielsweise durch die nachfolgenden Formelschemata erläutert werden: 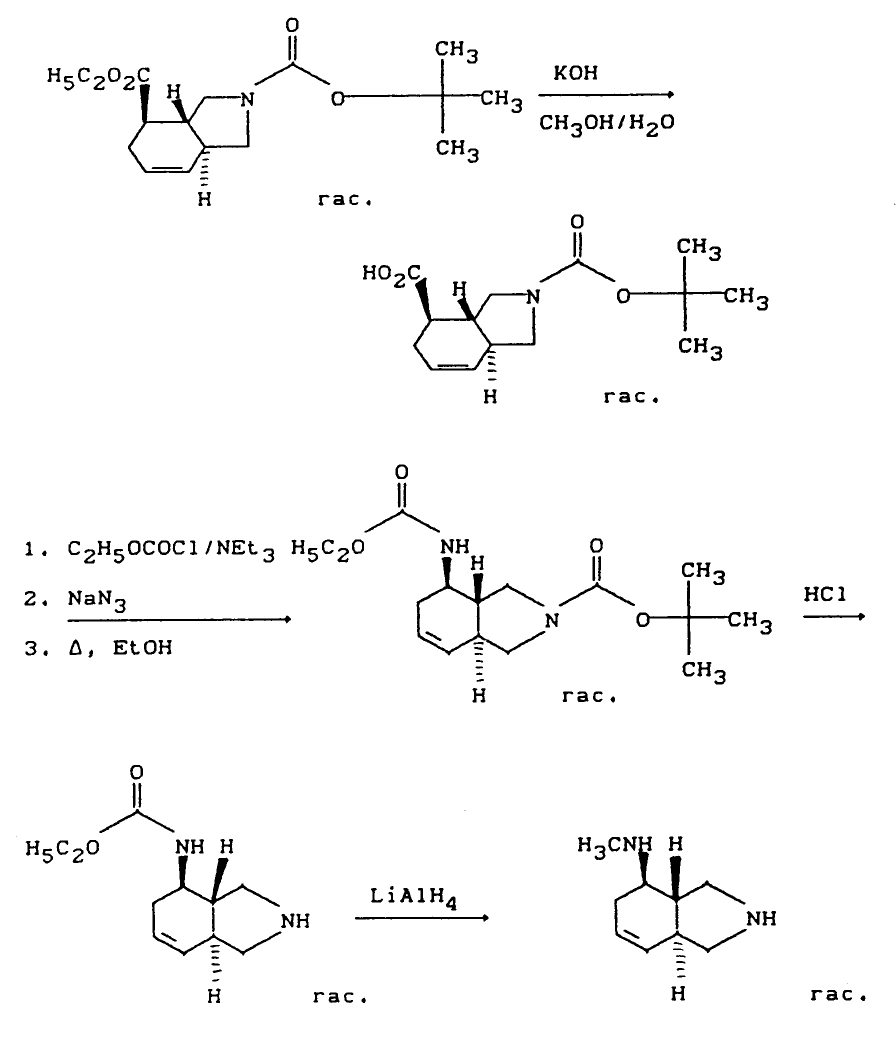 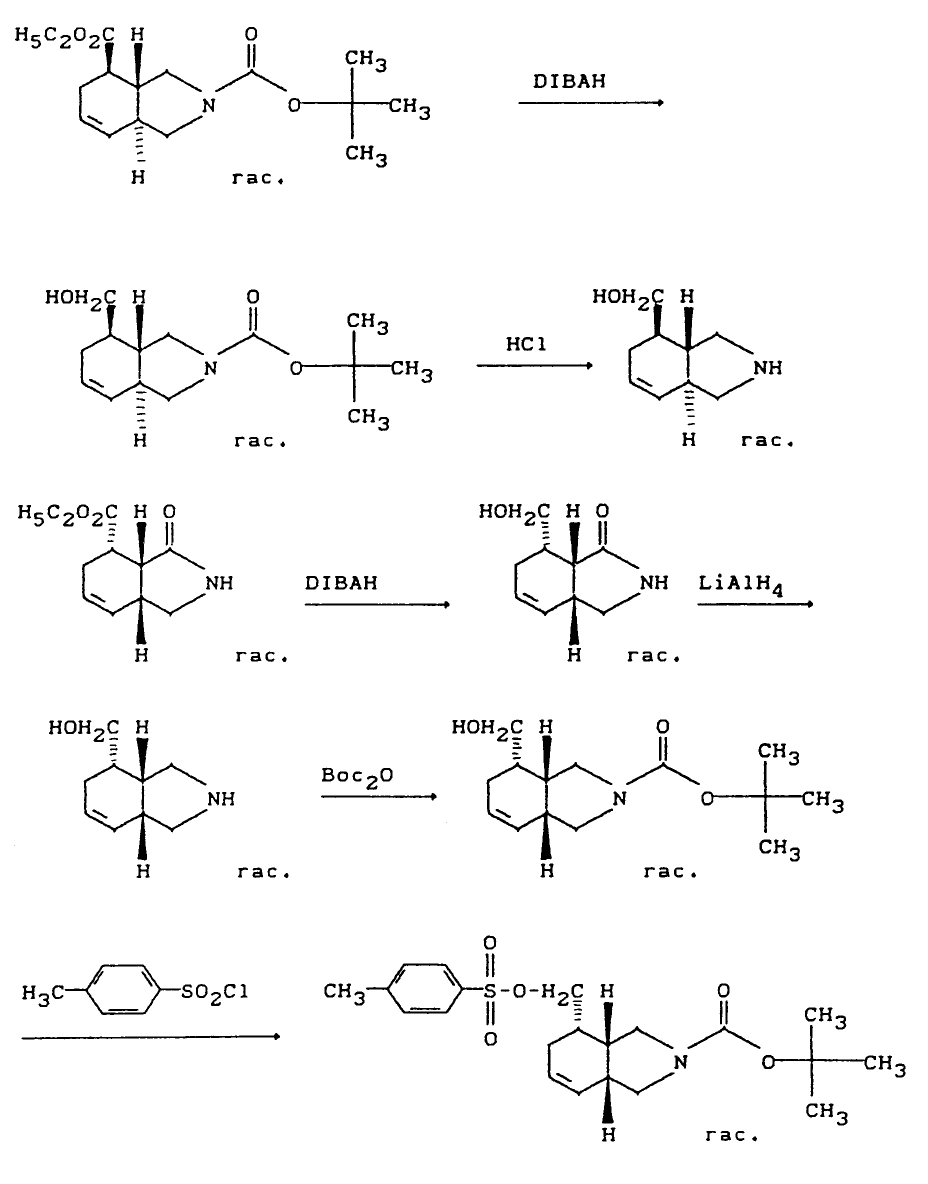 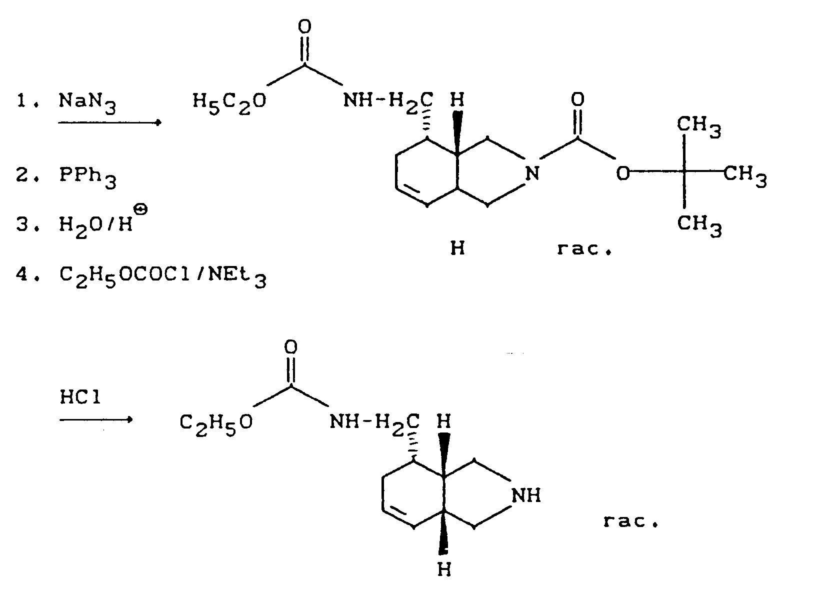
Die meisten Ausgangsstoffe der Formel (1), (2), (5) und (6) sind bekannt oder können nach bekannten Methoden der organischen Chemie hergestellt werden.

Als Beispiele für Verbindungen der Formel (III), die sowohl als Racemate als auch als enantiomeren- oder diastereomerenreine Verbindungen eingesetzt werden können, seien genannt:
8-Azabicyclo[4.3.0]non-2-en
8-Azabicyclo[4.3.0]non-4-en-2-carbonsäureethylester
2-Hydroxymethyl-8-azabicyclo[4.3.0]non-4-en
2-Amino-8-azabicyclo[4.3.0]non-4-en
2-Ethyloxycarbonylamino-8-azabicyclo[4.3.0]non-4-en
2-tert.-Butyloxycarbonylamino-8-azabicyclo[4.3.0]non-4-en
2-Benzyloxycarbonylamino-8-azabicyclo[4.3.0]non-4-en
2-Allyloxycarbonylaminomethyl-8-azabicyclo[4.3.0]non-4-en
2-Aminomethyl-8-azabicyclo[4.3.0]non-4-en
2-Ethyloxycarbonylaminomethyl-8-azabicyclo[4.3.0]non-4-en
2-tert.-Butyloxycarbonylaminomethyl-8-azabicyclo[4.3.0]non-4-en
2-Methylamino-8-azabicyclo[4.3.0]non-4-en
2-Ethylamino-8-azabicyclo[4.3.0]non-4-en
2-Cyclopropylamino-8-azabicyclo[4.3.0]non-4-en
2-Dimethylamino-8-azabicyclo[4.3.0]non-4-en
2-[(2-Hydroxyethyl)-amino]-8-azabicyclo[4.3.0]non-4-en
2-Amino-1-methyl-8-azabicyclo[4.3.0]non-4-en
2-Amino-2-methyl-8-azabicyclo[4.3.0]non-4-en
2-Amino-3-methyl-8-azabicyclo[4.3.0]non-4-en
2-Ethyloxycarbonylamino-3-methyl-8-azabicyclo[4.3.0]non-4-en
2-tert.-Butyloxycarbonylamino-3-methyl-8-azabicyclo[4.3.0]non-4-en
2-Benzyloxycarbonylamino-3-methyl-8-azabicyclo[4.3.0]non-4-en
2-Allyloxycarbonylaminomethyl-3-methyl-8-azabicyclo[4.3.0]non-4-en
2-Amino-4-methyl-8-azabicyclo[4.3.0]non-4-en
2-Amino-5-methyl-8-azabicyclo[4.3.0]non-4-en
2-Amino-6-methyl-8-azabicyclo[4.3.0]non-4-en
2-Amino-7-methyl-8-azabicyclo[4.3.0]non-4-en
2-Amino-9-methyl-8-azabicyclo[4.3.0]non-4-en
6-Amino-10-oxa-3-azatricyclo[5.2.1.0^{1,5}]dec-8-en
6-Ethyloxycarbonylamino-10-oxa-3-azatricyclo[5.2.1.0^{1,5}]dec-8-en
6-tert.-Butyloxycarbonylamino-10-oxa-3-azatricyclo[5.2.1.0^{1,5}] dec-8-en
6-Aminomethyl-10-oxa-3-azatricyclo[5.2.1.0^{1,5}]dec-8-en
6-Ethyloxycarbonylaminomethyl-10-oxa-3-azatricyclo[5.2.1.0^{1,5}] dec-8-en
6-tert.-Butyloxycarbonylaminomethyl-10-oxa-3-azatricyclo-[5.2.1.0^{1,5}]dec-8-en
6-Amino-3-azatricyclo[5.2.1.0^{1,5}]dec-8-en
6-Amino-3-azatricyclo[5.2.2.0^{1,5}]undec-8-en.

Außer den in den Beispielen genannten Wirkstoffen können auch die in der folgenden Tabelle aufgeführten Wirkstoffe hergestellt werden, die sowohl als Racemate als auch als enantiomeren- oder diastereomerenreine Verbindungen vorliegen können:

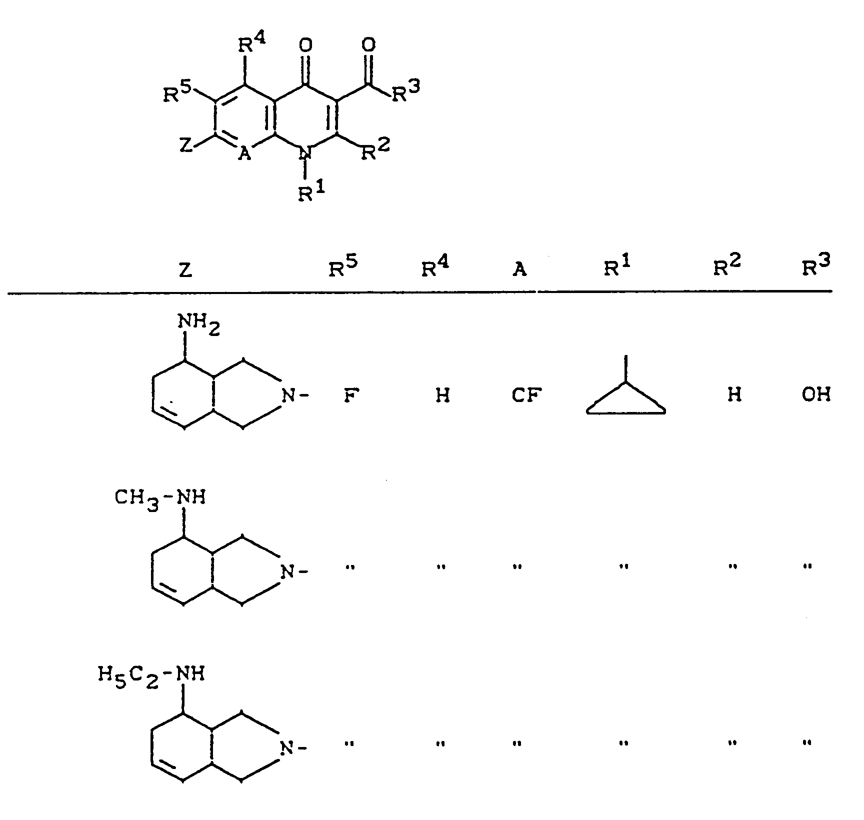

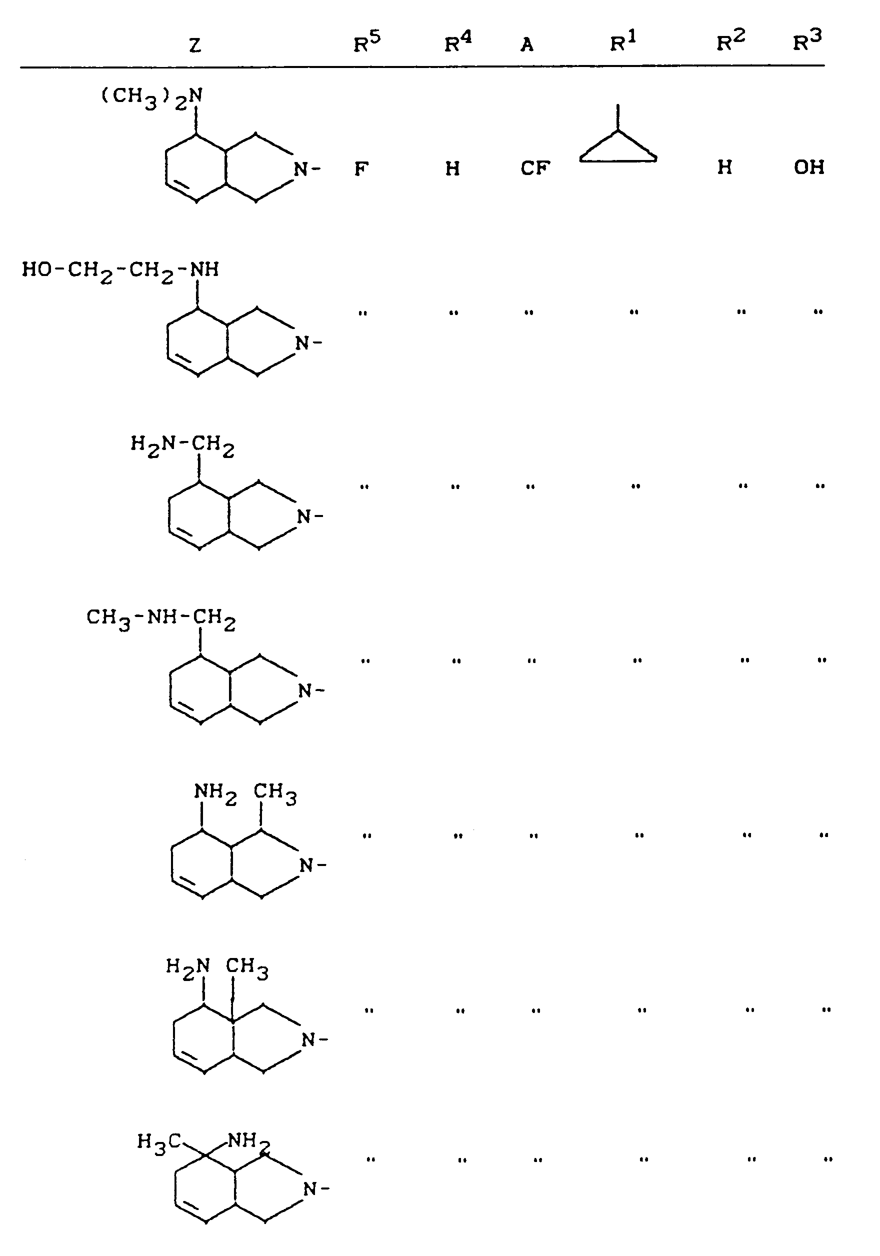

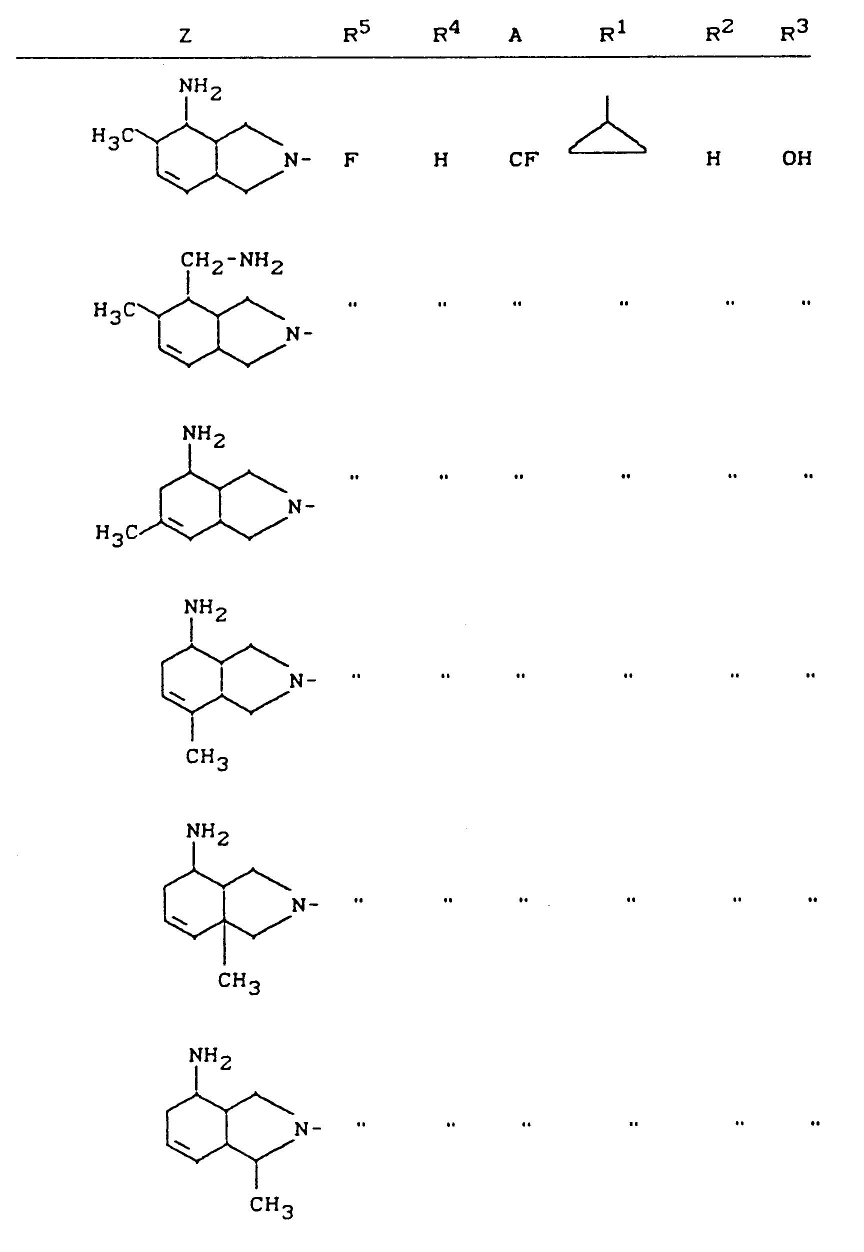

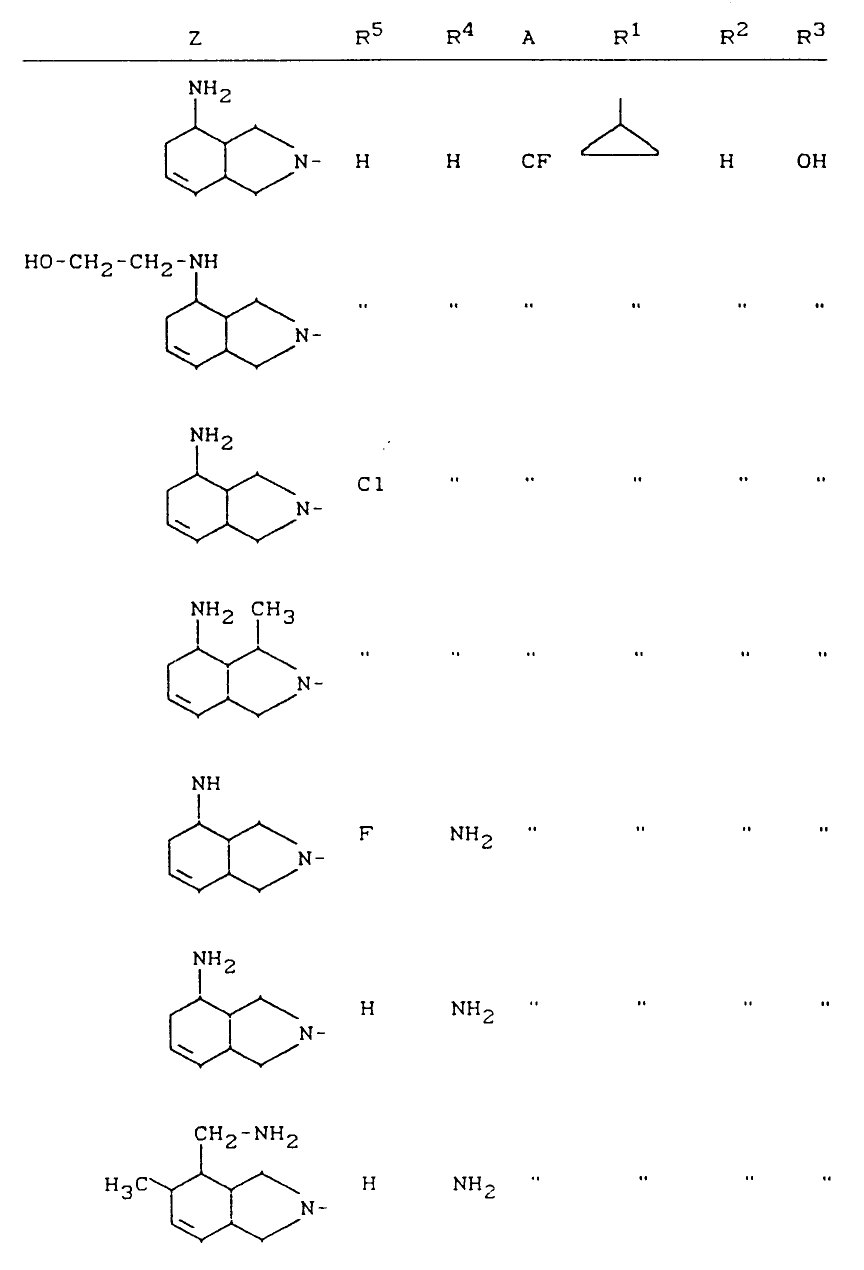

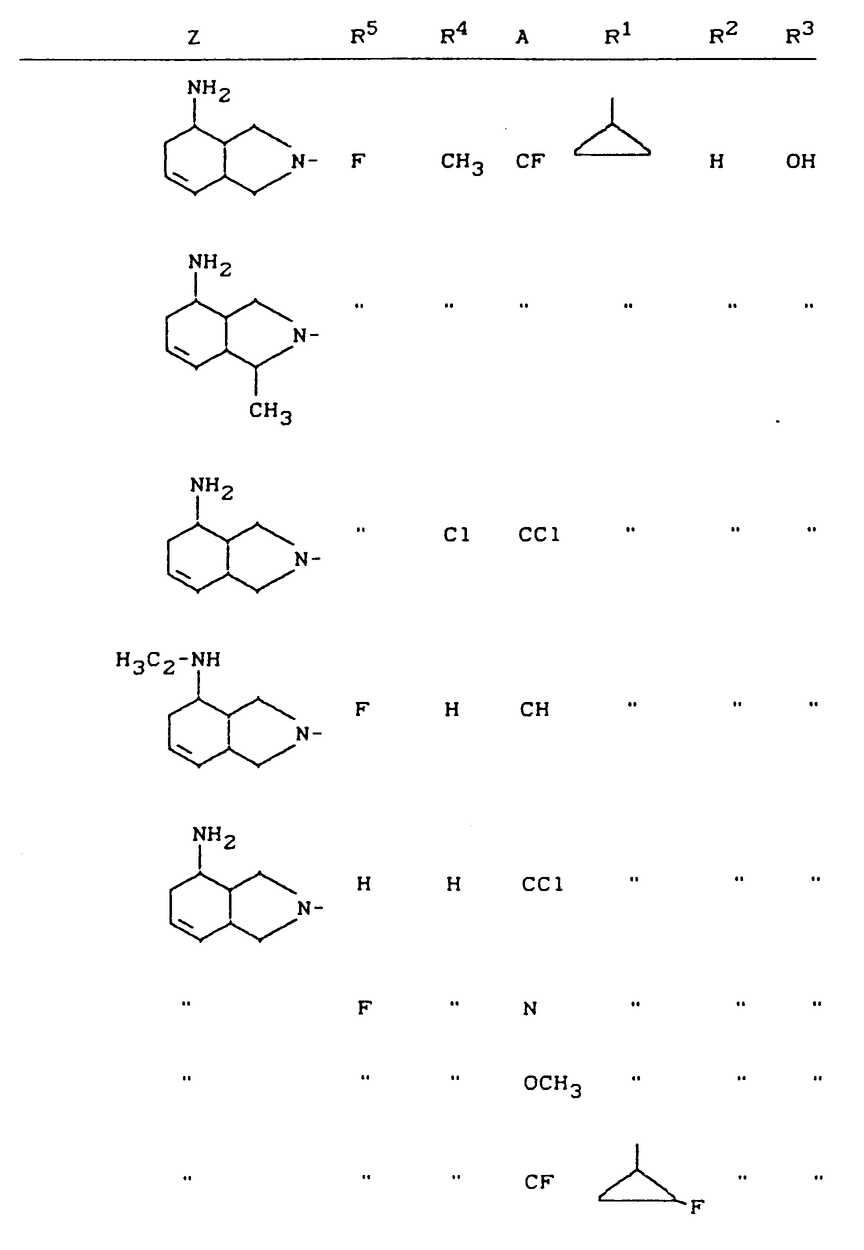

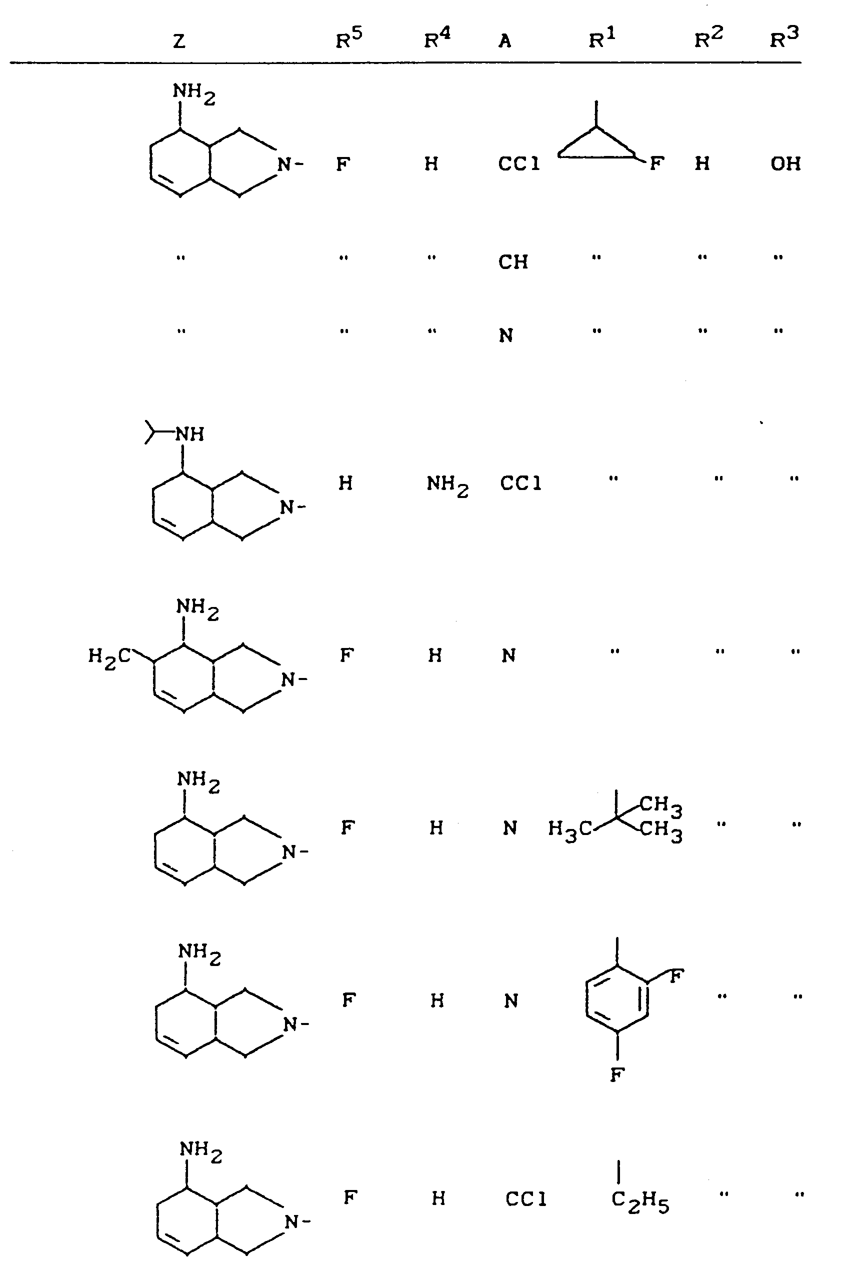

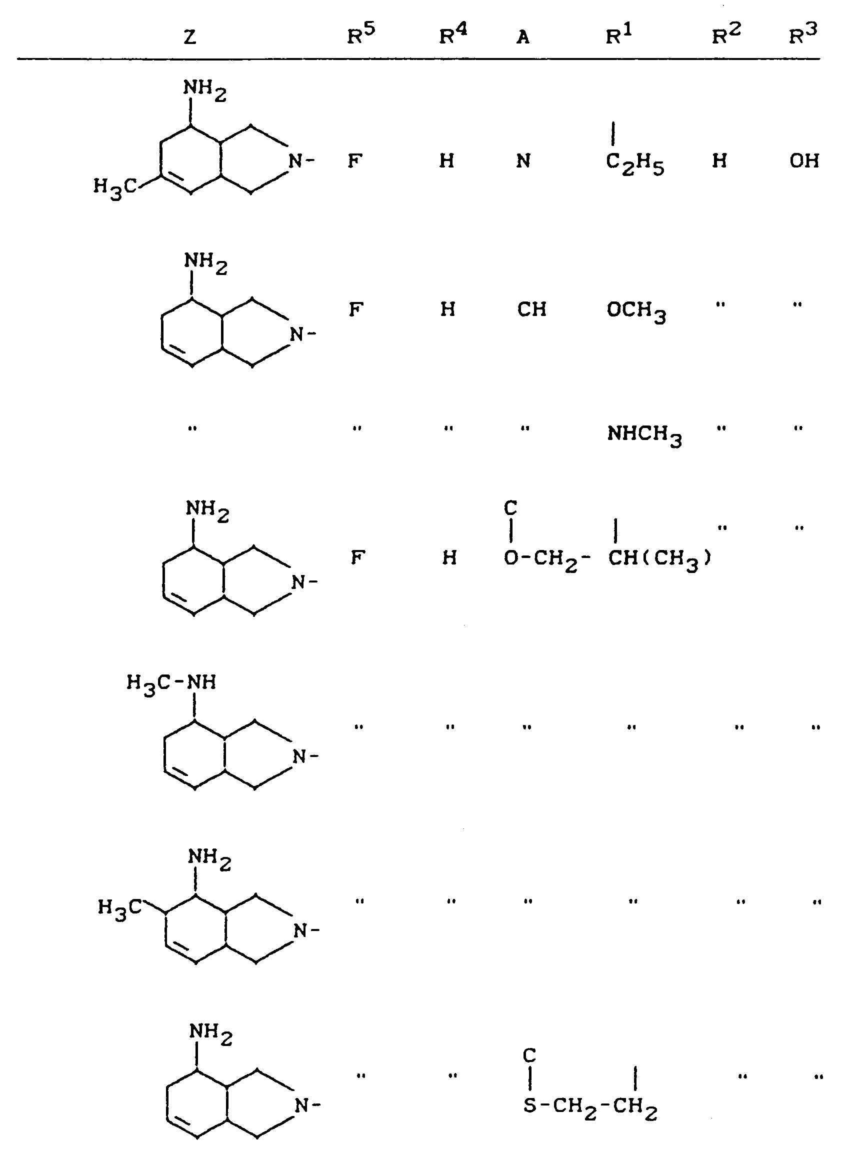

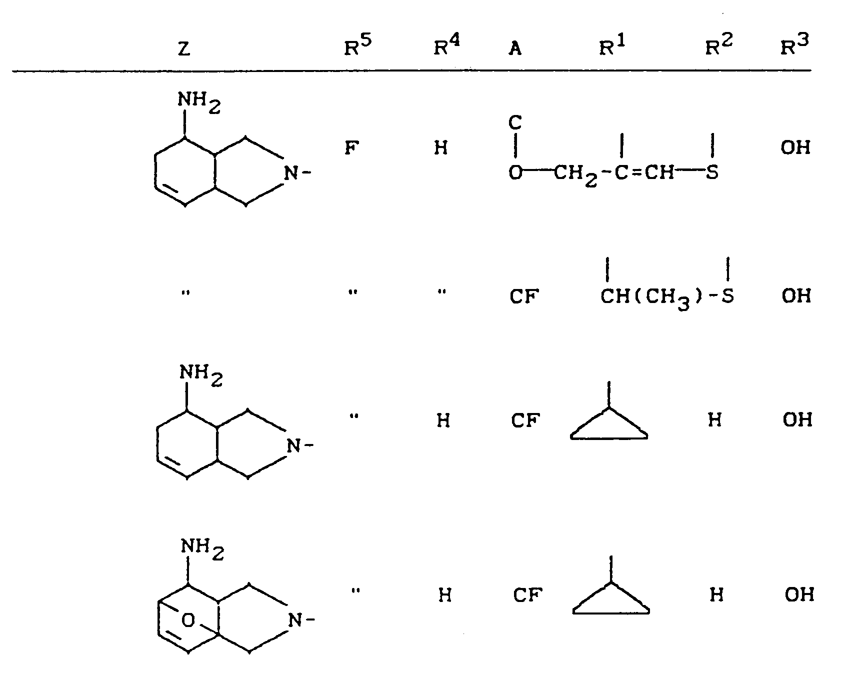

Die erfindungsgemäßen Verbindungen wirken stark antibiotisch und zeigen ein breites antibakterielles Spektrum gegen grampositive und gramnegative Keime, insbesondere gegen Enterobakterien; vor allem auch gegen solche, die resistent sind gegen verschiedene Antibiotika, wie z.B. Penicilline, Cephalosporine, Aminoglykoside, Sulfonamide, Tetracycline und andere Chinolone, Die erfindungsgemäßen Verbindungen zeichnen sich durch gute Verträglichkeit aus.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen weit unterhalb von Konzentrationen bisher bekannter Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Pseudomonas aeruginosa, Enterococcus faecalis und Escherichia coli beobachtet werden.

Auch gegenüber Bakterien, die gegenüber vergleichbaren Substanzen als weniger empfindlich eingestuft werden, insbesondere resistenten Staphylococcus aureus, Escherichia coli, Pseudomonas aeruginosa und Enterococcus faecalis zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Darüberhinaus eignen sich die erfindungsgemäßen Verbindungen zur Behandlung von Tumorerkrankungen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich 7-(4-Amino-1,3-dihydro-isoindol-2-yl)-1-cyclopropyl-6,8-difluor-1,4-dihydro-4-oxo-3-chinolincarbonsäure (EP-A-343 560, Beispiel 2) aufgeführt.

**Tabelle**

| MHK-Werte (µg/ml) | | | | | | | |
|---|---|---|---|---|---|---|---|
| Beispiel | * | 4 | 6 | 12 | 14 | 19 | 22 |
| E. coli Neumann | 0,5 | 0,0078 | ≦ 0,015 | 0,0039 | 0,03 | ≦ 0,015 | 0,03 |
| Klebsiella sp. 8085 | 4 | 0,015 | 0,03 | 0,015 | 0,125 | 0,03 | 0,06 |
| Enterobacter cloacae 2427 | - | 0,015 | 0,06 | 0,015 | 0,125 | 0,03 | 0,125 |
| Morganella morg. 932 | 1 | 0,015 | - | 0,015 | 0,125 | 0,03 | 0,125 |
| Providencia sp. 12012 | 1 | 0,015 | 0,03 | 0,015 | 0,125 | ≦ 0,015 | 0,125 |
| Micrococcus luteus 9341 | - | 0,015 | 0,06 | 0,015 | 0,125 | 0,06 | 0,125 |
| Staphylococcus aureus ICB 25701 | - | 0,03 | 0,125 | 0,015 | 0,25 | 0,03 | 0,125 |
| Staphylococcus aureus 1756 | 0,03 | 0,0039 | ≦ 0,015 | ≦ 0,0019 | 0,0078 | ≦ 0,015 | ≦ 0,015 |
| Enterococcus faecalis 27 101 | 0,5 | 0,15 | 0,03 | 0,0078 | 0,03 | ≦ 0,015 | 0,03 |
| Pseudomonas aeruginosa Walter | > 128 | 0,25 | 0,5 | 0,25 | 1 | 1 | 2 |
| *: Referenzverbindung: 7-(4-Amino-1,3-dihydro-isoindol-2-yl)-1-cyclopropyl-6,8-difluor-1,4-dihydro-4-oxo-3-chinolincarbonsäure (EP 343 560, Beispiel 2) | | | | | | | |

### Herstellung der Zwischenprodukte:

### Beispiel A:

### 8-Azabicyclo[4.3.0]non-2-en

### A.1. (E)-1-Brom-2,4-pentadien

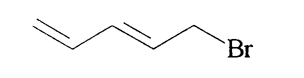

84 g (1,0 mol) 1,4-Pentadien-3-ol bei 0 C vorlegen. Unter Rühren 150 ml (≈ 1,3 mol) 48 %-ige wäßrige Bromwasserstoffsäure so zutropfen, daß die Innentemperatur 5 C nicht überschreitet. Nach vollständiger Zugabe 1 h bei Raumtemperatur nachrühren. Die organische Phase wird abgetrennt und ohne Reinigung weiter umgesetzt.
- Ausbeute:: 107-129 g (73-88 % der Theorie)

### A.2. (E)-1-(2-Propenylamino)-2,4-pentadien

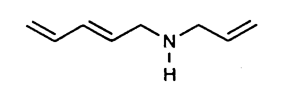

228 g (4,0 mol) 1-Amino-2-propen vorlegen. Unter Rühren 58,8 g (0,4 mol) (E)-1-Brom-2,4-pentadien (Titelverbindung aus Beispiel A.1.) zutropfen. Durch Kühlung die Innentemperatur im Bereich von 20-30 C halten. 5 h bei Raumtemperatur rühren. Ansatz bei 150 mbar einengen. 20 g (0,5 mol) Natriumhydroxid in 200 ml Wasser gelöst zugeben, mit zweimal je 100 ml Methylenchlorid extrahieren, mit Natriunlsulfat trocknen, 0,1 g 4-Hydroxyanisol zusetzen, einengen, bei 40 mbar destillieren. Zur Stabilisierung werden dem Destillat 10-20 ppm 4-Hydroxyanisol zugesetzt.
- Ausbeute:: 33-35 g (67-72 % der Theorie)
- Siedepunkt:: 77-82 C bei 40 mbar
¹H-NMR (CDCl₃): δ = 6,07-6,48 (m, 2H); 5,64-6,07 (m, 2H); 5,00-5,27 (m, 4H); 3,19-3,36 ppm (m, 4H).

### A.3. N-[(E)-2,4-Pentadienyl]-N-(2-propenyl)-acetamid

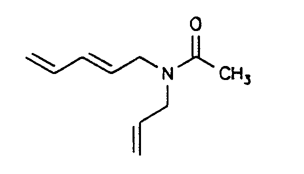

24,6 g (0,2 mol) (E)-1-(2-propenylamino)-2,4-pentadien (Titelverbindung aus Beispiel A.2.) vorlegen, 22,4 g Essigsäureanhydrid zutropfen und über Nacht bei Raumtemperatur rühren. Einengen und als Rohprodukt weiter umsetzen.

### A.4. 8-Acetyl-8-azabicyclo[4.3.0]non-2-en

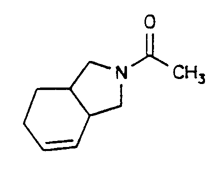

33,1 g (0,2 mol) N-[(E)-2,4-Pentadienyl]-N-(2-propenyl)-acetamid (Titelverbindung aus Beispiel A.3.) in 200 ml Xylol lösen, 15 min einen kräftigen Stickstoffstrom durchleiten, 0,1 g 4-Hydroxyanisol zusetzen, dann über Nacht zum Rückfluß erhitzen. Einengen, im Hochvakuum destillieren.
- Ausbeute:: 23,1 g (70 % der Theorie bezogen auf die Titelverbindung aus Beispiel A.2.)
- Siedepunkt:: 88-93 C bei 0,05 mbar

### A.5. 8-Azabicyclo[4.3.0]non-2-en

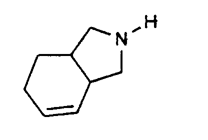

16,5 g (0,1 mol) 8-Acetyl-8-azabicyclo[4.3.0]non-2-en (Titelverbindung aus Beispiel A.4.) in einer Mischung aus 100 ml 45 %-iger Natronlauge, 50 ml Wasser und 100 ml 1,2-Ethandiol 3 h zum Rückfluß erhitzen. Nach Ablrühlung viermal mit je 50 ml Diethylether extrahieren. Vereinigte organische Phasen mit Natriumsulfat trocknen, im Hochvakuum destillieren.
- Ausbeute:: 6,6 g (54 % der Theorie)
- Siedepunkt:: 36-44 C bei 0,35 mbar
¹H-NMR (CDCl₃): δ = 5,79 (m, 1H); 5,74 (m, 1H); 3,02-3,17 (m, 2H); 2,47-2,72 (m, 2H); 2,06-2,30 (m, 2H); 1,91-2,06 (m, 2H); 1,68 (m, 1H); 1,45 ppm (m, 1H).

### Beispiel B:

### (1RS,2RS,6SR)-8-Azabicyclo[4.3.0]non-4-en-2-carbonsäureethylester (Diastereomer A) und

### (1RS,2RS,6RS)-8-Azabicyclo[4.3.0]non-4-en-2-carbonsäureethylester (Diastereomer B)

### B.1. N-[(E)-2,4-Pentadienyl]-phthalimid

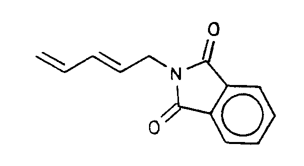

185 g (1,0 mol) Kaliumphthalimid in 800 ml DMF vorlegen. Unter Rühren 147 g (1,0 mol) (E)-1-Brom-2,4-pentadien (Titelverbindung aus Beispiel A.1.) zutropfen, dabei die Innentemperatur durch Kühlen unter 30 C halten. Uber Nacht bei Raumtemperatur rühren. Anschließend den Ansatz unter Rühren auf 1,6 l Eiswasser gießen, den Niederschlag absaugen, mit Wasser waschen, bei Raumtemperatur bis zum Erreichen der Gewichtskonstanz trocknen.
- Ausbeute:: 177-200 g (83-94 % der Theorie)
- Schmelzpunkt:: 118-121 C (Probe aus Ethanol umkrist.)
¹H-NMR (CDCl₃): δ = 7,85 und 7,72 (m, 4H, Aryl-H); 6,2-6,4 (m, 2H, H an C-3 und C-4; 5,75 (dt, 1H, H an C-2, J = 14 und 6 Hz); 5,20 (d, 1H, Hₐ an C-5, J = 15 Hz); 5,10 (d, 1H, H_{b} an C-5, J = 8 Hz); 4,33 ppm (d, 2H, H an C-1, J = 6 Hz).

### B.2. (E)-1-Amino-2,4-pentadien

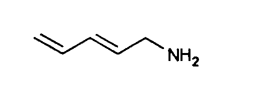

In einer 21-Destillationsapparatur mit 10 cm Vigreuxkolonne werden 400 g Bis-(2-aminoethyl)-amin und 213 g (1.0 mol) N-[(E)-2,4-Pentadienyl]-phthalimid (Titelverbindung aus Beispiel B. 1.) vorgelegt und bei 60 mbar zum Sieden erhitzt. Das Produkt destilliert im Bereich von 45-60 C bei 60 mbar. Zur Stabilisierung werden dem Destillat 10-20 ppm 4-Hydroxyanisol zugesetzt.
- Ausbeute:: 71-80 g (86-96 % der Theorie)

### B.3. (E)-4-[(E)-2,4-Pentadienylamino]-2-butensäureethylester

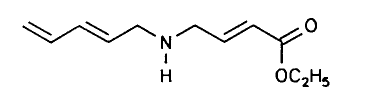

41,6 g (0,5 mol) (E)-1-Amino-2,4-pentadien (Titelverbindung aus Beispiel B.2.) und 50,6 g (0,5 mol) Triethylamin in 250 ml THF bei 0 C vorlegen und 96,5 g (0,5 mol) (E)-4-Brom-2-butensäureethylester in 250 ml THF gelöst zutropfen. Innentemperatur durch Eiskühlung unter 5 C halten. 5 h bei 0 C anschließend über Nacht bei Raumtemperatur rühren. 500 ml MTBE, dann 500 ml 1M Natronlauge zugeben, schütteln, Phasentrennung, wäßrige Phase einmal mit 100 ml MTBE extrahieren, vereinigte organische Phasen mit Natriumsulfat trocknen, 100 ml Toluol und 0,1 g 4-Hydroxyanisol zusetzen, einengen (dabei Temperaturen über 40 C vermeiden). Rückstand an 1 kg Kieselgel (63-200 µm) mit Cyclohexan/Aceton 2:1 säulenchromatographisch reinigen. Vor dem Einengen erneut 0,1 g 4-Hydroxyanisol zusetzen und beim Einengen Temperaturen über 40 C vermeiden.
- Ausbeute:: 52,7-58,6 g (54-60 % der Theorie) gelbliches Öl
- Rf =: 0,24
¹H-NMR (CDCl₃): δ = 6,99 (dt, 1H, J = 15 und 5,5 Hz); 6,1-6,45 (m, 2H); 5,98 (d, 1H, J = 15 Hz); 5,75 (dt, 1H, J = 15 und 6,5 Hz), 5,18 (d, 1H, J = 15 Hz); 5,06 (d, 1H, J = 10 Hz); 4,19 (q, 2H); 3,42 (dd, 2H); 3.31 (d 2H); 1,29 ppm (t, 3H).

### B.4. (1RS,2RS,6SR)-8-tert.-Butyloxycarbonyl-8-azabicyclo[4.3.0]non-4-en-2-carbonsäureethylester (Diastereomer A) und

### (1RS,2RS,6RS)-8-tert.-Butyloxycarbonyl-8-azabicyclo[4.3.0]non-4-en-2-carbonsäureethylester (Diastereomer B)

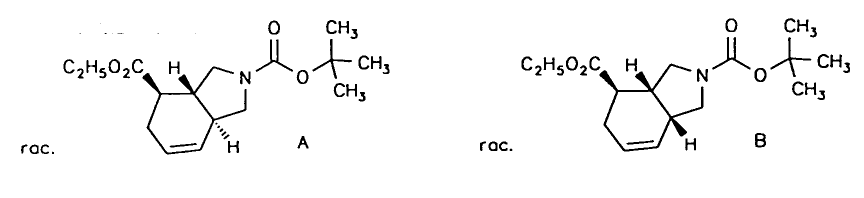

97,5 g (0,5 mol) (E)-4-[(E)-2,4-Pentadienylamino]-2-butensäureethylester (Titelverbindung aus Beispiel B.3.) in 250 ml Toluol gelöst vorlegen. 114,5 g (0,525 mol) Di-tert.-butyl-dicarbonat in 250 ml Toluol gelöst zutropfen, über Nacht bei Raumtemperatur rühren. Anschließend 15 min einen kräftigen Stickstoffstrom durchleiten, 0,1 g 4-Hydroxyanisol zusetzen, dann 6 h zum Rückfluß erhitzen. Einengen, Rückstand an 1 kg Kieselgel (63-200 µm) mit Cyclohexan/Aceton 8:1 säulenchromatographisch reinigen.
- Ausbeute:: 109-134 g (74-91 % der Theorie) gelbliches Öl; Gemisch aus zwei Diastereomeren A und B im Verhältnis A:B = 4:1
- Rf =: 0,25
¹H-NMR (Cl₂DC-CDCl₂; 80 C): δ = 5,77 (m, 1H(A) und 1H(B)); 5,68 (m, 1H(A) und 1H(B)); 4,14 (m, 2H(A) und 2H(B)); 3,65 (m, 2H(A) und 1H(B)); 3,48 (dd, 1H(B)); 3,27 (dd, 1H(B)); 3,00 (m, 1H(A) und 1H(B)); 2,85 (dd, 1H(A)); 2,76 (m, 1H(B)); 2,60 (m, 1H(A)); 2,25-2,55 (m, 3H(A) und 4H(B)); 1,93 (m, 1H(A)); 1,51 (s, 9H(B)); 1,44 (s, 9H(A)); 1,25 ppm (t, 3H(A) und 3H(B)).

### B.5. (1RS,2RS,6SR)-8-Azabicyclo[4.3.0]non-4-en-2-carbonsäureethylester (Diastereomer A) und

### (1RS,2RS,6RS)-8-Azabicyclo[4.3.0]non-4-en-2-carbonsäureethylester (Diastereomer B)

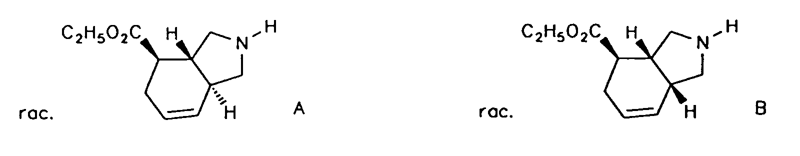

6,0 g (20 mmol) der Titelverbindung aus Beispiel B.4. in 20 ml Dioxan vorlegen. 20 ml konz. Salzsäure unter Kühlung so zutropfen, daß die Innentemperatur 30 C nicht übersteigt. Nach vollständiger Zugabe 10 min nachrühren. 40 ml Methylenchlorid zugeben und 40 ml 20 %-ige eisgekühlte Natronlauge unter Eiskühlung zutropfen. Organische Phase abtrennen, wäßrige Phase einmal mit Methylenchlorid extrahieren, vereinigte organische Phasen mit Natriumsulfat trocknen, einengen. 3,0 g Rohprodukt an 100 g Kieselgel (63-200 µm) mit Cyclohexan/Ethanol/17 %-iges wäßriges Ammoniak (1:2:0,1) säulenchromatographisch reinigen.
- Ausbeute:: 0,8 g Diastereomer A und
0,8 g Diastereomer B
- Rf =: 0,79 Titelverbindung aus Beispiel B.4.
0,21 Diastereomer B
0,11 Diastereomer A
¹H-NMR (CDCl₃):
Diastereomer A: δ = 5,83 (d, 1H); 5,69 (m, 1H); 4,15 (q, 2H); 3,21-3,38 (m, 2H); 2,52-2,89 (m, 3H); 2,21-2,52 (m, 3H); 1,95 (m, 1H); 1,28 ppm (t, 3H).
Diastereomer B: δ = 5,64-5,87 (m, 2H); 4,16 (q, 2H); 3,14-3,33 (m, 2H); 2,82 (dd, 1H); 2,15-2,74 (m, 6H); 1.28 ppm (t, 3H).

### Beispiel C:

### (1SR,2RS,6SR)-2-Ethyloxycarbonylamino-8-azabicyclo[4.3.0]non-4-en

### C.1. (1RS,2RS,6SR)-8-tert.-Butyloxycarbonyl-8-azabicyclo-[4.3.0]non-4-en-2-carbonsäure

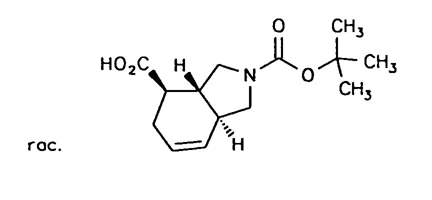

30,8 g (0,55 mol) Kaliumhydroxid in 500 ml Wasser gelöst vorlegen. 147,7 g (0,5 mol) der Titelverbindung aus Beispiel B.4. in 500 ml Methanol gelöst zugeben und 8 h bei 60 C unter einer Stickstoffatmosphäre rühren. Nach Abkühlung Reaktionslösung mit 500 ml Wasser verdünnen und unter Rühren 125 ml Essigsäure langsam zugießen. Nach vollständiger Zugabe 30 min im Eisbad stehen lassen, Niederschlag absaugen, mit Wasser nachwaschen, bei 50 C bis zur Gewichtskonstanz trocknen.
- Ausbeute:: 84-98 g (63-73 % der Theorie)
- Schmelzpunkt:: 174-176 C (Probe aus Isopropanol/Wasser 1:1 umkristallisiert.)
¹H-NMR (Cl₂DC-CDCl₂; 80 C): δ = 5,83 (m, 1H, H an C-5); 5,74 (m, 1H, H an C-4); 3,65-3,80 (m, 2H, Hₐ an C-7 und Hₐ an C-9); 3,09 (dd, 1H, H_{b} an C-9); 2,92 (dd, 1H, H_{b} an C-7); 2,70 (m, 1H, H an C-2); 2,35-2,60 (m, 3H, Hₐ und H_{b} an C-3 und H an C-6); 2,01 (m, 1H, H an C-1); 1,5 ppm (s, 9H).

### C.2. (1SR,2RS,6SR)-8-tert.-Butyloxycarbonyl-2-ethyloxycarbonylamino-8-azabicyclo[4.3.0]non-4-en

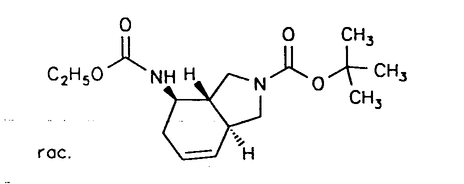

53,3 g, (0,2 mol) der Titelverbindung aus Beispiel C.1. und 22,2 g (0,22 mol) Triethylamin in 200 ml wasserfreiem THF gelöst vorlegen. Unter Kühlung mit einer Eis/Kochsalz-Mischung 22,8 g (0,21 mol) Chlorameisensäureethylester in 40 ml THF gelöst so zutropfen, daß die Innentemperatur - 10 C nicht überschreitet. Nach vollständiger Zugabe 1 h bei tiefer Temperatur nachrühren. Anschließend eine eisgekühlte Lösung von 15,6 g (0,24 mol) Natriumazid in 50 ml Wasser unter kräftigem Rühren so zutropfen, daß die Innentemperatur - 10 C nicht überschreitet. Nach vollständiger Zugabe 30 min bei tiefer Temperatur nachrühren. Anschließend nacheinander 300 ml Wasser und 400 ml Toluol zugeben.

Die organische Phase abtrennen, mit Natriumsulfat trocknen, bei 15 mbar auf die Hälfte des ursprünglichen Volumens einengen (Badtemperatur unter 25 C). Zugabe von 100 ml Ethanol, unter Rühren langsam aufheizen (in dem Maße, wie es die Stickstoffentwicklung zuläßt) und nach beendeter Stickstoffentwicklung 4 h zum Rückfluß kochen. Einengen und Rohprodukt aus Methanol/Wasser 85:15 umkristallisieren, bei 50 C bis zur Gewichtskonstanz trocknen.
- Ausbeute:: 24,2-28,5 g (39-46 % der Theorie) der Titelverbindung
- Schmelzpunkt:: 120-122 C
¹H-NMR (CDCl₃): δ = 5,78 und 5,73 (2d, 1H, H an C-5); 5,64 (m, 1H, H an C-4); (4,59 br. s, 1H, NH); 4,12 (m, 2H, Ethoxy-CH₂); 3,90 (m, 1H, H an C-2); 3,74 und 3,67 (2m, 1H, Hₐ an C-7); 3,67 und 3,56 (2m, 1H, Hₐ an C-9); 3,12 (m, 1H, H_{b} an C-9); 2,92 (m, 1H, H_{b} an C-7); 2,67 (m, 1H, Hₐ an C-3); 2,49 (m, 1H, H an C-6); 1,95 (m, 1H, H_{b} an C-3); 1,83 (m, 1H, H an C-1); 1,46 (s, 9H); 1,24 ppm (m, 3H, Ethoxy-CH₃).

Die wässrige Phase durch Zugabe von 10 %-iger Salzsäure auf einen pH von 2-3 einstellen, 30 min im Eisbad stehen lassen, den Niederschlag absaugen, mit Wasser waschen, bei 50 C bis zur Gewichtskonstanz trocknen.
- Ausbeute:: 16,0-19,2 g (30-36 % der Titelverbindung aus Beispiel C.1.) (zurückgewonnene Ausgangsverbindung)

### C.3. (1SR,2RS,6SR)-2-Ethyloxycarbonylamino-8-azabicyclo[4.3.0]non-4-en

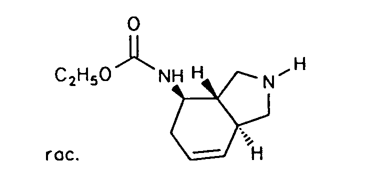

31,0 g (0,1 mol) der Titelverbindung aus Beispiel C.2. in 100 ml einer Mischung aus Methanol/Wasser (1:1) vorlegen (Suspension). 100 ml konz. Salzsäure rasch zulaufen lassen (leicht exotherm bis etwa 40 C, (man erhält eine homogene Lösung) und bis zum Ende der Gasentwicklung (etwa 10 min) nachrühren. 200 ml Eiswasser zugeben und 70 ml 45 %-ige Natronlauge unter Rühren und Eiskühlung zutropfen. Viermal mit je 50 ml Methylenchlorid extrahieren, die vereinigten organischen Phasen mit Natriumsulfat trocknen, einengen, im Hochvakuum Lösungsmittelreste abziehen. Die Substanz wird beim Einengen fest.
- Ausbeute:: 13,7-16,6 g (65-79 % der Theorie) braun-rosafarbener, amorpher Feststoff
- Rf =: 0,81 Titelverbindung aus Beispiel C.2.
0,11 Titelverbindung
Methylenchlorid/Methanol/17 %-iges wäßriges Ammoniak (15:4:0,5)
¹H-NMR (CDCl₃): δ = 5,78 (d, 1H, H an C-5); 5,63 (m, 1H, H an C-4); 4,94 (br.d, ¹H, NH); 4,10 (m, 2H, Ethoxy-CH₂); 3,88 (m, 1H, H an C-2); 3,28 (m, 1H, Hₐ an C-7); 3,19 (m, 1H, Hₐ an C-9); 2,84 (m, 1H, H_{b} an C-9); 2,57-2,62 (m, 2H, Hₐ an C-3 und H_{b} an C-7); 2,43 (m, 1H, H an C-6); 1,95 (m, 1H, H_{b} an C-3): 1,79 (m, 1H, H an C-1); 1,23 ppm (m, 3H, Ethoxy-CH₃).

### Beispiel D:

### (1SR,2RS,6SR)-2-Methylamino-8-azabicyclo[4.3.0]non-4-en

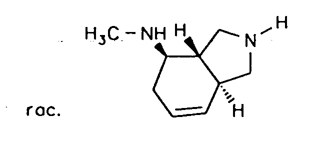

1,9 g (50 mmol) Lithiumaluminiumhydrid in 25 ml wasserfreiem Diethylether in einer Stickstoffatmosphäre vorlegen. 5,25 g (25 mmol) der Titelverbindung aus Beispiel C.3. in 50 ml wasserfreiem Tetrahydrofuran gelöst zutropfen und 3 h zum Rückfluß erhitzen. Weitere 0,95 g (25 mmol) Lithiumaluminiumhydrid zusetzen und nochmals 3 h zum Rückfluß erhitzen. Unter Eiskühlung langsam Wasser zutropfen, bis ein weißer Niederschlag entstanden ist. Niederschlag absaugen, zweimal mit je 100 ml Ethanol auskochen. Ethanolextrakte mit der Mutterlauge der Reaktion vereinigen, 50 ml Toluol zusetzen, einengen, Lösungsmittelreste im Hochvakuum abziehen.
- Ausbeute:: 1,95 g (77 % der Theorie) amorpher Feststoff
- Rf =: 0,11
Methylenchlorid/Methanol/17 %-iges wäßriges Ammoniak (2:4:1)
¹H-NMR (CDCl₃): δ = 5,77 (d, 1H, H an C-5); 5,67 (m, 1H, H an C-4); 3,33 (dd, 1H, Hₐ an C-7); 3,26 (dd, 1H, Hₐ an C-9); 2,73-2,82 und 2,54-2,63 (2m, 4H, H an C-2, Hₐ an C-3, H_{b} an C-7 und H_{b} an C-9); 2,41 (s, 3H, CH₃N); 2,34 (m, 1H, H an C-6); 1,90 (m, 1H, H_{b} an C-3); 1,70 ppm (m, 1H, H an C-1).

### Beispiel E:

### (1RS,2RS,6SR)-2-Hydroxymethyl-8-azabicyclo[4.3.0]non-4-en

### E.1. (1RS,2RS,6SR)-8-tert.-Butyloxycarbonyl-2-hydroxymethyl-8-azabicyclo[4.3.0]non-4-en (Diastereomer A) und

### (1RS,2RS,6RS)-8-tert.-Butyloxycarbonyl-2-hydroxymethyl-8-azabicyclo[4.3.0]non-4-en (Diastereomer B)

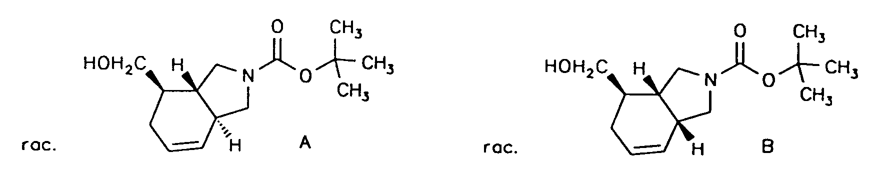

29,5 g (0,1 mol) der Titelverbindung aus Beispiel B.4. in 200 ml wasserfreiem 1,2-Dimethoxyethan in einer Stickstoffatmosphäre vorlegen. Bei einer Innentemperatur < - 65 C 150 ml einer 1,5 m DIBAH-Lösung in Toluol (0,225 mol) zutropfen. Nach vollständiger Zugabe Kühlbad entfernen und auf Raumtemperatur kommen lassen. 2 h bei Raumtemperatur nachrühren.

Unter kräftigem Rühren 60 ml Methanol zutropfen (exotherme Reaktion); Innentemperatur durch Kühlung mit einem Kaltwasserbad zwischen 35 und 45 C halten. Anschließend 20 ml 5 %-ige Natronlauge zutropfen. Nach vollständiger Zugabe 10 min nachrühren. Niederschlag absaugen, zweimal unter Rühren mit je 150 ml Ethanol auskochen, Ethanolextrakte und Reaktionslösung vereinigen, einengen, im Hochvakuum Lösungsmittelreste abziehen, Rückstand an 250 g Kieselgel (63-200 µm) mit Cyclohexan/Aceton (4:1) säulenchromatographisch reinigen.
- Ausbeute:: 12,9-17,7 g (51-70 % der Theorie) gelbliches Öl; Gemisch der Diastereomeren A und B im Verhältnis 4:1
- Rf =: 0,36 Titelverbindung aus Beispiel B.4.
0,12 Titelverbindung A und B
Das Rohprodukt wird nach längerem Stehen fest. Durch Umkristallisieren aus Ether/Petrolether kann eine diastereomerenreine Probe des Hauptdiastereomeren A erhalten werden.
¹H-NMR (CDCl₃): (Diastereomer A) δ = 5,67-5,82 (m, 2H, H an C-4 und C-5); 3,50-3,77 (m, 4H, Hₐ an C-7, Hₐ an C-9 und Hydroxymethyl-CH₂); 3,02 (dt, 1H, H_{b} an C-9); 2,85 (m, 1H, H_{b} an C-7); 2,2-2,4 (m, 3H); 1,87-2,00 (m, 3H); 1,62 (m, 1H, H an C-1); 1,46 ppm (s, 9H).

### E.2. (1RS,2RS,6SR)-2-Hydroxymethyl-8-azabicyclo[4.3.0]non-4-en

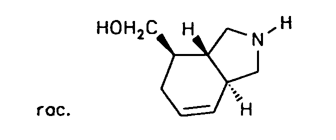

2,5 g (10 mmol) der Titelverbindung A aus Beispiel E.1. in 10 ml Methanol vorlegen. 10 ml konz. Salzsäure rasch zulaufen lassen und 30 min nachrühren. Mit Wasser auf das doppelte Volumen verdünnen dann 45 %-ige Natronlauge unter Rühren und Eiskühlung zutropfen, bis zu einem pH-Wert ≧ 12. Einengen, Rückstand zweimal unter Rühren mit Ethanol auskochen, Ethanolextrakte einengen, im Hochvakuum Lösungsmittelreste abziehen.
- Ausbeute:: 2,1 g (Produkt enthält NaCl-Rückstände)
- Rf =: 0,20
Methylenchlorid/Methanol/17 %-iges wäßriges Ammoniak (2:4:1)
¹H-NMR (d₆-DMSO): δ = 5,76 (d, 1H); 5,62 (d, 1H); 3,47-3,56 (m, 2H, Hₐ an C-7 und Hₐ an C-9); 3,32-3,47 (m, 1H, Hₐ von Hydroxymethyl-CH₂); 3,23-3,32 (m, 1H, H_{b} von Hydroxymethyl-CH₂); 2,77 (t, 1H, H_{b} an C-9); 2,64 (t, 1H, H_{b} an C-7); 2,10-2,24 (m, 2H, Hₐ an C-3 und H an C-6); 1,77-1,88 (m, 1H, H_{b} an C-3); 1,69 (m, 1H, H an C-2); 1,40 ppm (m, 1H, H an C-1).

### Beispiel F:

### (1RS,2RS,6SR)-2-Ethyloxycarbonylaminomethyl-8-azabicyclo[4.3.0]non-4-en

### F.1. (1RS,2RS,6SR)-8-tert.-Butyloxycarbonyl-2-(4-toluolsulfonyloxymethyl)-8-azabicyclo[4.3.0]non-4-en (Diastereomer A) und

### (1RS,2RS,6RS)-8-tert.-Butyloxycarbonyl-2-(4-toluolsulfonyloxymethyl)-8-azabicyclo[4.3.0]non-4-en (Diastereomer B)

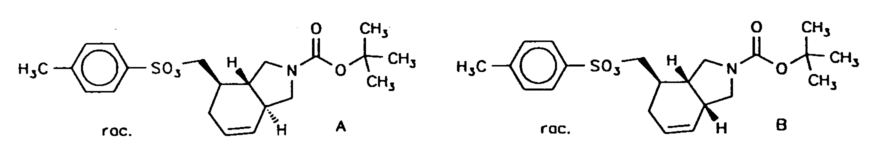

12,7 g (0,05 mol) der Titelverbindung aus Beispiel E.1. (Rohgemisch der Diastereomere A und B) in 25 ml wasserfreiem Pyridin vorlegen und auf - 15 C kühlen. 11,0 g (0,0575 mol) 4-Toluolsulfonsäurechlorid portionsweise so zugeben, daß die Innentemperatur - 5 C nicht übersteigt. Nach vollständiger Zugabe 2 h bei einer Temperatur von - 5 bis - 15 C, dann 3 h bei Raumtemperatur nachrühren. 5 g Eis zugeben, 5 min rühren, auf 50 ml Wasser geben, Niederschlag absaugen, mit Wasser waschen, bei 50 C bis zur Gewichtskonstanz trocknen.
- Ausbeute:: 14,4-16,3 g (71-80% der Theorie) blaßrosafarbener Feststoft Gemisch der Diastereomeren A und B
Durch Umkristallisieren aus Methanol kann eine diastereomerenreine Probe des Hauptdiastereomeren A erhalten werden.
- Schmelzpunkt:: 111-113 C
¹H-NMR (CDCl₃): (Diastereomer A) δ = 7,79 (m, 2H, Aryl-H); 7,36 (d, 2H, Aryl-H); 5,74 und 5,78 (2d, 1H, H an C-5); 5,64 (m, 1H, H an C-4); 3,87-3,97 (m, 2H, Tosyl-OCH₂-); 3,59 und 3,67 (2dd, 1H, Hₐ an C-7); 3,48 (dd, 1H, Hₐ an C-9); 2,78-2,96 (m, 2H, H_{b} an C-7 und H_{b} an C-9); 2,47 (s, 3H, Aryl-CH₃); 2,22-2,36 (m, 2H, Hₐ an C-3 und H an C-6): 2,06 (m, 1H, H an C-2); 1,80-1,98 (m, 1H, H_{b} an C-3); 1,59 (m, 1H, H an C-1); 1,45 und 1,47 ppm (2s, 9H).

### F.2. (1RS,2RS,6SR)-8-tert.-Butyloxycarbonyl-2-ethyloxycarbonylaminomethyl-8-azabicyclo[4.3.0]non-4-en (Diastereomer A) und

### (1RS,2RS,6RS)-8-tert.-Butyloxycarbonyl-2-ethyloxycarbonylaminomethyl-8-azabicyclo[4.3.0]non-4-en (Diastereomer B)

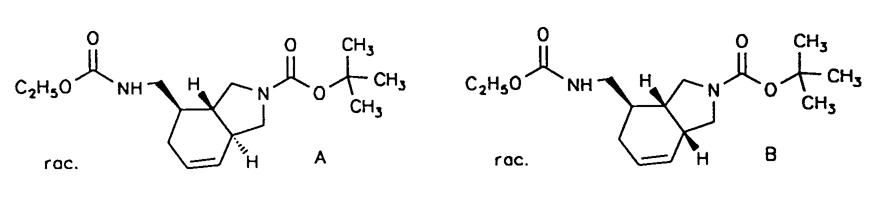

20,5 g (0,05 mol) der Titelverbindung aus Beispiel F.1. (Rohgemisch der Diastereomere A und B) und 6,5 g (0,1 mol) Natriumazid in 100 ml DMF 4 h auf 70 C erhitzen. Reaktionslösung auf 200 ml Wasser geben, einmal mit 200 ml Petrolether extrahieren, die Petroletherphase einmal mit 50 ml Wasser waschen, mit Natriumsulfat trocknen und bei Raumtemperatur einengen.

Den Rückstand in 80 ml THF aufnehmen und 13,1 g (0,05 mol) Triphenylphosphin in 80 ml THF gelöst zutropfen. Nach vollständiger Zugabe 20 h bei Raumtemperatur rühren, dann 150 ml Wasser langsam zutropfen, nach vollständiger Zugabe 15 min nachrühren. Unter Kühlung Salzsäure zutropfen (konz. HCl/Wasser 1:3), bis ein pH-Wert von 3-4 erreicht ist, THF bei Raumtemperatur im Vakuum abziehen, Reaktionslösung auf 0 C kühlen, ausgefallenes Triphenylphosphinoxid absaugen (oder mit MTBE aufnehmen, falls ölig).

Wäßrige Phase durch Zugabe von 10 %-iger Natronlauge auf einen pH-Wert ≧ 12 einstellen, zweimal mit je 100 ml Methylenchlorid extrahieren, vereinigte Extrakte mit Natriumsulfat trocknen, anschließend 6,0 g (0,06 mol) Triethylamin zugeben, unter Rühren 6,0 g (0,055 mol) Chlorameisensäureethylester in 20 ml Methylenchlorid gelöst zutropfen, uber Nacht bei Raumtemperatur rühren, Reaktionslösung einmal mit 100 ml Wasser waschen, mit Natriumsulfat trocknen und einengen.

23 g Rohprodukt an 100 g Kieselgel (63-200 µm) mit Cyclohexan/ Aceton (4:1) säulenchromatographisch reinigen.
- Ausbeute:: 12,4 g (76 % der Theorie) zähes Öl
Gemisch der Diastereomeren A und B
- Rf-Werte (Cyclohexan/Aceton 2:1):: 0,32 Diastereomer A
0,29 Diastereomer B
Die Diastereomere A und B werden an 250 g Kieselgel (35-70 µm) mit Cyclohexan/Aceton (8:1) säulenchromatographisch getrennt.
- Ausbeute:: 4,3 g (26 % der Theorie) Diastereomer A (zähes Öl)
2,4 g (15 % der Theorie) Mischfraktion
0,6 g (4 % der Theorie) Diastereomer B
¹H-NMR (Cl₂DC-CDCl₂; 80 C):
Diastereomer A: δ = 5,75 (d, 1H, H an C-5); 5,66 (m, 1H, H an C-4); 4,67 (br, 1H, NH); 4,08 (q, 2H, Ethoxy-CH₂); 3,62 (br, 2H, Hₐ an C-7 und Hₐ an C-9); 3,19 (br, 1H, Hₐ an CH₂-NH); 3,05 (br, H_{b} an CH₂-NH); 2,96 (dd, 1H, H_{b} an C-9); 2,81 (dd, 1H, H_{b} an C-7); 2,24-2,34 (m, 2H, Ha an C-3 und H an C-6); 1,78-1,94 (m, 2H, H an C-2 und H_{b} an C-3); 1,54 (m, 1H, H an C-1); 1,43 (s, 9H); 1,22 ppm (t, 3H, Ethoxy-CH₃).
Diastereomer B: δ = 5,69 (m, 1H, H an C-4); 5,57 (m, 1H, H an C-5); 4,65 (br, 1H, NH); 4,08 (q, 2H, Ethoxy-CH₂); 3,52 (dd, 1H, Hₐ an C-7); 3,41 (dd, 1H, Hₐ an C-9); 3,29 (dd, 1H, H_{b} an C-9); 3,24 (dd, 1H, Hₐ an CH₂-NH); 3,03-3,12 (m, 2H, H_{b} an C-7 und H_{b} an CH₂-NH); 2,68 (m, 1H, H an C-6); 2,12-2,22 (m, 2H, H an C-1 und Hₐ an C-3); 1,74-1,87 (m, 2H, H an C-2 und H_{b} an C-3); 1,43 (s, 9H); 1,22 ppm (t, 3H, Ethoxy-CH₃).

### F.3. (1RS,2RS,6SR)-2-Ethyloxycarbonylaminomethyl-8-azabicyclo [4.3.0]non-4-en

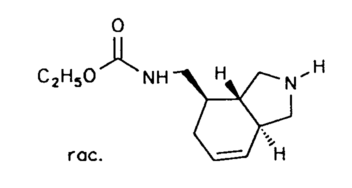

1,6 g (5,7 mmol) der Titelverbindung A aus Beispiel F.2. in 10 ml Methanol vorlegen. 8 ml konz. Salzsäure rasch zulaufen lassen und 30 min nachrühren. Mit Wasser auf das doppelte Volumen verdünnen dann 45 %-ige Natronlauge unter Rühren und Eiskühlung zutropfen, bis zu einem pH-Wert von ≧ 12. Viermal mit Methylenchlorid extrahieren, die vereinigten organischen Phasen mit Natriumsulfat trocknen, einengen, im Hochvakuum Lösungsmittelreste abziehen.
- Ausbeute:: 0,8 g (63 % der Theorie) zähes Öl
- Rf =: 0,16
Methylenchlorid/Methanol/17 %-iges wäßriges Ammoniak (15:4:0,5)
¹H-NMR (CDCl₃): δ = 5,81 (d, 1H, H an C-5); 5,67 (m, 1H, H an C-4); 5,00 (br, 1H, NH); 4,10 (q, 2H, Ethoxy-CH₂); 3,18-3,28 und 3,08 (m, 3H und m, 1H: Hₐ an C-7, Hₐ an C-9, Hₐ und H_{b} an CH₂-NH-CO); 2,67 (dd, 1H, H_{b} an C-9); 2,53 (dd, 1H, H_{b} an C-7); 2,34 (m, 1H, Hₐ an C-3); 2,25 (m, 1H, H an C-6); 1,79-1,96 (m, 2H, H an C-2 und H_{b} an C-3); 1,50 (m, 1H, H an C-1); 1,24 ppm (t, 3H, Ethoxy-CH₃).

### Beispiel G:

### (1RS,2SR,6RS)-2-Hydroxymethyl-8-azabicyclo[4.3.0]non-4-en

### G.1. (E)-1-tert.-Butyloxycarbonylamino-2,4-pentadien

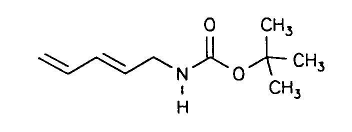

8,3 g (0,1 mol) (E)-1-Amino-2,4-pentadien (Titelverbindung aus Beispiel B.2.) in 50 ml MTBE vorlegen und 20 mg 4-Hydroxyanisol zusetzen. Anschließend bei einer Innentemperatur von 20-30 C 22,9 g (0,105 mol) Di-tert.-butyl-dicarbonat in 50 ml MTBE gelöst zutropfen. Nach vollständiger Zugabe 20 h bei Raumtemperatur rühren. Einengen, Reste von Di-tert.-butyl-dicarbonat im Hochvakuum bei 40 C abziehen.
- Ausbeute:: 18,9 g (Rohprodukt) farbloses Öl
- Rf =: 0,25
Cyclohexan/Aceton (4:1)
¹H-NMR (CDCl₃): δ = 6,05-6,43 (m, 2H, H an C-3 und C-4); 5,68 (dd, 1H, H an C-2, J= 14 und 6 Hz); 5,17 (dd, 1H, Hₐ an C-5, J = 16 Hz); 5,07 (dd, 1H, H_{b} an C-5, J = 10 Hz); 4,75 (br, 1H, NH); 3,77 (t, 2H, H an C-1); 1,45 ppm (s, 9H).

### G.2. (1RS,2RS,6RS)-2-tert.-Butyloxycarbonylaminomethyl-7,9-dioxo-8-oxabicyclo[4.3.0]non-3-en

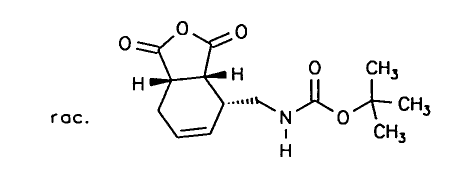

83,2 g (1,0 mol) (E)-1-Amino-2,4-pentadien (Titelverbindung aus Beispiel B.2.) in 250 ml MTBE vorlegen und 0,1 g 4-Hydroxyanisol zusetzen. Anschließend bei einer Innentemperatur von 20-30 C 229,2 g (1,05 mol) Di-tert.-butyl-dicarbonat in 250 ml MTBE gelöst zutropfen. Nach vollständiger Zugabe 20 h bei Raumtemperatur rühren. Reaktionsgemisch einengen und in 1 l Toluol aufnehmen. 103,0 g (1,05 mol) Maleinsäureanhydrid zusetzen und 24 h bei einer Innentemperatur von 60 C rühren. Niederschlag absaugen, mit Toluol waschen und bei 50 C bis zur Gewichtskonstanz trocknen.
- Ausbeute:: 208,2 g (74 % der Theorie)
weißer, kristalliner Feststoff
- Schmelzpunkt:: 157-159 C
¹H-NMR (d₆-DMSO): δ = 5,81 (m, 1H, H an C-4); 5,59 (d, 1H, H an C-3); 3,77 (dd, 1H Hₐ an CH₂-NH); 3,44 (m, 2H, H an C-1 und H_{b} an CH₂-NH); 2,94 (m, 1H, H an C-2); 2,66 (m, 1H, H an C-6); 2,16 (m, 1H, Hₐ an C-5); 2,06 (m, 1H, H_{b} an C-5); 1,43 ppm (s, 9H).

### G.3. (1RS,2SR,6RS)-9-Oxo-8-azabicyclo[4.3.0]non-4-en-2-carbonsäuremethylester

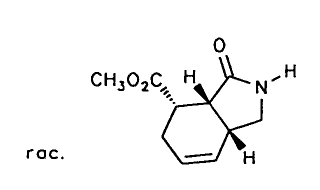

83,2 g (1,0 mol) (E)-1-Amino-2,4-pentadien (Titelverbindung aus Beispiel B.2.) in 250 ml THF vorlegen und 0,1 g 4-Hydroxyanisol zusetzen. Anschließend bei einer Innentemperatur von 20-30 C 229,2 g (1,05 mol) Di-tert.-butyl-dicarbonat in 250 ml THF gelöst zutropfen. Nach vollständiger Zugabe 20 h bei Raumtemperatur rühren. 103,0 g (1,05 mol) Maleinsäureanhydrid zusetzen und 5 h zum Rückfluß erhitzen. Einengen und den Rückstand in 500 ml Methanol aufnehmen, 30 ml p-Toluolsulfonsäure zusetzen, dann erneut 5 h zum Rückfluß erhitzen. Nach Abkühlung unter Eiskühlung und Rühren eine Lösung von 20 g Natriumcarbonat in 500 ml Wasser gelöst rasch zutropfen, Ansatz noch 30 min im Eisbad stehen lassen, Niederschlag absaugen, mit wenig Wasser waschen, bei 50 C bis zur Gewichtskonstanz trocknen.
- Ausbeute:: 125-148 g (64-76 % der Theorie)
weißer, kristalliner Feststoff
- Schmelzpunkt:: 190-193 C
¹H-NMR (d₆-DMSO): δ = 7,50 (s, 1H, NH); 5,77 (m, 1H, H an C-4); 5,56 (m, 1H, H an C-5); 3,60 (s, 3H, CH₃O); 3,42 (dd, 1H, Hₐ an C-7); 3,16 (dd, 1H, H an C-1); 3,00 (m, 1H, H an C-6); 2,88 (dd, 1H, H_{b} an C-7); 2,67 (m, 1H, H an C-2); 2,02-2,18 ppm (m, 2H, Hₐ und H_{b} an C-3).

### G.4. (1RS,2SR,6RS)-2-Hydroxymethyl-8-azabicyclo[4.3.0]non-4-en

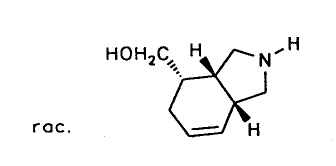

19,6 g (0,1 mol) der Titelverbindung aus Beispiel G.3. in 100 ml THF unter Inertgasatmosphäre vorlegen (Suspension). 100 ml (0,15 mol) 1,5 m DIBAH-Lösung in Toluol bei einer Innentemperatur von 10-20 C zutropfen. Die so erhaltene klare, homogene Lösung zu einer Suspension von 1,9 g Lithiumalanat in 50 ml THF zutropfen. Nach vollständiger Zugabe 15 min bei Raumtemperatur, anschließend 30 min bei Rückflußtemperatur rühren. Nach Abkühlung portionsweise 3,8 g (0,1 mol) Lithiumalanat zugeben, dann 24 h zum Rückfluß erhitzen. Nach Abkühlung nacheinander 50 ml Wasser und 10 ml 1M-Natronlauge zutropfen, den Niederschlag absaugen und dreimal mit je 150 ml Ethanol auskochen. Filtrat und Extrakte vereinigen und einengen.
- Ausbeute:: 16,4 g (Produkt enthält Lithium- und Aluminiumhydroxid)
- Rf =: 0,3
Methylenchlorid/Methanol/17 %-iges waßriges Ammoniak (2:4:1)

### Beispiel H:

### (1RS,2SR,6RS)-2-Ethyloxycarbonylaminomethyl-8-azabicyclo[4.3.0]non-4-en

### H.1. (1RS,2SR,6RS)-8-tert.-Butyloxycarbonyl-2-hydroxymethyl-8-azabicyclo[4.3.0]non-4-en

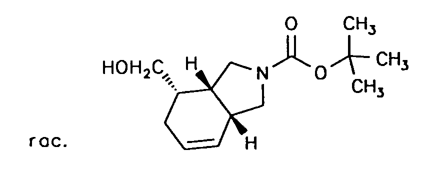

16,4 g Rohprodukt aus Beispiel G.4. (entspricht 0,1 mol der Titelverbindung aus Beispiel G.4.) in 100 ml THF lösen. Bei einer Innentemperatur von 0-5 C 22,9 g (0,105 mol) Di-tert.-butyl-dicarbonat in 100 ml THF gelöst zutropfen, 24 h bei 0 C, anschließend weitere 24 h bei Raumtemperatur rühren. Einengen, Rohprodukt an 250 g Kieselgel (63-200 µm) mit Cyclohexan/Aceton (2:1) säulenchromatographisch reinigen.
- Ausbeute:: 13,7 g (54 % der Theorie über 2 Stufen); zähes Öl
- Rf =: 0,21 Titelverbindung
0,08 Titelverbindung aus Beispiel G.4.

### H.2. (1RS,2SR,6RS)-8-tert.-Butyloxycarbonyl-2-(4-toluolsulfo-nyloxymethyl)-8-azabicyclo[4.3.0]non-4-en

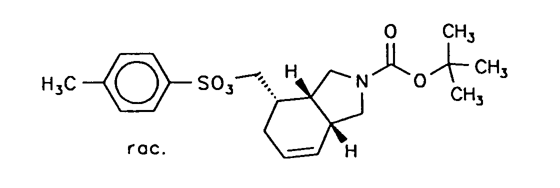

Analog Beispiel F.1. erhält man aus der Titelverbindung aus Beispiel H.1. die Titelverbindung.
- Ausbeute:: 81-83 % der Theorie
- Schmelzpunkt:: 160-162 C
¹H-NMR (CDCl₃): δ = 7,79 (m, 2H, Aryl-H); 7,37 (d, 2H, Aryl-H); 5,67 (m, 1H, H an C-4); 5,47 (m, 1H, H an C-5); 3,78-3,97 (m, 2H, Tosyl-OCH₂-); 3,13-3,42 (m, 3H, CH₂-N); 2,95 (t, 1H, CH₂-N); 2,74 (m, 1H); 2,54 (m, 1H); 2,47 (s, 3H, Aryl-CH₃); 2,32 (m, 1H, H an C-2); 2,06 (m, 1H, Hₐ an C-3); 1,66-1,83 (m, 1H, H_{b} an C-3); 1,44 ppm (s, 9H).

### H.3. (1RS,2SR,6RS)-8-tert.-Butyloxycarbonyl-2-ethyloxycarbonylaminomethyl-8-azabicyclo[4.3.0]non-4-en

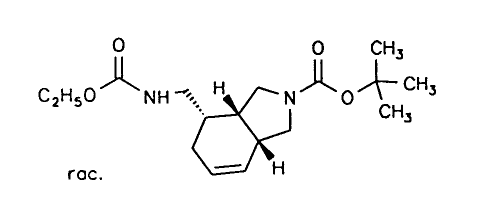

Analog Beispiel F.2. erhält man aus der Titelverbindung aus Beispiel H.2. die Titelverbindung.

Rohprodukt an Kieselgel (63-200 µm) mit Cyclohexan/Aceton (2:1) säulenchromatographisch reinigen.
- Ausbeute:: 76 % der Theorie; klares, zähes Öl
- Rf =: 0,35 (Cyclohexan/Aceton 2:1)
¹H-NMR (Cl₂DC-CDCl₂; 80 C): δ = 5,69 (m, 1H, H an C-4); 5,47 (d, 1H, H an C-5); 4,59 (br, 1H, NH); 4,10 (q, 2H, Ethoxy-CH₂); 3,38 (dd, 1H); 3,32 (m, 1H); 3,24 (m, 1H); 3,01-3,08 (m, 3H); 2,79 (m, 1H); 2,47 (m, 1H); 2,07 (m, 2H); 1,78 (m, 1H); 1,42 (s, 9H); 1,22 ppm (t, 3H, Ethoxy-CH₃).

### H.4. (1RS,2SR,6RS)-2-Ethyloxycarbonylaminomethyl-8-azabicyclo [4.3.0]non-4-en

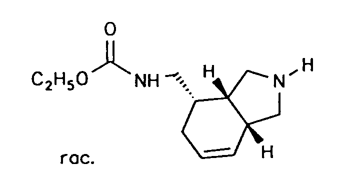

Analog Beispiel C.3. erhält man aus der Titelverbindung aus Beispiel H.3. die Titelverbindung.
- Ausbeute:: 42 % der Theorie
- Rf =: 0,93 Titelverbindung aus Beispiel H.3.
0,23 Titelverbindung
Methylenchlorid/Methanol/17 %-iges wäßriges Ammoniak (15:4:0,5)

### Beispiel I:

### (1SR,2RS,3RS,6SR)-2-Ethyloxycarbonylamino-3-methyl-8-azabicyclo[4.3.0]non-4-en

### I.1. N-[(2E,4E)-2,4-Hexadienyl]-phthalimid

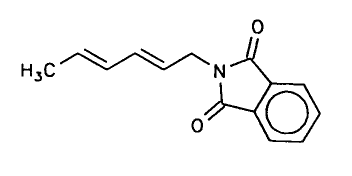

Analog Beispiel B.1. erhält man aus (2E,4E)-1-Brom-2,4-hexadien die Titelverbindung.
- Ausbeute:: 77-79 % der Theorie
- Schmelzpunkt:: 114-117 C (Probe aus Ethanol umkrist.)
¹H-NMR (CDCl₃): δ = 7,85 (m, 2H); 7,72 (m, 2H); 6,25 (dd, 1H); 6,00 (ddd, 1H); 5,5-5,8 (m, 2H); 4,29 (d, 2H); 1,74 ppm (d, 3H).

### I.2. (2E,4E)-1-Amino-2,4-hexadien

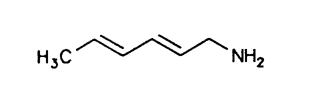

Analog Beispiel B.2. erhält man aus der Titelverbindung aus Beispiel I.1. die Titelverbindung; Siedebereich: 40-70 C bei 16-18 mbar.
- Ausbeute:: 67-83 % der Theorie

### I.3. (E)-4-[(2E,4E)-2,4-Hexadienylamino]-2-butensäureethylester

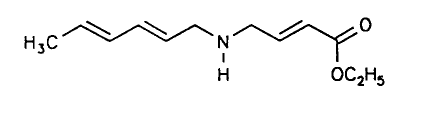

Analog Beispiel B.3. erhält man aus der Titelverbindung aus Beispiel I.2. die Titelverbindung.
- Ausbeute:: 46 % der Theorie
¹H-NMR (CDCl₃): δ = 6,98 (dt, 1H); 5,9-6,25 (m, 3H); 5,5-5,8 (m, 2H); 4,19 (q, 2H); 3,40 (dd, 2H); 3,27 (d, 2H); 1,76 (d, 3H); 1,29 ppm (t, 3H).

### I.4. (1RS,2RS,3RS,6SR)-8-tert.-Butyloxycarbonyl-3-methyl-8-azabicyclo[4.3.0]non-4-en-2-carbonsäureethylester (Diastereomer A) und

### (1RS,2RS,3SR,6RS)-8-tert.-Butyloxycarbonyl-3-methyl-8-azabicyclo[4.3.0]non-4-en-2-carbonsäureethylester (Diastereomer B)

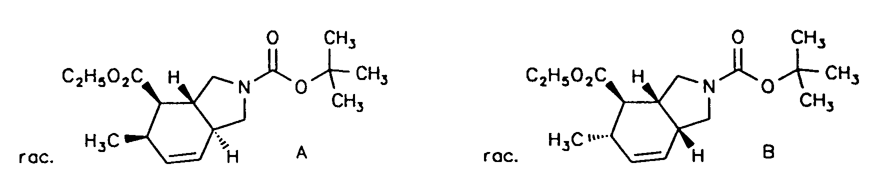

Analog Beispiel B.4. erhält man aus der Titelverbindung aus Beispiel I.3. die Titelverbindungen.
- Ausbeute:: 70 % der Theorie; Gemisch aus 2 Diastereomeren A und B im Verhältnis A:B = 4:1.
- Rf =: 0,49 (Cyclohexan/Aceton 2:1)

### I.5. (1RS,2RS,3RS,6SR)-8-tert.-Butyloxycarbonyl-3-methyl-8-azabicy clo[4.3.0]non-4-en-2-carbonsäure

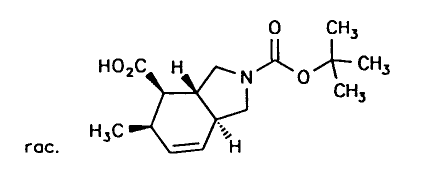

1,17 g (21 mmol) Kaliumhydroxid in 20 ml Wasser gelöst vorlegen. 5,9 g (19 mmol) der Titelverbindung aus Beispiel I.4. in 20 ml Methanol gelöst zugeben und 48 h unter einer Stickstoffatmosphäre zum Rückfluß erhitzen. Einengen, in Wasser aufnehmen, einmal mit Methylenchlorid extrahieren, wässrige Phase mit Essigsäure auf pH 3-4 einstellen, Niederschlag absaugen, mit Wasser waschen, bei Raumtemperatur trocknen, aus Cyclohexan/Aceton 6:1 umkristallisieren.
- Ausbeute:: 2,25 g (42 % der Theorie)
- Schmelzpunkt:: 189 C
¹H-NMR (d₆-DMSO): δ = 5,77 (d, 1H); 5,61 (m, 1H); 3,67 (m, 1H); 3,54 (m, 1H); 2,61-2,95 (m, 4H); 2,30 (m, 1H); 1,82 (m, 1H); 1,40 (s, 9H); 0,90 ppm (d, 3H).

### I.6. (1SR,2RS,3RS,6SR)-8-tert.-Butyloxycarbonyl-2-ethyloxycarbonylamino-3-methyl-8-azabicyclo[4.3.0]non-4-en

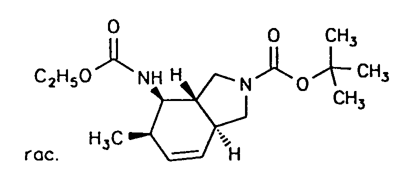

Analog Beispiel C.2. erhält man aus 2,25 g (8 mmol) der Titelverbindung aus Beispiel I.5. die Titelverbindung. Gegenüber Beispiel C.2. geändert: 8 h Rückfluß in Ethanol anstatt 4 h; Reinigung durch Säulenchromatographie an 100 g Kieselgel (63-200 µm) mit Toluol/Essigester (2:1).
- Ausbeute:: 1,6 g (59 % der Theorie) klares Öl
¹H-NMR (CDCl₃): δ = 5,68 und 5,72 (2d, 1H); 5,61 (m, 1H); 4,81 (m, 1H); 4,0-4,2 (m, 3H); 3,53 (m), 3,62 (m) und 3,72 (dd) [2H]; 3,08 (t, 1H); 2,92 (t, 1H); 2,75 (m, 1H); 2,47 (m, 1H); 1,83 (m, 1H); 1,47 (m, 9H); 1,25 (m, 3H); 0,97 ppm (d, 3H).

### I.7. (1SR,2RS,3RS,6SR)-2-Ethyloxycarbonylamino-3-methyl-8-azabicyclo[4.3.0]non-4-en

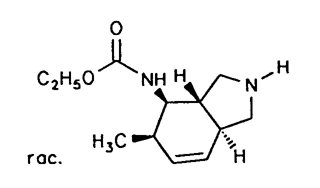

Analog Beispiel C.3. erhält man aus 1,6 g (4,7 mmol) der Titelverbindung aus Beispiel I.6. die Titelverbindung.
- Ausbeute:: 0,7 g (70 % der Theorie) gelbliches Öl;
- Rf =: 0,09
Methylenchlorid/Methanol/17 %-iges wäßriges Ammoniak (15:4:0,5)

### Beispiel K:

### (1RS,2RS,6RS)-2-Ethoxycarbonylaminomethyl-8-azabicyclo[4.3.0]non-4-en

### K.1. 3-Phthalimidomethyl-cyclohex-4-en-1,2-dicarbonsäurediethylester

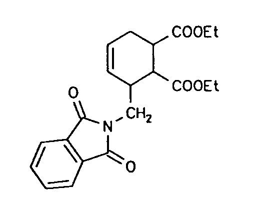

10.67 g (50 mmol) N-[(E)-2,4-Pentadienyl]-phthalimid (Titelverbindung aus Beispiel B.1.) und 8.61 g Fumarsäurediethylester werden in 50 ml Toluol 2 Tage zum Rückfluß erhitzt. Der Ansatz wird eingeengt und der Rückstand an Kieselgel chromatographiert (Laufmittel: Cyclohexan/Aceton 8:1).
- Ausbeute:: 14.8 g (77 % der Theorie).
- Schmelzpunkt:: 80-84 °C.

### K.2. Ethyl-(1RS,2RS,6RS)-9-oxo-8-azabicyclo[4.3.0]non-4-en-2-carboxylat (A) und Ethyl-(1RS,2RS,6SR)-9-oxo-8-azabicyclo[4.3.0]non-4-en-2-carboxylat (B)

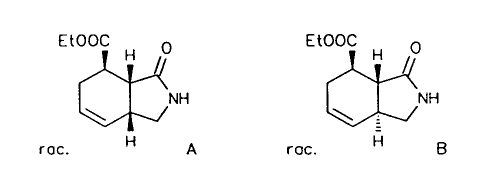

150.3 g (0.39 mol) der Titelverbindung aus Beispiel K.1. werden in 720 ml Ethanol vorgelegt und unter Eiskühlung 173.3 g ( 2.9 mol) Ethylendiamin zugetropft. Man rührt 20 h bei Raumtemperatur, engt in vacuo ein, verdünnt mit Wasser (ca. 700 ml) stellt mit konz. Salzsäure pH 2-3 ein und extrahiert dreimal mit jeweils 500 ml Dichlormethan. Die organische Phase wird getrocknet (Natriumsulfat) und in vacuo eingeengt. Die Trennung der Diastereomeren gelingt durch Chromatographie (Laufmittel: Cyclohexan/Aceton 1:1).
- Ausbeute:: 36.7 g Produkt A (45 % der Theorie) RF = 0.47 (Cyclohexan/Aceton 1:1),
37.0 g Produkt B (45 % der Theorie) RF = 0.22 (Cyclohexan/Aceton 1:1).

### K.3. (1RS,2RS,6RS)-2-Hydroxymethyl-8-azabicyclo[4.3.0]non-4-en

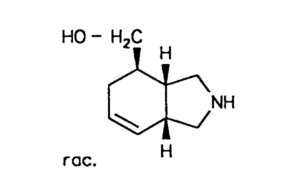

5,2 g (25 mmol) Ethyl-(1RS,2RS,6RS)-9-oxo-8-azabicyclo[4.3.0]non-4-en-2-carboxylat (Produkt A aus Beispiel K.2.) werden unter Stickstoff-Atmosphäre in 50 ml Tetrahydrofuran gelöst und nachfolgend 130 ml einer 1.5molaren Di(isobutyl)aluminiumhydrid-Lösung (195 mmol) zugetropft. Die Lösung wird 16 h unter Rückfluß erwärmt. Nach vollständiger Umsetzung werden nacheinander 60 ml Methanol, 30 ml tert.-Butylmethylether und 10 ml Wasser zugetropft und unter Zusatz von Tonsil abgesaugt. Der Nutschrückstand wird zweimal mit einer Mischung aus Ethanol/konz. Ammoniak/Wasser (10:1:1) verrührt und erneut abgesaugt. Die vereinigten Filtrate werden eingeengt und das Rohprodukt chromatographisch gereinigt (Laufmittel: Dichlormethan/Methanol/konz. Ammoniak 2:4:1).
- Ausbeute:: 2.7 g (71 % der Theorie)
¹H-NMR (DMSO-d₆): 5.69 (m, 1 H, 4-H); 5.60 (m, 1 H, 5-h); 3.39 (dd, 1 H, 10a-H); 3.26 (dd, 1 H, 10b-H); 2.97 (m, 2 H, 7a-H, 9a-H), 2.63 (m, 1 H, 9b-H); 2.38 (bs, 1 H, 6-H), 2.32 (dd, 1 H, 7b-H); 2.06 (m, 1 H, 3a-H); 1.95 (m, 1 H, 1-H), 1.77 (m, 1 H, 3b-H); 1.44 ppm (m, 1 H, 2-H).

### K.4. (1RS,2RS,6RS)-8-tert.-Butoxycarbonyl-2-hydroxymethyl-8-azabicyclo[4.3.0]non-4-en

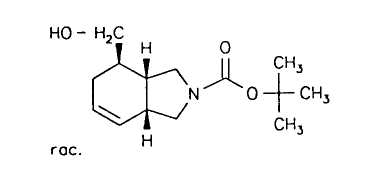

Das Produkt aus Beispiel K.3. (8.87 g; 58 mmol) wird umgesetzt, wie im Beispiel H.1. beschrieben.
- Ausbeute:: 11.0 g (75 % der Theorie).
- RF =: 0.25 (Cyclohexan/Aceton 2:1).

### K.5. (1RS,2RS,6RS)-8-tert.-Butoxycarbonyl-2-(4-toluolsulphonyloxymethyl)-8-azabicyclo[4.3.0]non-4-en

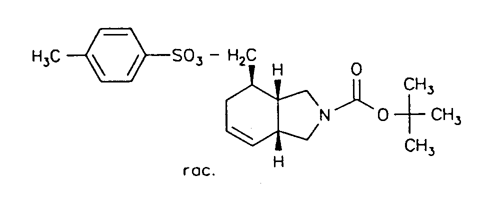

Die Titelverbindung wird aus dem Produkt von Beispiel K.4. in Analogie zum Beispiel F.1. erhalten.
- Ausbeute:: 97 % der Theorie.
- RF =: 0.40 (Cyclohexan/Aceton 2:1).

### K.6. (1RS,2RS,6RS)-8-tert.-Butoxycarbonyl-2-azidomethyl-8-azabicyclo[4.3.0]non-4-en

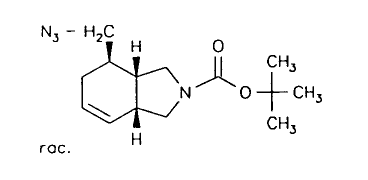

Eine Lösung von 33 g ( 0.08 mol) (1RS,2RS,6RS)-8-tert.-Butyloxycarbonyl-2-(4-toluol-sulphonyloxymethyl)-8-azabicyclo[4.3.0]non-4-en (Titelverbindung aus Beispiel K.5.) und 15.8 g ( 0.24 mol) Natriumazid in 200 ml N.N-Dimethylformamid wird 40 h bei 70 °C gerührt. Die abgekühlte Lösung wird mit Wasser (500 ml) verdünnt und dreimal mit je 250 ml Petrolether extrahiert. Die vereinigte organische Phase wird mit 5 %iger Natriumhydrogencarbonat-Lösung gewaschen, getrocknet (Natriumsulfat) und eingeengt.
- Ausbeute:: 21.6 g (97 %).
¹H-NMR (CDCl₃): 5.71 (m, 1 H, C=CH); 5.58 (m, 1 H,C=CH); 3.61 - 3.22 (m, 5 H); 3.10 (m, 1 H); 2.70 (bs, 1 H); 2.24 (m, 2 H); 1.91 (m, 2 H), 1.47 ppm (s, 9 H, tert.-butyl).

### K.7. (1RS,2RS,6RS)-8-tert.-Butoxycarbonyl-2-aminomethyl-8-azabicyclo[4.3.0]non-4-en

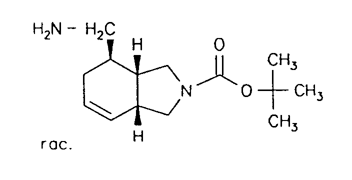

Eine Lösung der Azidoverbindung aus Beipiel K.6. (21.6 g; 78 mmol) in 150 ml Pyridin/Wasser (5:1) wird unter Eiskühlung mit Schwefelwasserstoff gesättigt und anschließend 20 h bei Raumtemperatur belassen. Nach vollständigem Umsatz wird in vacuo eingeengt, mehrfach mit Toluol nachdestilliert und der Rückstand chromatographiert (Laufmittel: Cyclohexan/Aceton 1:1).
- Ausbeute:: 11.0 g (66 % der Theorie).
- RF =: 0.12 (Cyclohexan/Aceton 1:1).

### K.8. (1RS,2RS,6RS)-8-tert.-Butoxycarbonyl-2-(ethyloxycarbonylaminomethyl)-8-azabicyclo[4.3.0]non-4-en

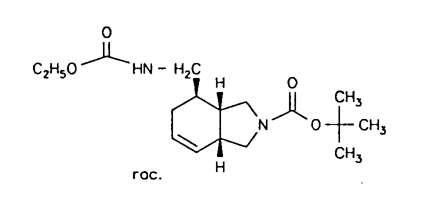

3.7 g ( 15 mmol) (1RS,2RS,6RS)-8-tert.-Butoxycarbonyl-2-aminomethyl-8-azabicyclo[4.3.0]non-4-en werden in 40 ml Dioxan und 15 ml Wasser vorgelegt, 2.3 g (16 mmol) Kaliumcarbonat zugegeben und 1.75 g (16 mmol) Chlorameisensäureethylester bei Raumtemperatur zugetropft. Nach zweistündigem Rühren wird in vacuo eingeengt, der Rückstand in Dichlormethan (70 ml) aufgenommen, zweimal mit je 25 ml Wasser ausgeschüttelt, getrocknet (Natriumsulfat) und eingeengt. Das Rohprodukt wird durch Chromatographie (Cyclohexan/Aceton 2:1) gereinigt.
- Ausbeute:: 2.8 g (59 % der Theorie).
- RF =: 0.53 (Cyclohexan/Aceton 1:1).

### K.9. (1RS,2RS,6RS)-2-(Ethoxycarbonylaminomethyl)-8-azabicyclo[4.3.0]non-4-en

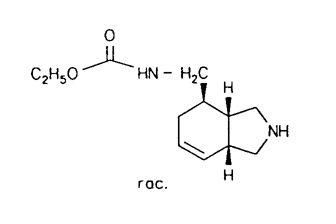

7.6 g (23 mmol) des Produktes aus Beispiel K.8. werden in 100 ml Methanol/Wasser (1:1) vorgelegt und bei RT 30 ml halbkonzentrierte Salzsäure zulaufen gelassen. Nachdem die Gasentwicklung beendet ist, wird 30 Minuten nachgerührt, mit Eiswasser (ca. 100 ml) verdünnt und mit konz. Natronlauge pH 12 eingestellt. Die wässrige Phase wird viermal mit je 100 ml Dichlormethan extrahiert. Die Extrakte werden vereinigt, über Natriumsulfat getrocknet und in vacuo eingeengt.
- Ausbeute:: 3.9 g (76 % der Theorie).
- RF =: 0.45 (Dichlormethan/Methanol/konz. Ammoniak (2:4:0.1)

### Beispiel L:

### (1RS,2RS,6RS)-2-Aminomethy1-8-azabicyclo[4.3.0]non-4-en-bis-trifluormethansulfonat

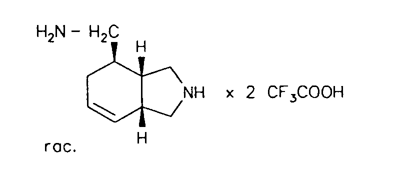

Eine Lösung von 2.0 g ( 8 mmol) (1RS,2RS,6RS)-8-tert.-Butoxycarbonyl-2-aminomethyl-8-azabicyclo[4.3.0]non-4-en (Produkt aus Beispiel K.7.) in 30 ml Dichlormethan wird mit 30 ml Trifluoressigsäure versetzt und 30 Minuten bei Raumtemperatur belassen. Das Lösungsmittel und die Säure werden in Gegenwart von Toluol abdestilliert, mehrfach mit Toluol nachdestilliert. Das Produkt wird im Vakuum-Exsikkator über Kaliumhydroxid/Phos-phorpentoxid (1:1) getrocknet.
- Ausbeute:: 1.5 g braunes Öl.
¹H-NMR (DMSO-d₆): 5.78 (m, 1 H, C=CH); 5.60 (m, 1 H, C=CH); 3.34 (M, 2 H), 3.03 (m, 1 H), 2.87 (m, 2 H), 2.73 (m, 1H); 2.45 (m, 1 H); 2.34 (m, 1 H); 2.22 (M, 1 H); 1.94 ppm (m, 2 H). FAB-MS: M+1 = 153.

### Beispiel M:

### (1RS,2RS,6SR)-2-Ethoxycarbonylaminomethyl-8-azabicyclo[4.3.0]non-4-en (Das Produkt ist identisch mit der Titelverbindung aus Beispiel F)

### M.1. (1RS,2RS,6SR)-2-Hydroxymethyl-8-azabicyclo[4.3.0]non-4-en

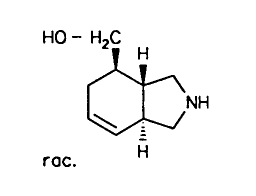

Ethyl-(1RS,2RS,6SR)-9-oxo-8-azabicyclo[4.3.0]non-4-en-2-carboxylat (Produkt B aus Beispiel K.2.) wird analog zu Beispiel K.3. umgesetzt.
- Ausbeute:: 75 % der Theorie.
- RF =: 0.22 (Dichlormethan/Methanol/konz. Ammoniak (15:4:0.5).

### M.2. (1RS,2RS,6SR)-8-tert.-Butoxycarbonyl-2-hydroxymethyl-8-azabicyclo[4.3.0]non-4-en

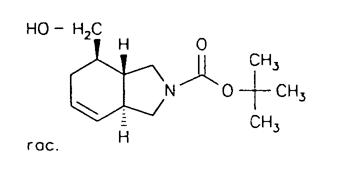

Das Produkt aus Beispiel M.1. wird analog zu Beispiel K.4. umgesetzt.
- Ausbeute:: 64 % der Theorie.
- RF =: 0.23 (Cyclohexan/Aceton 2:1).

### M.3. (1RS,2RS,6SR)-8-tert.-Butoxycarbonyl-2-(4-toluolsulphonyloxymethyl)-8-azabicyclo[4.3.0]non-4-en

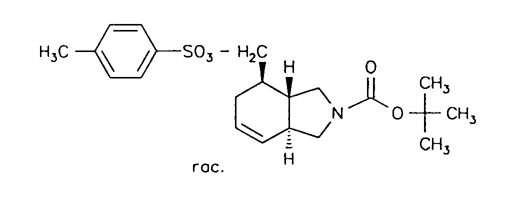

Die Titelverbindung wird aus dem Produkt von Beispiel M.2. in Analogie zum Beispiel F.1. erhalten.
- Ausbeute:: 91 - 98 % der Theorie.
- RF =: 0.59 (Cyclohexan/Aceton 2:1).

### M.4. (1RS,2RS,6SR)-8-tert.-Butoxycarbonyl-2-azidomethyl-8-azabicyclo[4.3.0]non4-en

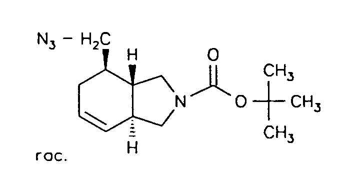

Eine Lösung von 13.0 g (32 mmol) des Produktes aus Beispiel M.3. in 80 ml N.N-Dimethylformamid wird mit 4.15 g (64 mmol) Natriumazid versetzt und 4 h bei 70 °C gerührt. Sodann wird nochmals die gleiche Menge Natriumazid nachgesetzt und weitere 6 h bei 100 °C gerührt. Anschließend wird aufgearbeitet, wie im Beispiel K.6. beschrieben
- Ausbeute:: 7.0 g (79 % der Theorie).
- RF =: 0.55 (Cyclohexan/Aceton 2:1).

### M.5. (1RS,2RS,6SR)-8-tert.-Butoxycarbonyl-2-aminomethyl-8-azabicyclo[4.3.0]non-4-en

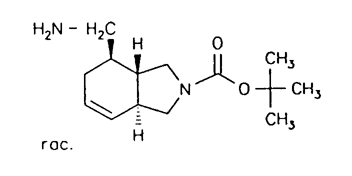

Die Azidoverbindung aus Beispiel M.4. wird umgesetzt, wie im Beispiel K.7. beschrieben. Die Chromatographie erfolgt mit Methanol/Dichlormethan/konz. Ammoniak 15:2:0.1).
- Ausbeute:: 75 % der Theorie.
- RF =: 0.12 (Methanol/Dichlormethan/konz. Ammoniak 15:2:0.1)

### M.6. (1RS,2RS,6SR)-8-tert.-Butoxycarbonyl-2-(ethyloxycarbonylaminomethyl)-8-azabicyclo[4.3.0]non-4-en

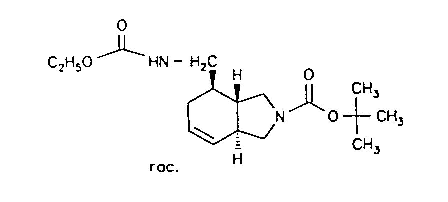

4.3 g (17 mmol) der Aminoverbindung aus Beispiel M.5. und 1.9 g (19 mmol) Triethylamin werden in 50 ml Dichlormethan vorgelegt, bei 0 °C 2.2 g (20 mmol) Chlorameisensäureethyl-ester, gelöst in 10 ml Dichlormethan zugetropft und 24 h bei Raumtemperatur nachgerührt. Die Lösung wird mit Wasser (50 ml) versetzt und die Phasen getrennt. Die wässrige Phase wird noch dreimal mit je 40 ml Dichlormethan extrahiert. Die organischen Phasen werden vereinigt, getrocknet (Natriumsulfat) und eingeengt.
- Ausbeute:: 5.3 g (96 % der Theorie).
¹H-NMR (CDCl₂-CDCl₂, 80 °C): 5.79 (ddd, 1 H, C=CH); 5.58 (m, 1 H, C=CH); 4.61 (bs, 1 H, Carbamat-NH); 4.23 (m, 1 H), 4.12 (q, 2 H, Ethyl-CH₂); 3.99 (m, 1 H); 3.20 - 3.08 (m, 2 H); 2.82 (m, 2 H); 2.25 (m, 2 H); 2.09 (m, 1 H); 1.84 (m, 2 H); 1.42 (s, 9 H, tert.-Butyl); 1.37 ppm (t, 3 H, Ethyl-CH₃).

### M.7. (1RS,2RS,6SR)-2-(Ethoxycarbonylaminomethyl)-8-azabicyclo[4.3.0]non-4-en

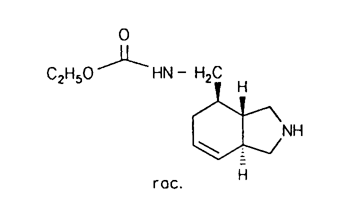

(1RS,2RS,6SR)-8-tert.-Butoxycarbonyl-2-(ethoxycarbonylaminomethyl)-8-azabicyclo[4.3.0]non-4-en wird umgesetzt, wie im Beispiel K.9. beschrieben.
- Ausbeute:: quantitativ.
- RF: = 0.55 (Methanol/Dichlormethan/konz.Ammoniak 15:4:0.5).

### Beispiel N:

### (1SR,2RS,6RS)-2-Methylamino-8-azabicyclo[4.3.0]non-4-en

### N.1. (1RS,2RS,6RS)-9-Oxo-8-azabicyclo[4.3.0]non-4-en-2-carbonsäure

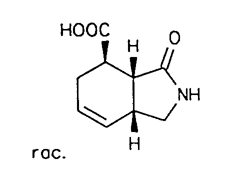

8.36 g (40 mmol) Ethyl-(1RS,2RS,6RS)-9-oxo-8-azabicyclo[4.3.0]non-4-en-2-carboxylat (Produkt A aus Beispiel K.2.) werden mit 30 ml Wasser und 5 g konz. Schwefelsäure 40 h bei 60 °C gerührt. Beim abkühlen fällt das Produkt aus. Der Niederschlag wird mit wenig kaltem Wasser gewaschen und im Vakuum-Trockenschrank bei 50 °C getrocknet.
- Ausbeute:: 4.80 g (66 % der Theorie).
¹H-NMR (DMSO-d₆): 12.35 (s, 1 H, COOH); 7.60 (s, 1 H, Laktam-NH); 5.74 (m, 1 H, C=CH); 5.59 (m, 1 H, C=CH); 3.45 (dd, 1 H, 7a-H); 2.95 - 2.85 (m, 4 H, 1-H, 2-H, 6-H, 7b-H); 2.29 (m, 1 H, 3a-H); 2.00 ppm (m, 1 H, 3b-H).

### N.2. (1SR,2RS,6RS)-2-Ethoxycarbonylamino-9-oxo-8-azabicyclo[4.3.0]non-4-en

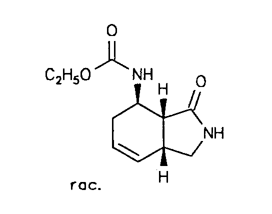

(1RS,2RS,6RS)-9-Oxo-8-azabicyclo[4.3.0]non-4-en-2-carbonsäure (Titelverbindung aus Beispiel N.1.) wird analog Beispiel C.2. umgesetzt.
- Ausbeute:: 68 % der Theorie
- RF =: 0.06 (Cyclohexan/Aceton 1:1).

### N.3. (1SR,2RS,6RS)-2-Methylamino-8-azabicyclo[4.3.0]non-4-en

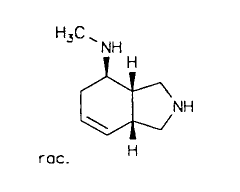

Die Titelverbindung wird erhalten, indem das Produkt aus Beispiel N.2. mit 10 Equivalenten Di(isobutyl)aluminiumhydrid analog Beispiel K.3. umgesetzt und aufgearbeitet wird.
- Ausbeute:: 51 % der Theorie.
¹H-NMR (CDCl₃): 5.72 (m, 1 H, C=CH); 5.68 (m, 1 H, C=CH); 3.19 - 3.10 (m, 2 H); 2.88 (dd, 1 H); 2.60 (dd, 1 H); 2.50 (m, 1 H); 2.44 (s, 3 H, N-CH₃); 2.33 - 2.28 (m, 2 H); 2.19 (m, 1 H); 1.89 ppm (m, 1 H).

### Beispiel O:

### (1SR,2SR,6RS)-2-Methylamino-8-azabicyclo[4.3.0]non-4-en

### O.1. (1RS,2SR,6RS)-9-Oxo-8-azabicyclo[4.3.0]non-4-en-2-carbonsäure

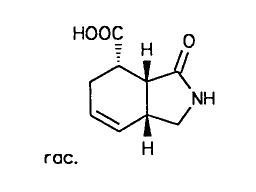

0.2 g konz. Schwefelsäure, 25 ml Wasser und 25 ml Essigsäure werden bei 60 °C vorgelegt. 9.8 g (50 mmol) des Produktes aus Beispiel G.3. werden in kleinen Portionen zugegeben.Man rührt 5 h bei 60 °C nach. Zur Aufarbeitung wird eine Lösung von 0.8 g Natrium-hydrogencarbonat in 10 ml Wasser zugegeben und in vacuo eingeengt. Der Rückstand wird in 40 ml Wasser suspendiert und unter Eiskühlung durch Zugabe konz. Natronlauge in Lösung gebracht. Nachdem von unlöslichen Anteilen abgesaugt worden ist, wird mit halbkonzentrierter Salzsäure sauer gestellt und wieder auf 0 °C gekühlt. Das ausfallende Produkt wird mit wenig kaltem Wasser gewaschen und nachfolgend im Vakuum-Trockenschrank bei 50 °C getrocknet.
- Ausbeute:: 4.8 g (53 % der Theorie).
- Schmelzpunkt:: 192-193 °C.

### O.2. (1SR,2SR,6RS)-2-Ethoxycarbonylamino-9-oxo-8-azabicyclo[4.3.0]non-4-en

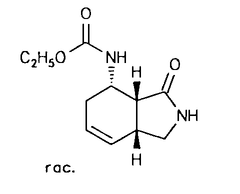

(1RS,2SR,6RS)-9-Oxo-8-azabicyclo[4.3.0]non-4-en-2-carbonsäure (Titelverbindung aus Beispiel O.1.) wird umgesetzt, wie im Beispiel C.2. beschrieben.
- Ausbeute:: 68 % der Theorie.
- Schmelzpunkt:: 160-164 °C.

### O.3. (1SR,2SR,6RS)-2-Methylamino-8-azabicyclo[4.3.0]non-4-en

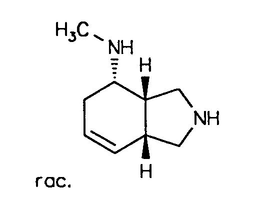

Die Titelverbindung wird erhalten, indem das Produkt aus Beispiel O.2. mit 10 Equivalenten Di(isobutyl)aluminiumhydrid analog Beispiel K.3. umgesetzt und aufgearbeitet wird.
- Ausbeute:: 81 % der Theorie.
¹H-NMR (CDCl₃): 5.72 (m, 1 H, C=CH); 5.50 (m, 1 H, C=CH); 3.04 - 2.77 (m, 6 H); 2.60 (m; 1 H); 2.49 (s, 3 H, N-CH₃); 2.31 (bs, 2 H, 2x NH); 2.25 (m, 1 H); 1.89 ppm (m, 1 H).

### Herstellung der Wirkstoffe:

### Beispiel 1:

### 7-(8-Azabicyclo[4.3.0]non-2-en-8-yl)-1-cyclopropyl-6,8-difluor-1,4-dihydro-4-oxo-3-chinolincarbonsäure

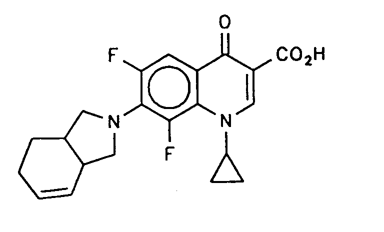

1,4 g (5 mmol) 1-Cyclopropyl-6,7,8-trifluor-1,4-dihydro-4-oxo-3-chinolincarbonsäure, 0,6 g (5 mmol) DABCO und 0,8 g (6 mmol) der Titelverbindung aus Beispiel A in einer Mischung aus 15 ml Acetonitril und 7,5 ml DMF 3 h zum Rückfluß erhitzen. Nach Abkühlung 25 ml Wasser und 2,5 ml Essigsäure zugeben, Niederschlag absaugen, mit Wasser waschen, bei 50 C trocknen.
- Ausbeute:: 1,8 g (93 % der Theorie)
- Schmelzpunkt:: 224-226 C

| Elementaranalyse: (C₂₁H₂₀F₂N₂O₃) | | | | |
|---|---|---|---|---|
| Berechnet | C: 65,3 | H: 5,2 | N: 7,3 | F: 9,8 |
| Gefunden | C: 65,35 | H: 5,15 | N: 7,25 | F: 9,7 |

### Beispiel 2:

### (1'RS,2'RS,6'SR)-1-Cyclopropyl-7-(2'-ethyloxycarbonyl-8'-azabicyclo[4.3.0]non-4'-en-8'-yl)-6,8-difluor-1,4-dihydro-4-oxo-3-chinolincarbonsäure

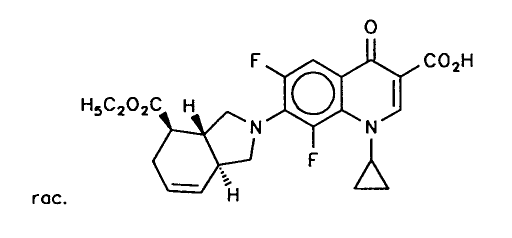

0,7 g (2,5 mmol) 1-Cyclopropyl-6,7,8-trifluor-1,4-dihydro-4-oxo-3-chinolincarbonsäure, 0,3 g (2,5 mmol) DABCO und 0,6 g (3 mmol) der Verbindung A aus Beispiel B in einer Mischung aus 10 ml Acetonitril und 5 ml DMF 5 h zum Rückfluß erhitzen. Nach Abkühlung 30 ml Wasser und 1 ml Essigsäure zugeben, Niederschlag absaugen, mit Wasser waschen, über KOH im Exsikkator trocknen.
- Ausbeute:: 0,95 g (83 % der Theorie)
- Schmelzpunkt:: 212-214 C

| Elementaranalyse: (C₂₄H₂₄F₂N₂O₅) | | | | |
|---|---|---|---|---|
| Berechnet | C: 62,9 | H: 5,2 | N: 6,1 | F: 8,3 |
| Gefunden | C: 62,7 | H: 5,3 | N: 6,1 | F: 8,3 |

¹H-NMR (d₆-DMSO): δ = 8,62 (s, 1H, H an C-2); 7,72 (d, 1H, H an C-5); 5,91 (d, 1H, H an C-5'); 5,76 (m, 1H, H an C-4'); 4,05-4,19 (m, 3H, Ethoxy-CH₂ und Cyclopropyl-CH); 3,84 (m, 1H, CH₂N); 3,76 (m, 2H, CH₂N); 3,53 (m, 1H, CH₂N); 2,84 (m, 1H, H an C-2'); 2,43-2,58 (m, 2H, Hₐ an C-3' und H an C-6'); 2,33 (m, 1H, H_{b} an C-3'); 1,94 (m, 1H, H an C-1'); 1,22 (t, 3H, Ethoxy-CH₃); 1,07-1,26 ppm (m, 4H, Cyclopropyl-CH₂).

### Beispiel 3:

### (1'SR,2'RS,6'SR)-1-Cyclopropyl-7-(2'-ethyloxycarbonylamino-8'-azabicyclo[4.3.0]non-4'-en-8'-yl)-6,8-difluor-1,4-dihydro-4-oxo-3-chinolincarbonsäure

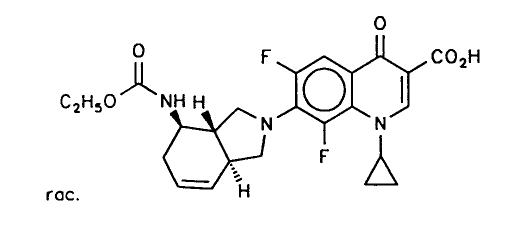

2,8 g (10 mmol) 1-Cyclopropyl-6,7,8-trifluor-1,4-dihydro-4-oxo-3-chinolincarbonsäure, 1,1 g (10 mmol) DABCO und 2,6 g (12 mmol) der Titelverbindung aus Beispiel C in einer Mischung aus 30 ml Acetonitril und 15 ml DMF 3 h zum Rückfluß erhitzen. Nach Abkühlung 45 ml Wasser und 5 ml Essigsäure zügeben, Niederschlag absaugen, mit Wasser waschen, bei 50 C trocknen.
- Ausbeute:: 4,8 g (94 % der Theorie) der Titelverbindung als Addukt mit 1/2 mol DMF.
- Schmelzpunkt:: 239-245 C

| Elementaranalyse: (C₂₄H₂₅F₂N₃O₅ x 1/2 C₃H₇NO) | | | | |
|---|---|---|---|---|
| Berechnet | C: 60,1 | H: 5,6 | N: 9,6 | F: 7,5 |
| Gefunden | C: 60,35 | H: 5,85 | N: 9,7 | F: 7,35 |

¹H-NMR (CDCl₃): δ = 8,69 (s, 1H, H an C-2); 7,75 (d, 1H, H an C-5); 5,84 (d, 1H, H an C-5'); 5,74 (m, 1H, H an C-4'); 4,15 (m, 2H, Ethoxy-CH₂); 3,87-4,05 (m, 3H), 3,82 (m, 1H), 3,64-3,57 (m, 2H): H an C-7'/9', H an C-2', Cyclopropyl-CH; 2,73 (m, 1H, Hₐ an C-3'); 2,63 (m, 1H, H an C-6'); 1,96-2,09 (m, 2H, H an C-1' und H_{b} an C-3'); 1,08-1,32 ppm (m, 7H, Cyclopropyl-CH₂ und Ethoxy-CH₃).

### Beispiel 4:

### (1'SR,2'RS,6'SR)-7-(2'-Amino-8'-azabicyclo[4.3.0]non-4'-en-8'-yl)-1-cyclopropyl-6,8-difluor-1,4-dihydro-4-oxo-3-chinolincarbonsäure

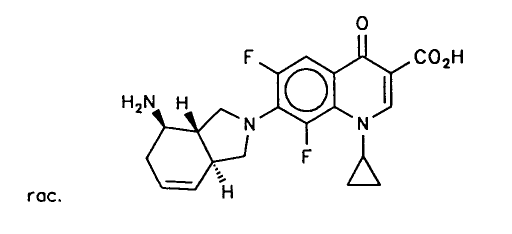

2,55 g (5 mmol) der Titelverbindung aus Beispiel 3 in einer Mischung aus 50 ml 10 %-iger Kalilauge und 25 ml 1,2-Ethandiol 3 h zum Rückfluß erhitzen. Nach Abkühlung 50 ml Methanol zusetzen, mit Salzsäure (konz. HCl/H₂O 1:3) einen pH-Wert von 4-5 einstellen, über Nacht bei 0 C stehen lassen, absaugen, Niederschlag in 70 ml einer Mischung aus Methanol/Wassser 1:1 suspendieren, mit Salzsäure auf pH 2-3 einstellen, gründlich verrühren, erneut absaugen, mit wenig Wasser nachwaschen, bei 50 C trocknen.
- Ausbeute:: 1,95 g (88 % der Theorie) der Titelverbindung als Addukt mit 1 mol HCl und 1/2 mol H₂O
- Schmelzpunkt:: > 300 C

| Elementaranalyse: (C₂₁H₂₁F₂N₃O₃ x HCl x 1/2 H₂O) | | | | | |
|---|---|---|---|---|---|
| Berechnet | C: 56,4 | H: 5,2 | N: 9,4 | Cl: 8,0 | F: 8,5 |
| Gefunden | C: 56,1 | H: 5,35 | N: 9,4 | Cl: 7,45 | F: 8,25 |

¹H-NMR (CF₃CO₂D): δ = 9,24 (s, 1H, H an C-2); 8,06 (d, 1H, H an C-5); 6,03 (d, 1H, H an C-5'); 5,88 (m, 1H, H an C-4'); 4,47 (m, 1H), 4,37 (m, 1H), 4,22 (m, 2H), 4,04 (m, 1H), 3,96 (m, 1H): H an C-7'/9', H an C-2', Cyclopropyl-CH; 2,99 (m, 1H, Hₐ an C-3'); 2,84 (m, 1H, H an C-6'); 2,58 (m, 1H, H_{b} an C-3'); 2,48 (m, 1H, H an C-1'); 1,57 (m, 2H, Cyclopropyl-CH₂); 1,42 ppm (m, 2H, Cyclopropyl-CH₂).

### Beispiel 5:

### (1'SR,2'RS,6'SR)-1-Cyclopropyl-7-(2'-ethyloxycarbonylamino-8'-azabicyclo[4.3.0]non-4'-en-8'-yl)-6-fluor-1,4-dihydro-4-oxo-3-chinolincarbonsäure

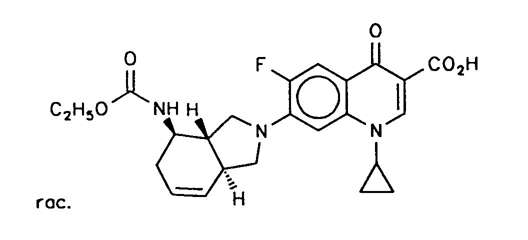

1,35 g (5 mmol) 1-Cyclopropyl-6,7-difluor-1,4-dihydro-4-oxo-3-chinolincarbonsäure, 0,6 g (5 mmol) DABCO und 1,3 g (6 mmol) der Titelverbindung aus Beispiel C in einer Mischung aus 30 ml Acetonitril und 15 ml DMF 3 h zum Rückfluß erhitzen. Nach Abkühlung 30 ml Wasser zugeben und durch Zugabe von Essigsäure pH 4 einstellen, Niederschlag absaugen, in Wasser suspendieren und gründlich verrühren, erneut absaugen und bei 50 C trocknen.
- Ausbeute:: 2,2 g (97 % der Theorie)
- Schmelzpunkt:: 197-199 C
¹H-NMR (d₆-DMSO): δ = 8,57 (s, 1H); 7,78 (d, 1H); 7,34 (br d, 1H); 7,04 (d, 1H); 5,88 (d, 1H); 5,70 (m, 1H); 4,02 (q, 2H); 3,68-3,93 (m, 5H); 3,49 (m, 1H); 2,48-2,72 (m, 2H); 1,93-2,08 (m, 2H); 1,08-1,33 ppm (m, 7H).

### Beispiel 6:

### (1'SR,2'RS,6'SR)-7-(2'-Amino-8'-azabicyclo[4.3.0]non-4'-en-8'-yl)-1-cyclopropyl-6-fluor-1,4-dihydro-4-oxo-3-chinolincarbonsäure

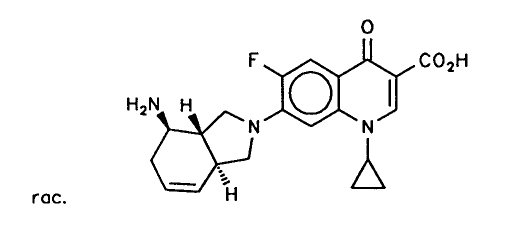

1,15 g (2,5 mmol) der Titelverbindung aus Beispiel 5 in einer Mischung aus 25 ml 10 %-iger Kalilauge und 12,5 ml 1,2-Ethandiol 3 h zum Rückfluß erhitzen. Nach Abkühlung 50 ml Methanol und 35 ml Wasser zusetzen, mit 10 %-iger Salzsäure einen pH-Wert von 4-5 einstellen, Niederschlag absaugen, mit Wasser nachwaschen, bei 50 C trocknen.
- Ausbeute:: 0,9 g (82 % der Theorie) der Titelverbindung als Addukt mit 1 mol HCl und 1 mol H₂O
- Schmelzpunkt:: > 300 C

| Elementaranalyse: (C₂₁H₂₂FN₃O₃ x HCl x H₂O) | | | | |
|---|---|---|---|---|
| Berechnet | C: 57,6 | H: 5,7 | N: 9,6 | F: 4,3 |
| Gefunden | C: 57,5 | H: 5,5 | N: 9,5 | F: 4,1 |

¹H-NMR (CF₃CO₂D): δ = 9,18 (s, 1H); 8,14 (d, 1H); 7,39 (d, 1H); 6,06 (d, 1H); 5,89 (m, 1H); 4,38 (m, 1H); 4,22 (m, 1H); 4,06 (m, 1H); 3,97 (m, 2H); 3,64 (m, 1H); 2,88-3,05 (m, 2H); 2,57 (m, 2H); 1,68 (m, 2H); 1,39 ppm (m, 2H).

### Beispiel 7:

### (1'SR,2'RS,6'SR)-1-tert.-Butyl-7-(2'-ethyloxycarbonylamino-8'-azabicyclo[4.3.0]non-4'-en-8'-yl)-6-fluor-1,4-dihydro-4-oxo-3-chinolincarbonsäure

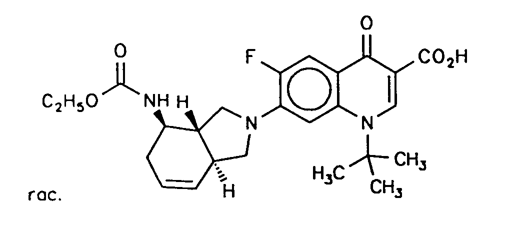

Analog Beispiel 5 erhält man aus 1,4 g (5 mmol) 1-tert.-Butyl-6,7-difluor-1,4-dihydro-4-oxo-3-chinolincarbonsäure und der Titelverbindung aus Beispiel C die Titelverbindung.
- Ausbeute:: 2,3 g (98 % der Theorie) der Titelverbindung als Addukt mit 1/2 mol DMF.
- Schmelzpunkt:: 256-257 C

| Elementaranalyse: (C₂₅H₃₀FN₃O₅ x 1/2 C₃H₇NO) | | | | |
|---|---|---|---|---|
| Berechnet | C: 62,7 | H: 6,6 | N: 9,7 | F: 3,7 |
| Gefunden | C: 62,45 | H: 6,65 | N: 9,6 | F: 3,6 |

¹H-NMR (CDCl₃): δ = 8,94 (s, 1H); 7,87 (d, 1H); 6,91 (d, 1H); 5,87 (d, 1H); 5,75 (m, 1H); 5,03 (br., 1H); 4,16 (m, 2H); 4,01 (m, 1H); 3,82 (m, 1H); 3,70 (m, 1H); 3,60 (m, 1H); 3,33 (m, 1H); 2,65-2,77 (m, 2H); 2,03-2,13 (m, 2H); 1,92 (s, 9H); 1,28 ppm (m, 3H).

### Beispiel 8:

### (1'SR,2'RS,6'SR)-7-(2'-Amino-8'-azabicyclo[4.3.0]non-4'-en-8'-yl)-1-tert.-butyl-6-fluor-1,4-dihydro-4-oxo-3-chinolincarbonsäure

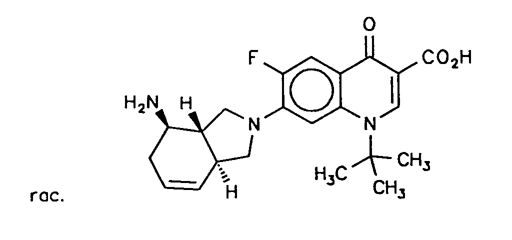

1,2 g (2,3 mmol) der Titelverbindung aus Beispiel 7 in einer Mischung aus 25 ml 10 %-iger Kalilauge und 12,5 ml 1,2-Ethandiol 3 h zum Rückfluß erhitzen. Nach Abkühlung 25 ml Wasser zusetzen, mit 10 %-iger Salzsäure einen pH-Wert von 6-7 einstellen, Niederschlag absaugen, mit Wasser nachwaschen, bei 50 C trocknen.
- Ausbeute:: 0,95 g (96 % der Theorie)
- Schmelzpunkt:: > 300 C
¹H-NMR (CF₃CO₂D): δ = 9,44 (s, 1H); 8,19 (d, 1H); 7,47 (d, 1H); 6,04 (d, 1H); 5,88 (m, 1H); 4,38 (m, 1H); 4,37 (m, 1H); 4,16 (m, 1H); 4,05 (m, 1H); 3,97 (m, 1H); 3,63 (m, 1H); 2,87-3,04 (m, 2H); 2,50-2,64 (m, 2H); 2,11 ppm (s, 9H).

### Beispiel 9:

### (1'SR,2'RS,6'SR)-1-Ethyl-7-(2'-ethyloxycarbonylamino-8'-azabicyclo[4.3.0]non-4'-en-8'-yl)-6-fluor-1,4-dihydro-4-oxo-3-chinolincarbonsäure

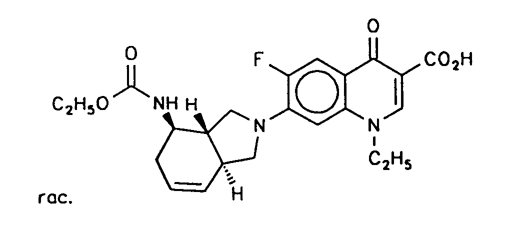

1,3 g (5 mmol) 1Ethyl-6,7-difluor-1,4-dihydro-4-oxo-3-chinolincarbonsäure, 0,6 g (5 mmol) DABCO und 1,3 g (6 mmol) der Titelverbindung aus Beispiel C in einer Mischung aus 15 ml Acetonitril und 7,5 ml DMF 3 h zum Rückfluß erhitzen. Nach Abkühlung 10 ml Acetonitril, 20 ml Wasser und 2,5 ml Essigsäure zugeben, Niederschlag absaugen, mit Acetonitril/Wasser 1:1 waschen und bei 50 C trocknen.
- Ausbeute:: 2,1 g (95 % der Theorie)
- Schmelzpunkt:: 293-295 C

| Elementaranalyse: (C₂₃H₂₆FN₃O₅) | | | | |
|---|---|---|---|---|
| Berechnet | C: 62,3 | H: 5,9 | N: 9,5 | F: 4,3 |
| Gefunden | C: 61,35 | H: 6,05 | N: 9,35 | F: 4,1 |

¹H-NMR (d₆-DMSO): δ = 8,82 (s, 1H); 7,77 (d, 1H); 7,35 (d, 1H): 6,62 (d, 1H); 5,88 (d, 1H); 5,70 (m, 1H); 4,49 (q, 2H); 4,03 (q, 2H); 3,70-3,86 (m, 3H); 3,48 (m, 1H); 3,28 (m, 1H); 2,48-2,69 (m, 2H); 1,94-2,07 (m, 2H); 1,41 (t, 3H); 1,20 ppm (t, 3H).

### Beispiel 10:

### (1'SR,2'RS,6'SR)-7-(2'-Amino-8'-azabicyclo[4.3.0]non-4'-en-8'-yl)-1-ethyl-6-fluor-1,4-dihydro-4-oxo-3-chinolincarbonsäure

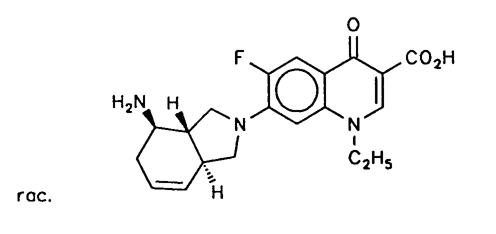

1,35 g (3 mmol) der Titelverbindung aus Beispiel 9 in einer Mischung aus 30 ml 10 %-iger Kalilauge und 15 ml 1,2-Ethandiol 4 h zum Rückfluß erhitzen. Nach Abkühlung 30 ml Acetonitril zusetzen, mit 10 %-iger Salzsäure einen pH-Wert von 2 einstellen, Niederschlag absaugen, mit Acetonitril/Wasser (1:1) nachwaschen, bei 50 C trocknen.
- Ausbeute:: 1,20 g (98 % der Theorie) der Titelverbindung als Hydrochlorid
- Schmelzpunkt:: > 300 C
¹H-NMR (CF₃CO₂D): δ = 9,14 (s, 1H); 8,17 (d, 1H); 6,92 (d, 1H); 6,05 (d, 1H); 5,89 (m, 1H); 4,75 (q, 2H); 4,36 (m, 1H); 4,21 (m, 1H); 4,04 (m, 1H); 3,92 (m, 1H); 3,64 (m, 1H); 3,00 (m, 1H); 2,92 (m, 1H); 2,50-2,63 (m, 2H); 1,78 ppm (t, 3H).

### Beispiel 11:

### (1'SR,2'RS,6'SR)-8-Chlor-1-cyclopropyl-7-(2'-ethyloxycarbonylamino-8'-azabicyclo[4.3.0]non-4'-en-8'-yl)-6-fluor-1,4-dihydro-4-oxo-3-chinolincarbonsäure

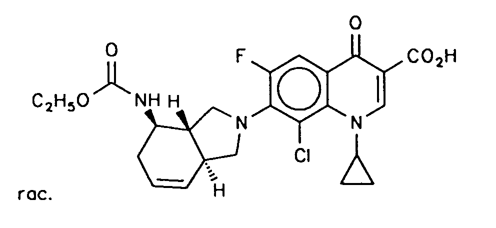

1,5 g (5 mmol) 8-Chlor-1-cyclopropyl-6,7-difluor-1,4-dihydro-4-oxo-3-chinolincarbonsäure, 0,6 g (5 mmol) DABCO und 1,3 g (6 mmol) der Titelverbindung aus Beispiel C in einer Mischung aus 15 ml Acetonitril und 7,5 ml DMF 3 h zum Rückfluß erhitzen. Nach Abkühlung 35 ml Wasser zugeben, durch Zugabe von Essigsäure pH 4 einstellen, Niederschlag absaugen, mit wenig Wasser waschen und bei 50 C trocknen.
- Ausbeute:: 2,3 g (94 % der Theorie)
- Schmelzpunkt:: 225-227 C

| Elementaranalyse: (C₂₄H₂₅ClFN₃O₅) | | | | | |
|---|---|---|---|---|---|
| Berechnet | C: 58,8 | H: 5,1 | N: 8,6 | Cl: 7,2 | F: 3,9 |
| Gefunden | C: 58,55 | H: 5,4 | N: 8,65 | Cl: 7,05 | F: 4,05 |

¹H-NMR (CDCl₃): δ = 8,86 (s, 1H); 7,92 (d, 1H); 5,85 (d, 1H); 5,74 (m, 1H); 4,65 (br., 1H); 4,30 (m, 1H); 4,07-4,19 (m, 3H); 4,04 (m, 1H): 3,86 (m, 1H); 3,57 (dd, 1H); 3,48 (dd, 1H); 2,64-2,78 (m, 2H); 1,98-2,12 (m, 2H); 1,39 (m, 1H); 1,27 (m, 3H); 1,17 (m, 1H); 1,05 (m, 1H); 0,81 ppm (m, 1H).

### Beispiel 12:

### (1'SR,2'RS,6'SR)-7-(2'-Amino-8'-azabicyclo[4.3.0]non-4'-en-8'-yl)-8-chlor-1-cyclopropyl-6-fluor-1,4-dihydro-4-oxo-3-chinolincarbonsäure

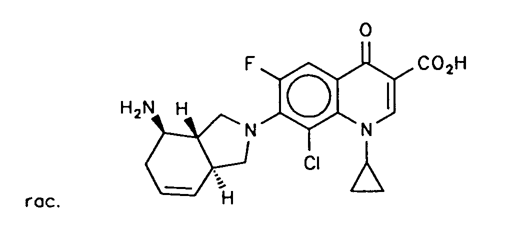

1,0 g (2 mmol) der Titelverbindung aus Beispiel 11 in einer Mischung aus 20 ml 10 %-iger Kalilauge und 10 ml 1,2-Ethandiol 3 h zum Rückfluß erhitzen. Nach Abkühlung mit 10 %-iger Salzsäure einen pH-Wert von 6 einstellen, Niederschlag absaugen, mit Wasser waschen, bei 50 C trocknen.
- Ausbeute:: 0,8 g (96 % der Theorie)
- Schmelzpunkt:: > 300 C
¹H-NMR (CF₃CO₂D): δ = 9,43 (s, 1H); 8,14 (d, 1H); 6,04 (d, 1H); 5,89 (m, 1H); 4,81 (m, 1H); 4,48 (m, 1H); 4,23 (m, 1H); 4,07 (m, 2H); 3,94 (m, 1H); 2,98 (m, 1H); 2,89 (m, 1H); 2,45-2,61 (m, 2H); 1,76 (m, 1H); 1,45 (m, 1H); 1,32 (m, 1H); 1,08 ppm (m, 1H).

### Beispiel 13:

### (1'SR,2'RS,6'SR)-10-(2'-Ethyloxycarbonylamino-8'-azabicyclo[4.3.0]non-4'-en-8'-yl)-9-fluor-2,3-dihydro-3-methyl-7-oxo-7H-pyrido[1,2,3-de][1,4]benzoxazin-6-carbonsäure

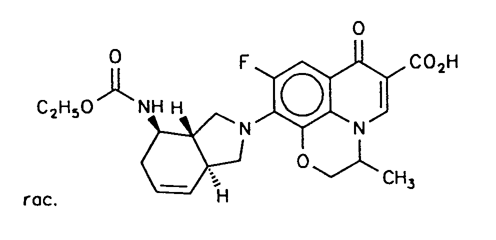

1,4 g (5 mmol) 9,10-Difluor-2,3-dihydro-3-methyl-7-oxo-7H-pyrido[1,2,3-de][1,4]benzoxazin-6-carbonsäure, 0,6 g (5 mmol) DABCO und 1,3 g (6 mmol) der Titelverbindung aus Beispiel C in einer Mischung aus 15 ml Acetonitril und 7,5 ml DMF 7 h zum Rückfluß erhitzen. Nach Abkühlung 20 ml Acetonitril, 20 ml Wasser und 2,5 ml Essigsäure zugeben, Niederschlag absaugen, in 40 ml Acetonitril/Wasser erneut suspendieren, kräftig verrühren, erneut absaugen, mit wenig Wasser waschen und bei 50 C trocknen.
- Ausbeute:: 1,6 g (67 % der Theorie)
- Schmelzpunkt:: 275-286 C
¹H-NMR (d₆-DMSO): δ = 8,88 (s, 1H, H an C-5); 7,52 (d, 1H, H an C-8); 7,30 (d, 1H, NH); 5,84 (d, 1H, H an C-5'); 5,68 (m, 1H, H an C-4'); 4,88 (q, 1H, H an C-3); 4,53 (d, 1H, Hₐ an C-2); 4,24 (d, 1H, H_{b} an C-2); 4,00 (q, 2H, Ethoxy-CH₂); 3,71-3,83 (m, 3H), 3,66 (m, 1H), 3,58 (m, 1H): H an C-7'/9' und H an C-2'); 2,47-2,57 (m, 2H, Hₐ an C-3' und H an C-6'); 2,00 (m, 1H, H_{b} an C-3'); 1,89 (m, 1H, H an C-1'); 1,46 (d, 3H, CH₃ an C-3); 1,18 ppm (t, 3H, Ethoxy-CH₃).

### Beispiel 14:

### (1'SR,2'RS,6'SR)-10-(2'-Amino-8'-azabicyclo[4.3.0]non-4'-en-8'-yl)-9-fluor-2,3-dihydro-3-methyl-7-oxo-7H-pyrido[1,2,3-de][1,4]benzoxazin-6-carbonsäure

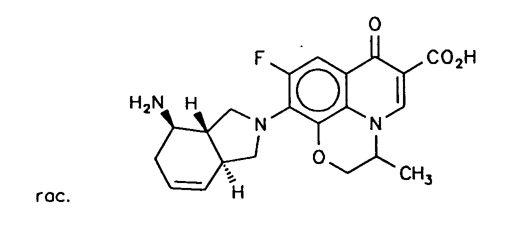

1,2 g (2,5 mmol) der Titelverbindung aus Beispiel 13 in 25 ml 10 %-iger Kalilauge 10 h zum Rückfluß erhitzen. Nach Abkühlung 10 ml Acetonitril zusetzen, mit 10 %-iger Salzsäure einen pH-Wert von 6-7 einstellen, Niederschlag absaugen, mit wenig Wasser waschen, bei 50 C trocknen.
- Ausbeute:: 0,95 g (95 % der Theorie)
- Schmelzpunkt:: > 300 C
¹H-NMR (CF₃CO₂D): δ = 9,44 (2s, 1H); 8,18 (2d, 1H); 5,97 (m, 2H); 5,76 (m, 1H); 4,82-4,98 (m, 2H); 4,70 (m, 1H); 4,40-4,54 (m, 2H); 4,17 (m, 1H); 4,07 (t, 1H); 3,39 (m, 1H); 2,98-3,12 (m, 2H); 2,64 (m, 1H); 1,86 ppm (d, 3H).

### Beispiel 15:

### (1'SR,2'RS,6'SR)-1-Cyclopropyl-7-(2'-ethyloxycarbonylamino-8'-azabicyclo[4.3.0]non-4'-en-8'-yl)-8-fluor-1,4-dihydro-4-oxo-3-chinolincarbonsäure

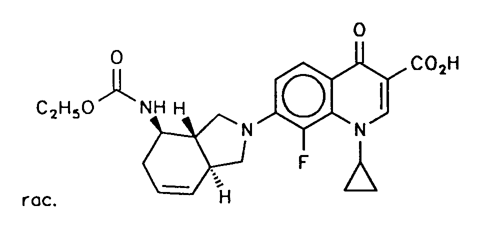

2,0 g (7,5 mmol) 1-Cyclopropyl-7,8-difluor-4-oxo-3-chinolin-carbonsäure, 1,7 g (15 mmol) DABCO und 1,9 g (9 mmol) der Titelverbindung aus Beispiel C in 75 ml DMSO eine Stunde auf 100 C erwärmen. Das Lösungsmittel im Hochvakuum abdestillieren, den Rückstand mit Acetonitril verrühren, absaugen und bei 100 C trocknen.
- Ausbeute:: 3,1 g (90 % er Theorie)
- Schmelzpunkt:: 278-280 C
¹H-NMR (CF₃CO₂D): δ = 9,20 (s, 1H, H an C-2); 8,32 (d, 1H, H an C-5); 7,39 (dd, 1H, H an C-6); 5,95 (d, 1H, H an C-5'); 5,88 (dd, 1H, H an C-4'); 4,40 (m, 3H, H an C-2', Hₐ an C-7' und Hₐ an C-9'); 4,15 (m, 3H, Cyclopropyl-CH und Ethoxy-CH₂); 3,85 (t, 1H, H_{b} an C-7'); 3,64 (t, 1H, H_{b} an C-9'); 2,85 (m, 2H, Hₐ an C-3' und H an C-6'); 2,23 (m, 2H, H an C-1' und H_{b} an C-3'); 1,55 (m, 2H, Cyclopropyl-CH₂); 1,42 ppm (m, 5H, Cyclopropyl-CH₂ und Ethoxy-CH₃).

### Beispiel 16:

### (1'SR,2'RS,6'SR)-7-(2'-Amino-8'-azabicyclo[4.3.0]non-4'-en-8'-yl)-1-cyclopropyl-8-fluor-1,4-dihydro-4-oxo-3-chinolincarbonsäure

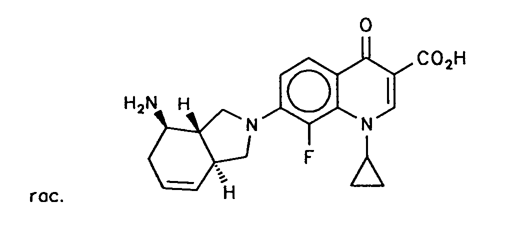

2,3 g (5 mmol) der Titelverbindung aus Beispiel 15 in einer Mischung aus 50 ml 10 %-iger Kalilauge und 25 ml 1,2-Ethandiol 6 h zum Rückfluß erhitzen. 50 ml Methanol zusetzen und mit halbkonzentrierter Salzsäure einen pH-Wert von 4-5 einstellen. Niederschlag absaugen, in 35 ml Methanol suspendieren, mit Salzsäure auf pH 2-3 einstellen und nach Zugabe von 35 ml Wasser gründlich verrühren, erneut absaugen, mit wenig Wasser nachwaschen und bei 100 C trocknen.
- Ausbeute:: 1,6 g (84 % er Theorie) der Titelverbindung als Hydrochlorid
- Schmelzpunkt:: > 300 C
¹H-NMR (CF₃CO₂D): δ = 9,22 (s, 1H, H an C-2); 8,37 (d, 1H, H an C-5); 7,39 (dd, 1H, H an C-6); 6,05 (d, 1H, H an C-5'); 5,89 (dd, 1H, H an C-4'); 4,47 (m, 1H), 4,38 (m, 1H), 4,18 (m, 1H), 4,05 (m, 1H): H an C-2', Hₐ an C-7', Hₐ an C-9' und Cyclopropyl-CH; 3,99 (t, 1H, H_{b} an C-9'); 3,67 (t, 1H, H_{b} an C-7'); 3,00 (m, 1H, Hₐ an C-3'); 2,92 (m, 1H, H an C-6'); 2,55 (m, 2H, H an C-1' und H_{b} an C-3'); 1,55 (m, 2H, Cyclopropyl-CH₂); 1,42 ppm (m, 2H, Cyclopropyl-CH₂).

### Beispiel 17:

### (1'SR,2'RS,6'SR)-1-Cyclopropyl-7-(2'-ethyloxycarbonylamino-8'-azabicyclo[4.3,0]non-4'-en-8'-yl)-5,6,8-trifluor-1,4-dihydro-4-oxo-3-chinolincarbonsäure

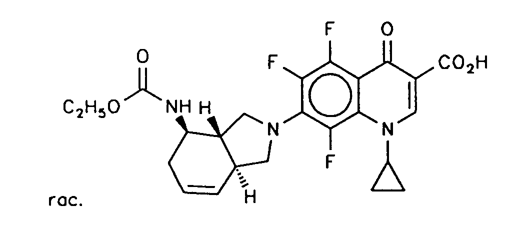

1,8 g (6 mmol) 1-Cyclopropyl-5,6,7,8-tetrafluor-1,4-dihydro-4-oxo-3-chinolincarbonsäure, 0,7 g (6 mmol) DABCO und 1,5 g (7,2 mmol) der Titelverbindung aus Beispiel C in einer Mischung aus 15 ml Acetonitril und 7,5 ml DMF 3 h zum Rückfluß erhitzen. Nach Abkühlung 20 ml Acetonitril, 20 ml Wasser und 2,5 ml Essigsäure zugeben, Niederschlag absaugen, in 40 ml Acetonitril/Wasser (1:1) suspendieren, erneut absaugen, mit Wasser nachwaschen, bei 50 C trocknen.
- Ausbeute:: 2,55 g (87 % er Theorie
- Schmelzpunkt:: 242-244 C

| Elementaranalyse: (C₂₄H₂₄F₃N₃O₅) | | | | |
|---|---|---|---|---|
| Berechnet | C: 58,7 | H: 4,9 | N: 8,6 | F: 11,6 |
| Gefunden | C: 58,65 | H: 5,2 | N: 8,7 | F: 11,35 |

¹H-NMR (CDCl₃): δ = 8,66 (s, 1H); 5,84 (d, 1H); 5,75 (m, 1H); 4,15 (m, 1H); 3,82-4,05 (m, 4H); 3,77 (m, 1H); 3,56 (m, 1H); 2,72 (m, 1H); 2,62 (m, 1H); 1,96-2,10 (m, 2H); 1,28 (m, 3H); 1,06-1,24 ppm (m, 4H).

### Beispiel 18:

### (1'SR,2'RS,6'SR)-5-Amino-1-Cyclopropyl-7-(2'-ethyloxycarbonylamino-8'-azabicyclo[4.3.0]non-4'-en-8'-yl)-6,8-fluor-1,4-dihydro-4-oxo-3-chinolincarbonsäure

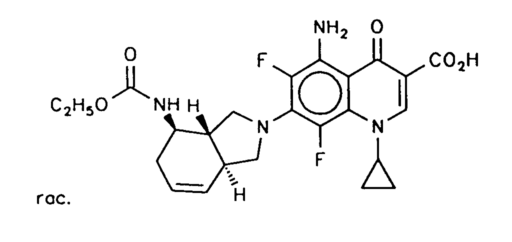

2,5 g (2,5 mmol) der Titelverbindung aus Beispiel 17 in 50 ml DMSO vorlegen, während 8 h Ahmoniak-Gas einleiten und auf 140 C erhitzen. Nach Abkühlung auf 100 ml Wasser geben, mit 10 %-iger Salzsäure auf pH 2-3 stellen, Niederschlag absaugen, mit Wasser waschen, bei 50 C trocknen.
- Ausbeute:: 1,9 g (78 % der Theorie)
- Schmelzpunkt:: 225-229 C
¹H-NMR (d₆-DMSO): δ = 8,44 (s, 1H); 7,31 (d, 1H); 7,12 (br., 2H); 5,83 (d, 1H); 5,68 (m, 1H); 3,93-4,04 (m, 3H); 3,46-3,81 (m, 5H); 2,46-2,61 (m, 2H); 2,03 (m, 1H); 1,90 (m, 1H); 0,97-1,21 ppm (m, 7H).

### Beispiel 19:

### (1'SR,2'RS,6'SR)-5-Amino-7-(2'-Amino-8'-azabicyclo[4.3.0]non-4'-en-8'-yl)-1-cyclopropyl-6,8-difluor-1,4-dihydro-4-oxo-3-chinolincarbonsäure

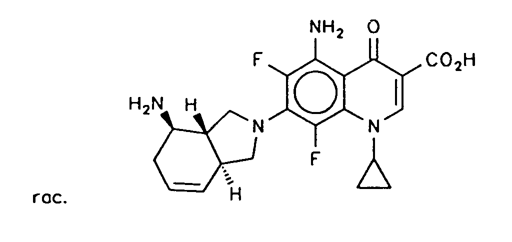

1,2 g (2,6 mmol) der Titelverbindung aus Beispiel 18 in einer Mischung aus 20 ml 10 %-iger Kalilauge und 10 ml 1,2-Ethandiol 15 h zum Rückfluß erhitzen. Nach Abkühlung 10 ml Acetonitril zusetzen, mit 10 %-iger Salzsäure einen pH-Wert von 3-4 einstellen, 4 h bei 0 C stehen lassen, Niederschlag absaugen, mit wenig Wasser waschen, bei 50 C trocknen.
- Ausbeute:: 1,1 g (95 % der Theorie) der Titelverbindung als Hydrochlorid
- Schmelzpunkt:: > 300 C
¹H-NMR (d₆-DMSO): δ = 8,46 (s, 1H); 8,39 (br, 2H); 7,16 (br, 2H); 5,90 (d, 1H); 5,70 (m, 1H); 3,97 (m, 1H); 3,69-3,82 (m, 3H); 3,53 (m, 2H); 2,56-2,68 (m, 2H); 2,23 (m, 1H); 2,07 (m, 1H); 1,00-1,21 ppm (m, 7H).

### Beispiel 20:

### (1'SR,2'RS,6'SR)-1-Cyclopropyl-7-(2'-ethyloxycarbonylamino-8'-azabicyclo[4.3.0]non-4'-en-8'-yl)-6-fluor-1,4-dihydro-4-oxo-1,8-naphthyridin-3-carbonsäureethylester

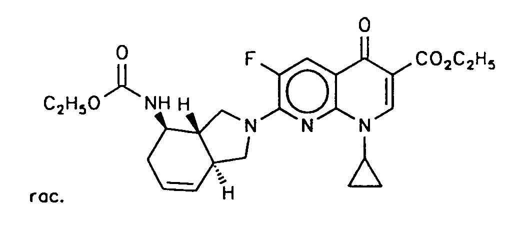

1,24 g (4 mmol) 7-Chlor-1-cyclopropyl-6-fluor-1,4-dihydro-4-oxo-1,8-naphthyridin-3-carbonsäureethylester, 0,48 g (4 mmol) DABCO und 0,84 g (6 mmol) der Titelverbindung aus Beispiel C in 20 ml Acetonitril gelöst 3 h bei Raumtemperatur rühren. Ansatz auf Wasser geben, mit Methylenchlorid extrahieren, vereinigte Extrakte mit Natriumsulfat trocknen, einengen. Rohprodukt an 100 g Kieselgel (63-200 µm) mit Cyclohexan/Aceton (1:1) säulenchromatographisch reinigen.
- Ausbeute:: 0,5 g (26 % der Theorie)
- Rf =: 0,18
Cyclohexan/Aceton (1:1)
¹H-NMR (CDCl₃): δ = 8,45 (s, 1H); 8,00 (d, 1H, J = 13 Hz); 5,86 (d, 1H); 5,72 (m, 1H); 4,94 (br d, 1H); 4,38 (q, 2H); 3,86-4,25 (m, 5H); 3,33-3,70 (m, 3H); 2,50-2,84 (m, 2H); 1,90-2,15 (m, 2H); 0,80-1,34 ppm (m, 10H).

### Beispiel 21:

### (1'SR,2'RS,6'SR)-7-(2'-Amino-8'-azabicyclo[4.3.0]non-4'-en-8'-yl)-1-cyclopropyl-6-fluor-1,4-dihydro-4-oxo-1,8-naphthyridin-3-carbonsäure

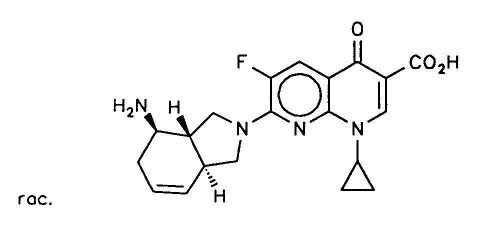

Analog Beispiel 10 erhält man aus 0,1 g (0,2 mmol) der Titelverbindung aus Beispiel 20 die Titelverbindung.
- Ausbeute:: 0,08 g (95 % der Theorie) der Titelverbindung als
Hydrochlorid
- Schmelzpunkt:: > 300 C
¹H-NMR (CF₃CO₂D): δ = 9,17 (s, 1H); 8,14 (d, 1H); 6,05 (dd 1H); 5,89 (d, 1H); 4,46-4,78 (m, 3H); 3,55-4,15 (m, 3H); 2,82-3,04 (m, 2H); 2,42-2,64 (m, 2H); 1,58 (m, 2H); 1,30 ppm (m, 2H).

### Beispiel 22:

### (1'SR,2'RS,6'SR)-1-Cyclopropyl-6,8-difluor-1,4-dihydro-7-(2'-methylamino-8'-azabicyclo[4.3.0]non-4'-en-8'-yl)-4-oxo-3-chinolincarbonsäure

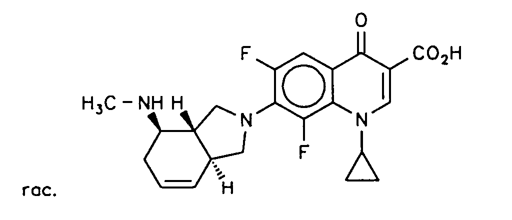

0,84 g (3 mmol) 1-Cyclopropyl-6,7,8-trifluor-1,4-dihydro-4-oxo-3-chinolincarbonsäure, 0,68 g (6 mmol) DABCO und 0,65 g (4,6 mmol) der Titelverbindung aus Beispiel D in einer Mischung aus 10 ml Acetonitril und 5 ml DMF 21 h zum Rückfluß erhitzen. Nach Abkühlung 20 ml Acetonitril und 10 ml Wasser zugeben, durch Zugabe von 10 %-iger Salzsäure einen pH-Wert von 3-4 einstellen, Niederschlag absaugen, in 30 ml Acetonitril/Wasser suspendieren, erneut absaugen, mit wenig Wasser nachwaschen, bei 50 C trocknen.
- Ausbeute:: 1,15 g (85% der Theorie) der Titelverbindung als Hydrochlorid
- Schmelzpunkt:: > 300 C
¹H-NMR (CF₃CO₂D): δ = 9,24 (s, 1H); 8,06 (d, 1H); 6,04 (d, 1H); 5,90 (m, 1H); 4,47 (m, 1H); 4,37 (m, 1H); 4,21 (m, 2H); 3,94 (m, 1H); 3,87 (m, 1H); 3,06 (s, 3H); 2,98 (m, 1H); 2,84 (m, 1H); 2,57 (m, 1H); 2,46 (m, 1H); 1,58 (m, 2H); 1,41 ppm (m, 2H).

### Beispiel 23:

### (1'SR,2'RS,6'SR)-8-Chlor-1-cyclopropyl-6-fluor-1,4-dihydro-7-(2'-methylamino-8'-azabicyclo[4.3.0]non-4'-en-8'-yl)-4-oxo-3-chinolincarbonsäure

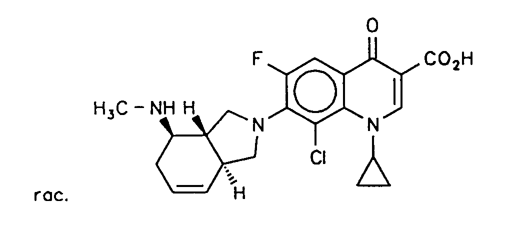

Analog Beispiel 22 erhält man aus 0,9 g (3 mmol) 8-Chlor-1-cyclopropyl-6,7-difluor-1,4-dihydro-4-oxo-3-chinolincarbonsäure, 0,34 g (3 mmol) DABCO und 0,61 g (3,6 mmol) der Titelverbindung aus Beispiel D die Titelverbindung.
- Ausbeute:: 1,3 g (92 % der Theorie) der Titelverbindung als Hydrochlorid
- Schmelzpunkt:: > 300 C
¹H-NMR (CF₃CO₂D): δ = 9,43 (s, 1H); 8,15 (d, 1H); 6,06 (d, 1H); 5,92 (m, 1H); 4,81 (m, 1H); 4,51 (m, 1H); 4,24 (m, 1H); 4,14 (m, 1H); 3,87-3,98 (m, 2H); 3,05 (s, 3H); 2,88-3,03 (m, 2H); 2,48-2,63 (m, 2H); 1,74 (m, 1H); 1,47 (m, 1H); 1,33 (m, 1H); 1,12 ppm (m, 1H).

### Beispiel 24:

### (1'SR,2'RS,6'SR)-1-(2,4-Difluorphenyl)-6-fluor-1,4-dihydro-7-(2'-methylamino-8'-azabicyclo[4.3.0]non-4'-en-8'-yl)-4-oxo-3-chinolincarbonsäure

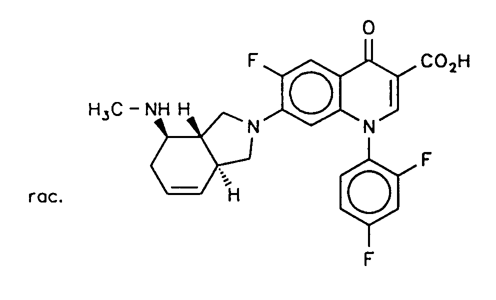

Analog Beispiel 22 erhält man aus 0,85 g (2,5 mmol) 1-(2,4-Difluorphenyl)-6-fluor-1,4-dihydro-4-oxo-3-chinolincarbonsäure, 0,50 g (4,5 mmol) DABCO und 0,76 g (5 mmol) der Titelverbindung aus Beispiel D die Titelverbindung.
- Ausbeute:: 0,6 g (47 % der Theorie) der Titelverbindung als Hydrochlorid
- Schmelzpunkt:: > 300 C
¹H-NMR (CF₃CO₂D): δ = 9,01 (s, 1H); 8,20 (d, 1H); 7,70 (m, 1H); 7,34 (m, 2H); 5,95 (d, 1H); 5,86 (m, 1H); 4,28 (m, 1H); 3,7-3,9 (m, 3H); 3,01 (s, 3H); 2,94 (m, 1H); 2,82 (m, 1H); 2,50 (m, 1H); 2,41 ppm (m, 1H).

### Beispiel 25:

### (1'RS,2'RS,6'SR)-1-Cyclopropyl-6,8-difluor-1,4-dihydro-7-(2'-hydroxymethyl-8'-azabicyclo[4.3.0]non-4'-en-8'-yl)-4-oxo-3-chinolincarbonsäure

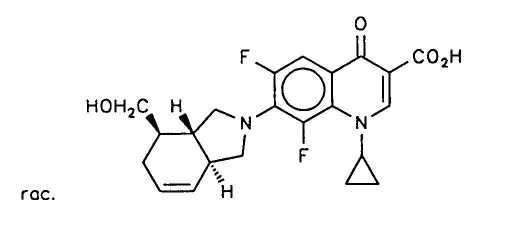

0,9 g (3 mmol) 1-Cyclopropyl-6,7,8-trifluor-1,4-dihydro-4-oxo-3-chinolincarbonsäure, 0,35 g (3 mmol) DABCO und 0,7 g (4,5 mmol) der Titelverbindung aus Beispiel E in einer Mischung aus 10 ml Acetonitril und 5 ml DMF 4 h zum Rückfluß erhitzen. Nach Abkühlung 10 ml Wasser und 1,5 ml Essigsäure zugeben, Niederschlag absaugen, in 40 ml Acetonitril/Wasser (1:1) suspendieren, erneut absaugen, bei 50 C trocknen.
- Ausbeute:: 0,95 g (76 % der Theorie)
- Schmelzpunkt:: 244-247 C
¹H-NMR (d₆-DMSO): δ = 8,58 (s, 1H); 7,67 (d, 1H); 5,87 (d, 1H); 5,75 (m, 1H); 4,07 (m, 1H); 3,78 (m, 2H); 3,68 (s, 1H); 3,27-3,52 (m, 3H); 2,40 (m, 1H); 2,23 (m, 1H); 1,82-1,95 (m, 2H); 1,62 (m, 1H); 1,08-1,23 ppm (m, 4H).

### Beispiel 26:

### (1'RS,2'RS,6'SR)-1-Cyclopropyl-7-(2'-ethyloxycarbonylaminomethyl-8'-azabicyclo[4.3.0]non-4'-en-8'-yl)-6,8-difluor-1,4-dihydro-4-oxo-3-chinolincarbonsäure

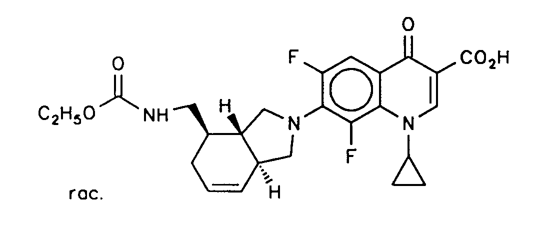

0,6 g (2,1 mmol) 1-Cyclopropyl-6,7,8-trifluor-1,4-dihydro-4-oxo-3-chinolincarbonsäure, 0,25 g (2,1 mmol) DABCO und 0,7 g (3,1 mmol) der Titelverbindung aus Beispiel F in einer Mischung aus 8 ml Acetonitril und 4 ml DMF 3 h zum Rückfluß erhitzen. Nach Abkühlung 10 ml Acetonitril, 10 ml Wasser und 1 ml Essigsäure zugeben, Niederschlag absaugen, in 20 ml Acetonitril/Wasser (1:1) suspendieren, erneut absaugen, bei 50 C trocknen.
- Ausbeute:: 0,8 g (79 % der Theorie)
- Schmelzpunkt:: 220-222 C
¹H-NMR (CDCl₃): δ = 8,70 (s, 1H, H an C-2); 7,77 (d, 1H, H an C-5); 5,86 (d, 1H, H an C-5'); 5,77 (m, 1H, H an C-4'); 4,81 (br., 1H, NH); 4,13 (m, 2H, Ethoxy-CH₂); 3,96 (m, 1H, Cyclopropyl-CH); 3,77 (H an CH₂-NH und Hₐ an C-9'); 3,60 (m, 1H, Hₐ an C-7'); 3,36 (m, 1H, H_{b} an C-9'); 3,14 (m, 1H, H_{b} an C-7'); 2,37-2,51 (m, 2H, Hₐ an C-3' und H an C-6'); 2,04 (m, 1H, H an C-2'); 1,94 (m, 1H, H_{b} an C-3'); 1,73 (m, 1H, H an C-1'); 1,08-1,22 ppm (m, 7H, Cyclopropyl-CH₂ und Ethoxy-CH₃).

### Beispiel 27:

### (1'RS,2'RS,6'SR)-7-(2'-Aminomethyl-8'-azabicyclo[4.3.0]non-4'-en-8'-yl)-1-cyclopropyl-6,8-difluor-1,4-dihydro-4-oxo-3-chinolincarbonsäure

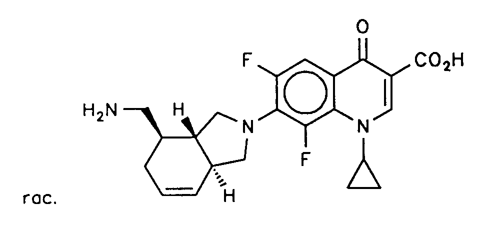

0,75 g (1,5 mmol) der Titelverbindung aus Beispiel 26 in einer Mischung aus 15 ml 10 %-iger Kalilauge und 4 ml 1,2-Ethandiol 3 h zum Rückfluß erhitzen. Nach Abkühlung 15 ml Acetonitril zusetzen, mit 10 %-iger Salzsäure einen pH-Wert von 2-3 einstellen, Niederschlag absaugen, mit Acetonitril/Wasser (1:1) waschen, bei 50 C trocknen.
- Ausbeute:: 0,6 g (90 % der Theorie) der Titelverbindung als Hydrochlorid
- Schmelzpunkt:: 236-240 C
¹H-NMR (CF₃CO₂D): δ = 9,24 (s, 1H); 8,06 (d, 1H); 6,01 (d, 1H); 5,90 (m, 1H); 4,47 (m, 1H); 4,18 (m, 2H); 4,06 (m, 1H); 3,88 (m, 1H); 3,58 (d, 1H); 3,28 (t, 1H); 2,73 (m, 2H); 2,50 (m, 1H); 2,18 (m, 1H); 2,00 (m, 1H); 1,56 (m, 2H); 1,42 ppm (m, 2H).

### Beispiel 28:

### (1'RS,2'SR,6'RS)-1-Cyclopropyl-6,8-difluor-1,4-dihydro-7-(2'-hydroxymethyl-8'-azabicyclo[4.3.0]non-4'-en-8'-yl)-4-oxo-3-chinolincarbonsäure

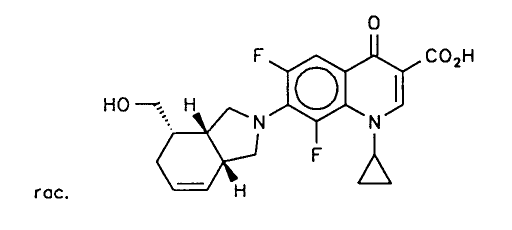

Analog Beispiel 9 erhält man aus 0,59 g (3 mmol) 1-Cyclopropyl-6,7,8-trifluor-1,4-dihydro-4-oxo-3-chinolincarbonsäure und 0,69 g (4,5 mmol) der Titelverbindung aus Beispiel G die Titelverbindung.
- Ausbeute:: 1,1 g (88% der Theorie)
- Schmelzpunkt:: 270-272 C

| Elementaranalyse: (C₂₂H₂₂F₂N₂O₄) | | | | |
|---|---|---|---|---|
| Berechnet | C: 63,5 | H: 5,3 | N: 6,7 | F: 9,1 |
| Gefunden | C: 63,85 | H: 5,5 | N: 6,7 | F: 8,8 |

¹H-NMR (d₆-DMSO): δ = 8,58 (s, 1H); 7,66 (d, 1H); 5,77 (m, 1H); 5,55 (d, 1H); 4,06 (m, 2H); 3,68 (m, 1H); 3,47 (m, 2H); 3,28-3,42 (m, 3H); 2,88 (m, 1H); 2,66 (m, 1H); 2,06 (m, 2H); 1,83 (m, 1H); 1,08-1,24 ppm (m, 4H).

### Beispiel 29:

### (1'RS,2'SR,6'RS)-1-Cyclopropyl-7-(2'-ethyloxycarbonylaminomethyl-8-azabicyclo[4.3.0]non-4'-en-8'-yl)-6,8-difluor-1,4-dihydro-4-oxo-3-chinolincarbonsäure

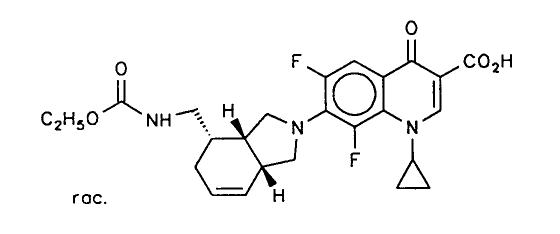

Analog Beispiel 9 erhält man aus 0,63 g (2,23 mmol) 1-Cyclopropyl-6,7,8-trifluor-1,4-dihydro-4-oxo-3-chinolincarbonsäure und 0,60 g (2,68 mmol) der Titelverbindung aus Beispiel H die Titelverbindung.
- Ausbeute:: 0,7 g (65 % der Theorie)
- Schinelzpunkt:: 226-229 C
¹H-NMR (CDCl₃): δ = 8,71 (s, 1H); 7,76 (d, 1H); 5,79 (m, 1H); 5,54 (d, 1H); 4,91 (br, 1H); 4,12 (m, 3H); 3,96 (m, 1H); 3,79 (m, 1H); 3,56 (m, 1H); 3,47 (m, 1H); 3,18 (m, 2H); 2,91 (m, 1H); 2,65 (m, 1H); 2,20 (m, 2H); 1,91 (m, 1H); 1,08-1,33 ppm (m, 7H).

### Beispiel 30:

### (1'RS,2'SR,6'RS)-7-(2'-Aminomethyl-8'-azabicyclo[4.3.0]non-4'-en-8'-yl)-1-cyclopropyl-6,8-difluor-1,4-dihydro-4-oxo-3-chinolincarbonsäure

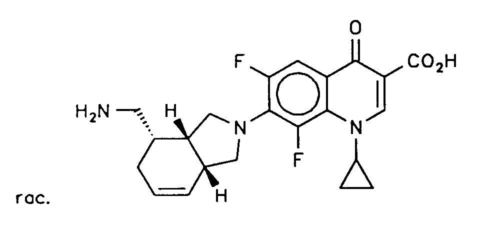

Analog Beispiel 10 erhält man aus 0,48 g (1,0 mmol) der Titelverbindung aus Beispiel 29 die Titelverbindung.
- Ausbeute:: 0,39 g (86 % der Theorie) der Titelverbindung als Hydrochlorid
- Schmelzpunkt:: 304 C
¹H-NMR (CF₃CO₂D): δ = 9,20 (s, 1H); 8,02 (d, 1H); 5,91 (m, 1H); 5,68 (d, 1H); 4,43 (m, 2H); 4,12 (m, 1H); 3,94 (m, 1H); 3,87 (m, 1H); 3,28-3,42 (m, 2H); 3,14 (m, 1H); 2,87 (m, 1H); 2,67 (m, 1H); 2,46 (m, 1H); 2,12 (m, 1H); 1,56 (m, 2H); 1,38 ppm (m, 2H).

### Beispiel 31:

### (1'SR,2'RS,3'RS,6'SR)-1-Cyclopropyl-7-(2'-ethyloxycarbonylamino-3'-methyl-8'-azabicyclo[4.3.0]non-4'-en-8'-yl)-6,8-difluor-1,4-dihydro-4-oxo-3-chinolincarbonsäure

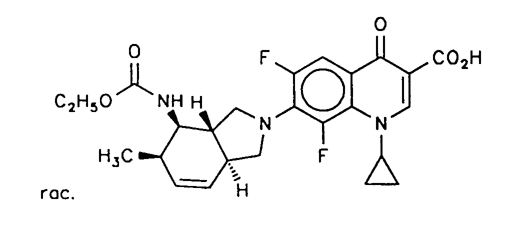

Analog Beispiel 9 erhält man aus 0,59 g (2,1 mmol) 1-Cyclopropyl-6,7,8-trifluor-1,4-dihydro-4-oxo-3-chinolincarbonsäure und 0,6 g (2,6 mmol) der Titelverbindung aus Beispiel I die Titelverbindung.
- Ausbeute:: 0,8 g (78 % der Theorie)
- Schmelzpunkt:: 258-259 C

### Beispiel 32:

### (1'SR,2'RS,3'RS,6'SR)-7-(2'-Amino-3'-methyl-8'-azabicyclo[4.3.0]non-4'-en-8'-yl)-1-cyclopropyl-6,8-difluor-1,4-dihydro-4-oxo-3-chinolincarbonsäure

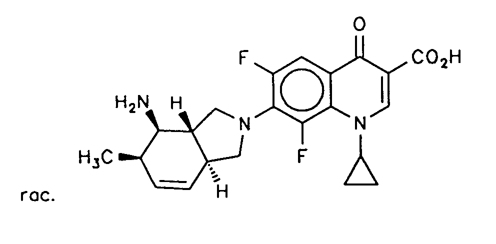

Analog Beispiel 10 erhält man aus 0,6 g (1,23 mmol) der Titelverbindung aus Beispiel 31 die Titelverbindung.
- Ausbeute:: 0,55 g (99 % der Theorie) der Titelverbindung als Hydrochlorid
- Schmelzpunkt:: > 300 C
¹H-NMR (CF₃CO₂D): δ = 9,24 (s, 1H); 8,06 (d, 1H); 5,98 (d, 1H); 5,86 (m, 1H); 4,47 (m, 1H); 4,07-4,32 (m, 3H); 3,95 (m, 1H); 3,10 (m, 1H); 2,82 (m, 1H); 2,50 (m, 1H); 1,57 (m 2H); 1,40 (m 2H); 1,32 ppm (d, 3H).

### Beispiel 33:

### (1'SR,2'RS,6'SR)-1-Ethyl-7-(2'-ethyloxycarbonylamino-8'-azabicyclo[4.3.0]non-4'-en-8'-yl)-6,8-difluor-1,4-dihydro-4-oxo-3-chinolincarbonsäure

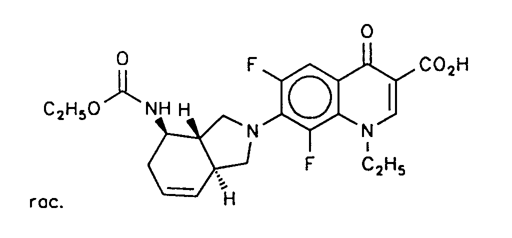

Analog Beispiel 9 erhält man aus 1,12 g (4,16 mmol) 1-Ethyl-6,7,8-trifluor-1,4-dihydro-4-oxo-3-chinolincarbonsäure und 1,05 g (5,0 mmol) der Titelverbindung aus Beispiel C die Titelverbindung.
- Ausbeute:: 1,9 g (99 % der Theorie)
- Schmelzpunkt:: 285-287 C

### Beispiel 34:

### (1'SR,2'RS,6'SR)-7-(2'-Amino-8'-azabicyclo[4.3.0]non-4'-en-8'-yl)-1-ethyl-6,8-difluor-1,4-dihydro-4-oxo-3-chinolincarbonsäure

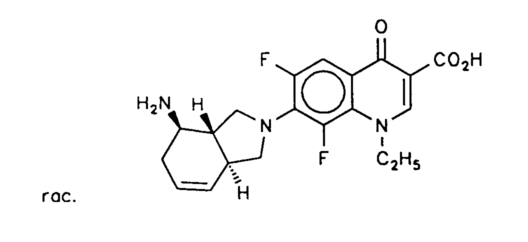

Analog Beispiel 10 erhält man aus 0,6 g (1,23 mmol) der Titelverbindung aus Beispiel 33 die Titelverbindung.
- Ausbeute:: 1,44 g (97 % der Theorie) der Titelverbindung als Hydrochlorid
- Schmelzpunkt:: > 300 C
¹H-NMR (CF₃CO₂D): δ = 9,12 (s, 1H); 8,10 (d, 1H); 6,02 (d, 1H); 5,88 (m, 1H); 4,94 (m, 2H); 4,37 (m, 1H); 4,21 (m, 2H); 4,03 (m, 1H); 3,95 (m, 1H); 2,99 (m, 1H); 2,83 (m, 1H); 2,58 (m, 1H); 2,49 (m, 1H); 1,78 ppm (t, 3H).

### Beispiel 35:

### (1'SR,2'RS,6'SR)-8-Chlor-1-cyclopropyl-7-(2'-ethyloxycarbonylamino-8'-azabicyclo[4.3.0]non-4'-en-8'-yl)-1,4-dihydro-4-oxo-3-chinolincarbonsäure

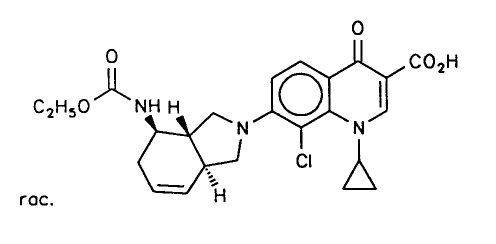

1,06 g (3,75 mmol) 8-Chlor-1-cyclopropyl-7-fluor-4-oxo-3-chinolincarbonsäure, 0,84 g (7,5 mmol) DABCO und 0,95 g (4,5 mmol) der Titelverbindung aus Beispiel C in 35 ml DMSO 4 h auf 100 C erwärmen. Das Lösungsmittel im Hochvakuum abdestillieren, den Rückstand mit Acetonitril verrühren, absaugen und bei 100 C trocknen.
- Ausbeute:: 1,5 g (85 % der Theorie)
- Schmelzpunkt:: 260-261 C

### Beispiel 36:

### (1'SR,2'RS,6'SR)-7-(2'-Amino-8'-azabicyclo[4.3.0]non-4'-en-8'-yl)-8-chlor-1-cyclopropyl-1,4-dihydro-4-oxo-3-chinolincarbonsäure

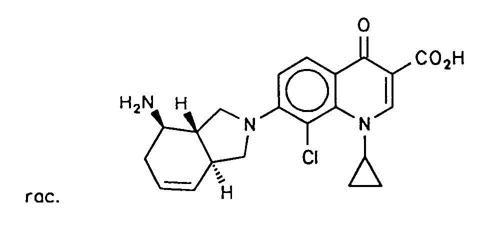

Analog Beispiel 16 erhält man aus 1,5 g (3,2 mmol) der Titelverbindung aus Beispiel 35 die Titelverbindung.
- Ausbeute:: 0,72 g (52 % der Theorie) der Titelverbindung als Hydrochlorid
- Schmelzpunkt:: > 300 C

### Beispiel 37:

### 6,8-Difluor-1-[(1RS,2SR)-2-fluorcyclopropyl]-1,4-dihydro-7-[(1'SR,2'RS,6'SR)-2'-methylamino-8'-azabicyclo[4.3.0]non-4'-en-8'-yl]-4-oxo-3-chinolincarbonsäure und

### 6,8-Difluor-1-[(1SR,2RS)-2-fluorcyclopropyl]-1,4-dihydro-7-[(1'SR,2'RS,6'SR)-2'-methylamino-8'-azabicyclo[4.3.0]non-4'-en-8'-yl]-4-oxo-3-chinolincarbonsäure

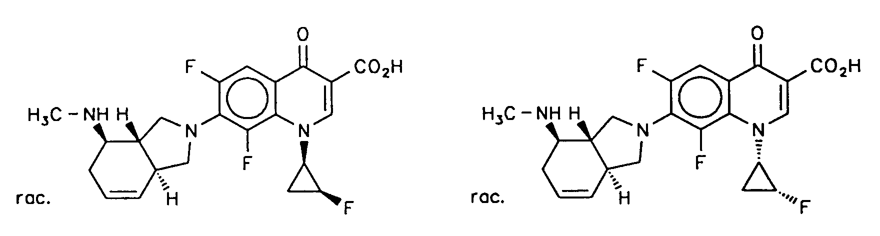

151 mg (0,5 mmol) 6,7,8-Trifluor-1-[(1RS,2SR)-2-fluorcyclopropyl]-1,4-dihydro-4-oxo-3-chinolincarbonsäure, 63 mg (0,55 mmol) DABCO und 84 mg (0,55 mmol) der Titelverbindung aus Beispiel D in einer Mischung aus 2 ml Acetonitril und 1 ml DMF 1 h zum Rückfluß erhitzen. Nach Abkühlung die Suspension absaugen, den Niederschlag in 30 ml Wasser suspendieren, erneut absaugen, bei 70 C trocknen.
- Ausbeute:: 93 mg (43 % der Theorie)
- Schmelzpunkt:: > 300 C
¹H-NMR (CF₃CO₂D): δ = 9,20 und 9,24 (2s, 1H); 8,08 (d, 1H); 6,04 (d, 1H); 5,90 (m, 1H); 5,14 (dm, 1H); 4,38 (m, 1H); 4,32 (m, 1H); 4,20 (m, 2H); 3,82-3,98 (m, 2H); 3,05 (s, 3H); 2,98 (m, 1H); 2,84 (m, 1H); 2,56 (m, 1H); 2,43 (m, 1H); 1,78-2,04 ppm (m, 2H).

### Beispiel 38:

### 8-Chlor-6-fluor-1-[(1RS,2SR)-2-fluorcyclopropyl]-1,4-dihydro-7-[(1'SR,2'RS,6'SR)-2'-methylamino-8'-azabicyclo[4.3.0]non-4'-en-8'-yl]-4-oxo-3-chinolincarbonsäure und

### 8-Chlor-6-fluor-1-[(1SR,2RS)-2-fluorcyclopropyl]-1,4-dihydro-7-[(1'SR,2'RS,6'SR)-2'-methylamino-8'-azabicyclo[4.3.0]non-4'-en-8'-yl]-4-oxo-3-chinolincarbonsäure

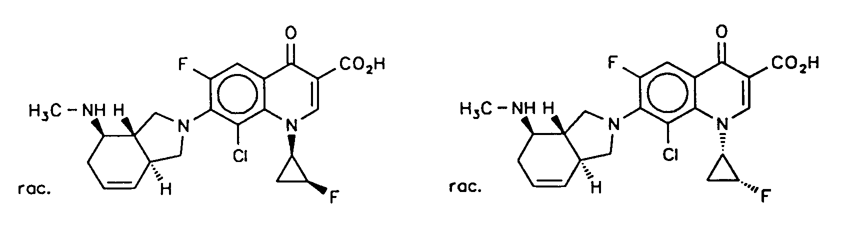

Analog Beispiel 37 erhält man aus 159 mg (0,5 mmol) 8-Chlor-6,7-difluor-1-[(1RS,2SR)-2-fluorcyclopropyl]-1,4-dihydro-4-oxo-3-chinolincarbonsäure und 84 mg (0,55 mmol) der Titelverbindung aus Beispiel D die Titelverbindung.
- Ausbeute:: 130 mg (58 % der Theorie)
- Schmelzpunkt:: 247-249 C
¹H-NMR (CF₃CO₂D): δ = 9,22 und 9,43 (d und s, 1H); 8,16 (d, 1H); 6,05 (d, 1H); 5,92 (m, 1H); 5,07 und 5,20 (2dm, 1H); 4,64 und 4,80 (2m, 1H); 4,41-4,54 (m, 1H); 4,18 und 4,26 (2m, 1H); 4,07 (m, 1H); 3,86-3,97 (m, 2H); 3,06 (s, 3H); 2,98 (m, 1H); 2,90 (m, 1H); 2,55 (m, 1H); 2,47 (m, 1H); 1,88 und 2,07 (2m, 1H); 1,43 und 1,72 ppm (2m, 1H).

### Beispiel 39:

### 6,8-Difluor-1-[(1RS,2SR)-2-fluorcyclopropyl]-1,4-dihydro-7-[(1'SR,2'RS,6'SR)-2'-ethyloxycarbonylamino-8'-azabicyclo[4.3.0]non-4'-en-8'-yl]-4-oxo-3-chinolincarbonsäure und

### 6,8-Difluor-1-[(1SR,2RS)-2-fluorcyclopropyl]-1,4-dihydro-7-[(1'SR,2'RS,6'SR)-2'-ethyloxycarbonylamino-8'-azabicyclo[4.3.0]non-4'-en-8'-yl]-4-oxo-3-chinolincarbonsäure

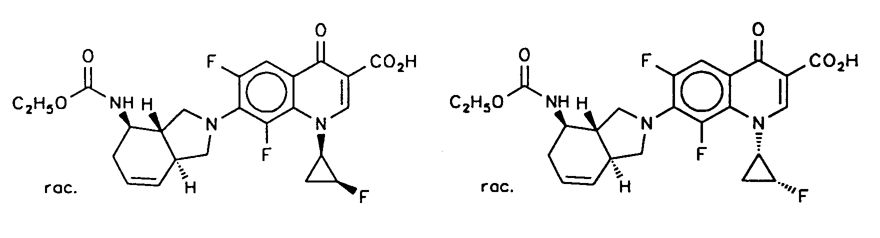

Analog Beispiel 37 erhält man aus 301 mg (1 mmol) 6,7,8-Trifluor-1-[(1RS,2SR)-2-fluorcyclopropyl]-1,4-dihydro-4-oxo-3-chinolincarbonsäure und 231 mg (1,1 mmol) der Titelverbindung aus Beispiel C die Titelverbindung.
- Ausbeute:: 428 mg (89 % der Theorie)
- Schmelzpunkt:: 282-283 C
¹H-NMR (CF₃CO₂D): δ = 9,16 und 9,22 (2s, 1H); 8,04 (d, 1H); 5,91 (d, 1H); 5,85 (m, 1H); 5,13 (dm, 1H); 4,37 (m, 3H); 4,16 (m, 4H); 3,91 (m, 1H); 2,72-2,87 (m, 2H); 2,20 (m, 2H); 1,66-2,04 (m, 2H) 1,42 ppm (t, 3H).

### Beispiel 40:

### (1'RS,2'RS,6'SR)-8-Chlor-1-cyclopropyl-7-(2'-ethoxycarbonylaminoethyl-8'-azabicyclo[4.3.0]non-4'en-8'-yl)-6-fluor-1,4-dihydro-4-oxo-3-chinolincarbonsäure

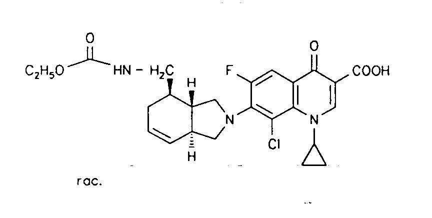

Die Titelverbindung wird erhalten, indem 8-Chlor-1-cyclopropyl-6,7-difluor-1,4-dihydro-4-oxo-3-chinolincarbonsäure mit der Titelverbindung aus Beispiel F umgesetzt wird, wie im Beispiel 26 beschrieben.
- Ausbeute:: quantitativ
¹H-NMR (DMSO-d₆): 8.81 (s, 1 H, 2-H); 7.89 (d, 1 H, 5-H); 7.19 (t, 1 H, Carbamat-NH); 5.71; 5.58 (2m, 2x 1 H, HC=CH); 4.38 (m, 1 H, Cyclopropyl-H); 3.99 (q, 2 H, Ethyl-CH₂); 3.45 - 3.40 (m, 3 H); 3.12 (m, 1 H); 3.00 (m, 2H), 2.30 (m, 2 H), 2.12 (m, 1 H); 1.96 - 1.87 (m, 2 H), 1.20 - 1.11 (m, 5 H, 2x Cyclopropyl-H, Ethyl-CH₃), 1.00 - 0.90 ppm (m, 2 H, 2x Cyclopropyl-H).

### Beispiel 41:

### (1'RS,2'RS,6'SR)-7-(2'-Aminomethyl-8'-aza-bicyclo[4.3.0]non-4'-en-8'-yl)-8-chlor-1-cyclopropyl-6-fluor-1,4-dihydro-4-oxo-3-chinolincarbonsäure

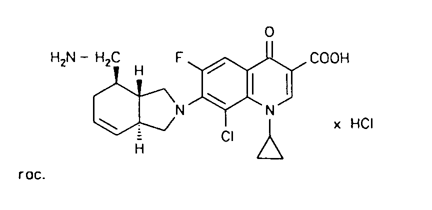

Das Produkt aus Beispiel 40 wird umgesetzt, wie im Beispiel 27 beschrieben.
- Ausbeute:: 82 % der Theorie, Hydrochlorid.
¹H-NMR (DMSO-d₆): 8.82 (s, 1 H, 2-H); 7.91 (d, 1 H, 5-H); 5.88; 5.60 (2 m, 2x 1 H, HC=CH);4.39 (m, 1 H, Cyclopropyl-H); 3.45 - 3.35 (m, 3 H); 3.18 (m, 1 H); 2.80 (m, 2 H); 2.46 (m, 1 H); 2.31 (m, 1 H); 2.19 (m, 1 H); 2.06 (m, 2 H); 1.19; 0.95 ppm (2m, 2x 2 H, 4x Cyclopropyl-H).

### Beispiel 42:

### (1'RS,2'RS,6'SR)-1-Cyclopropyl-7-(2'-ethoxycarbonylaminomethyl-8'-azabicyclo[4.3.0]-non-4'en-8'-yl)-6-fluor-1,4-dihydro-4-oxo-3-chinolincarbonsäure

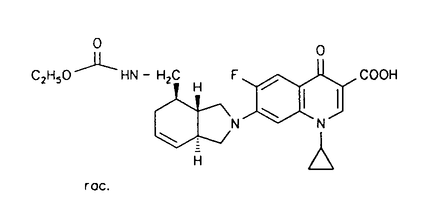

Die Titelverbindung wird erhalten, indem 1-Cyclopropyl-6,7-difluor-1,4-dihydro-4-oxo-3 -chinolincarbonsäure mit der Titelverbindung aus Beispiel F umgesetzt wird, wie im Beispiel 26 beschrieben.
- Ausbeute:: 95 % der Theorie.
¹H-NMR (DMSO-d₆): 8.61 (s, 1 H, 2-H), 7.81 (d, 1 H, 5-H), 7.49 (d, 1 H, 8-H); 7.21 (t, 1 H, Carbamat-NH); 5.80; 5.63 (2m, 2x 1 H, HC=CH); 4.00 (q, 2 H, Ethyl-CH₂); 3.82 - 3.78 (m, 2 H), 3.63 (m, 1 H); 3.18 - 3.00 (m, 4 H); 2.38 (m, 2 H); 2.00 (m, 2 H); 1.89 (m, 1 H); 1.30 (m, 2 H, 2x Cyclopropyl-H); 1.17 ppm (m, 5 H, 2x Cyclopropyl-H, Ethyl-CH₃).

### Beispiel 43:

### (1'RS,2'RS,6'SR)-7-(2'-Aminomethyl-8'-azabicyclo[4.3.0]-non-4'en-8'-yl)-1-cyclopropyl-6-fluor-1,4-dihydro-4-oxo-3-chinolincarbonsäure

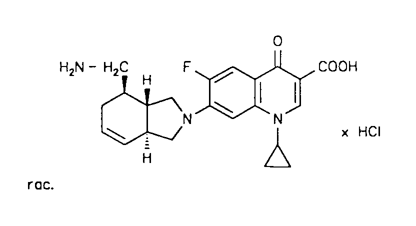

Das Produkt aus Beispiel 42 wird umgesetzt, wie im Beispiel 27 beschrieben.
- Ausbeute:: 72 % der Theorie, Hydrochlorid.
¹H-NMR (DMSO-d₆): 8.64 (s, 1 H, 2-H); 7.87 (d, 1 H, 5-H), 7.51 (d, 1 H, 8-H); 5.84; 5.68 (2m, 2x 1 H, HC=CH); 3.84 - 3.78 (m, 2 H); 3.68 (m, 1 H); 3.17 (m, 1 H); 3.09 (m, 1 H); 2.81 (m, 2 H); 2.53 (m, 2H), 2.36 (m, 1 H); 2.19 (m, 1 H); 2.05 (m, 1 H); 1.31; 1.19 ppm (2m, 2x 2 H, 4x Cyclopropyl-H).

### Beispiel 44:

### (1'RS,2'RS,6'RS)-1-Cyclopropyl-7-(2'-ethoxycarbonylaminomethyl-8'-aza bicyclo[4.3.0]-non-4'-en-8'-yl)-6,8-difluor-1,4-dihydro-4-oxo-3-chinolincarbonsäure

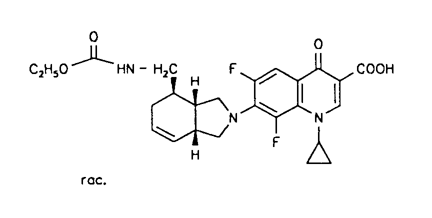

Die Titelverbindung wird erhalten, indem 1-Cyclopropyl-6,7,8-trifluor-1,4-dihydro-4-oxo-3-chinolincarbonsäure mit der Titelverbindung aus Beispiel K umgesetzt wird, wie im Beispiel 26 beschrieben.
- Ausbeute:: 91 % der Theorie.
¹H-NMR (CDCl₃): 8.71 (S, 1 H, 2-H); 7.80 (d, 2 H, 5-H); 5.80; 5.67 (2m, 2x 1 H, HC=CH); 4.80 (t, 1 H, Carbamat-NH); 4.12 (q, 2 H, Ethyl-CH₂); 3.97 (m, 2 H); 3.86 (m, 1 H); 3.81 (m, 1 H), 3.54 (m, 1 H), 3.37 (m, 1 H); 3.18 (m, 1 H), 2.86 (m, 1 H); 2.38 - 2.25 (m, 2 H); 2.00 -1.90 (m, 2 H); 1.30 - 1.20 (m, 5 H, 2x Cyclopropyl-H, Ethyl-CH₃), 1.14 ppm (m, 2 H, 2x Cyclopropyl-H).

### Beispiel 45:

### (1'RS,2'RS,6'RS)-7-(2'-Aminomethyl-8'-azabicyclo[4.3.0]-non-4'-en-8'-yl)-1-cyclopropyl-6,8-difluor-1,4-dihydro-4-oxo-3-chinolincarbonsäure

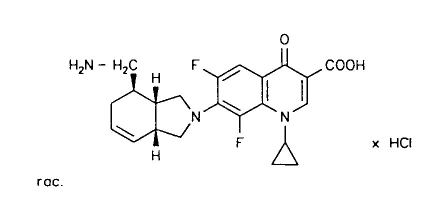

Das Produkt aus Beispiel 44 wird umgesetzt, wie im Beispiel 27 beschrieben.
- Ausbeute:: 84 % der Theorie, Hydrochlorid.
¹H-NMR (CF₃COOD): 9.21 (s, 1 H, 2-H), 8.04 (d, 1 H, 5-H), 5.91; 5.80 (2m, 2x 1 H, HC=CH); 4.45 (m, 1 H); 4.35 (m, 1 H); 4.18 (m, 2 H); 3.92 (m, 1 H); 3.55 (m, 1 H); 3.35 (m, 1 H); 3.08 (m, 1 H); 2.69 - 2.58 (m, 2 H), 2.47 (m, 1 H); 2.19 (m, 1 H), 1.57; 1.40 ppm (2m, 2x 2H, 4x Cyclopropyl-H).

### Beispiel 46:

### (1'RS,2'RS,6'RS)-8-Chlor-1-cyclopropyl-7-(2'-ethyloxycarbonylaminomethyl-8'-azabicyclo[4.3.0]non-4'en-8'-yl)-6-fluor-1,4-dihydro-4-oxo-3-chinolincarbonsäure

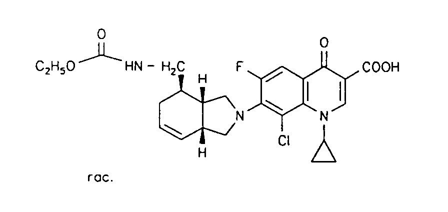

Die Titelverbindung wird erhalten, indem 8-Chlor-1-cyclopropyl-6,7-difluor-1,4-dihydro-4-oxo-3-chinolincarbonsäure mit der Titelverbindung aus Beispiel K umgesetzt wird, wie im Beispiel 26 beschrieben.
- Ausbeute:: quantitativ.
- Schmelzpunkt:: > 300 °C.

### Beispiel 47:

### (1'RS,2'RS,6'RS)-7-(2'-Aminomethyl-8'-aza-bicyclo[4.3.0]non-4'-en-8'-yl)-8-chlor-1-cyclopropyl-6-fluor-1,4-dihydro-4-oxo-3-chinolincarbonsäure

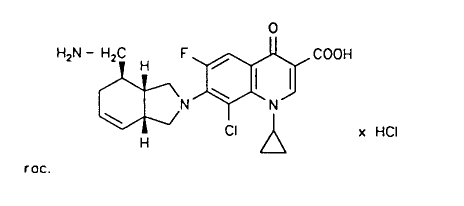

Das Produkt aus Beispiel 46 wird umgesetzt, wie im Beispiel 27 beschrieben.
- Ausbeute:: 58 % der Theorie, Hydrochlorid.
- Schmelzpunkt:: > 300 °C.

### Beispiel 48:

### (1'RS,2'RS,6'RS)-7-(2'-Aminomethyl-8'-aza-bicyclo[4.3.0]non-4'-en-8'-yl)-1-cyclopropyl-6-fluor-1,4-dihydro-4-oxo-3-chinolincarbonsäure

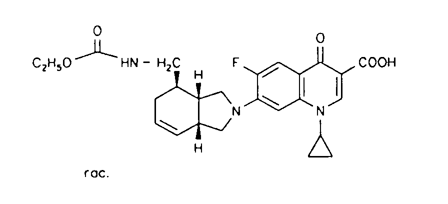

Die Titelverbindung wird erhalten, indem 1-Cyclopropyl-6,7-difluor-1,4-dihydro-4-oxo-3-chinolincarbonsäure mit der Titelverbindung aus Beispiel K umgesetzt wird, wie im Beispiel 26 beschrieben.
- Ausbeute:: 75 % der Theorie.
- Schmelzpunkt:: > 300 °C.

### Beispiel 49:

### (1'RS,2'RS,6'RS)-7-(2'-Aminomethyl-8'-aza-bicyclo[4.3.0]non-4'-en-8'-yl)-1-cyclopropyl-6-fluor-1,4-dihydro-4-oxo-3-chinolincarbonsäure

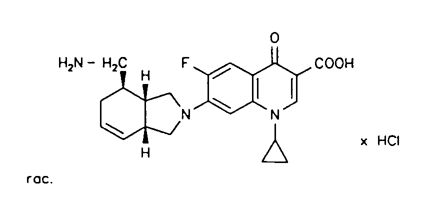

Das Produkt aus Beispiel 48 wird umgesetzt, wie im Beispiel 27 beschrieben.
- Ausbeute:: 77 % der Theorie, Hydrochlorid.
- Schmelzpunkt:: > 300 °C.

### Beispiel 50:

### (1'RS,2'RS, 6'RS)-10-(2'-Ethyloxycarbonylaminomethyl-8'-azabicyclo[4.3.0]non-4'-en-8'-yl)-9-fluor-2,3-dihydro-3-methyl-7-oxo-7H-pyrido[1,2,3-de][1,4]benzoxazin-6-carbonsäure

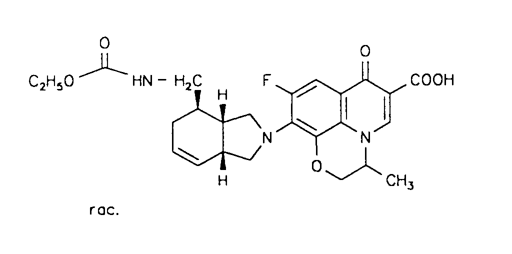

Die Titelverbindung wird erhalten indem 9,10-Difluor-2,3-dihydro-3-methyl-7-oxo-7H-pyrido-[1,2,3-de][1,4]benzoxyzin-6-carbonsäure mit der Titelverbindung aus Beispiel K umgesetzt wird, wie im Beispiel 13 beschrieben.
- Ausbeute:: 80 % der Theorie.
- Schmelzpunkt:: 190 °C.

### Beispiel 51:

### (1'RS,2'RS, 6'RS)-10-(2'-Aminomethyl-8'-azabicyclo[4.3.0]non-4'-en-8'-yl)-9-fluor-2,3-dihydro-3-methyl-7-oxo-7H-pyrido[1,2,3-de][1,4] benzoxazin-6-carbonsäure

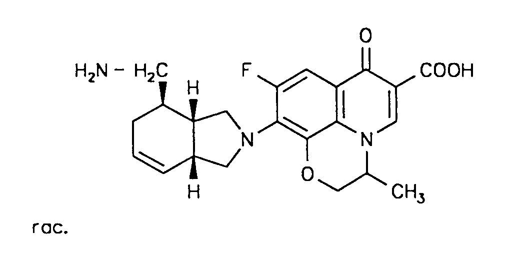

Das Produkt aus Beispiel 50 wird umgesetzt, wie im Beispiel 14 beschrieben.
- Ausbeute:: 95 % der Theorie, Hydrochlorid.
- Schmelzpunkt:: > 300 °C.

### Beispiel 52:

### Ethyl-(1'RS,2'RS,6'RS)-1-Cyclopropyl-7-(2'-ethyloxycarbonylaminomethyl-8'azabicyclo[4.3.0]non-4'-en-8'-yl)-6-fluor-1,4-dihydro-4-oxo-1,8-naphthyridin-3-carboxylat

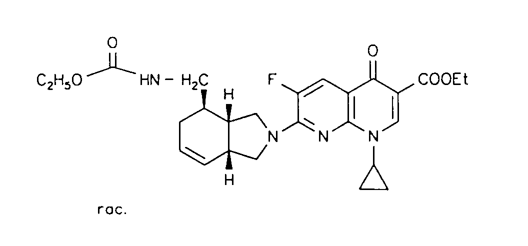

Ein Gemisch aus 828 mg (2.6 mmol) Ethyl-7-chlor-1-cyclopropyl-6-fluor-1,4-dihydro-4-oxo-1,8-naphthyridin-3-carboxylat, 900 mg (4 mmol) des Produktes aus Beispiel K und 20 ml Acetonitril wird drei Tage bei Raumtemperatur gerührt. Anschließend wird von unlöslichen Bestandteilen abgesaugt und die Lösung in vacuo eingeengt. Das Rohprodukt wird chromatographisch gereinigt (Laufmittel: Dichlormethan/Methanol/konz. Ammoniak 15:4:0.5).
- Ausbeute:: 700 mg (56 % der Theorie).
¹H-NMR (DMSO-d₃): 8.36 (s 1 H, 2.H); 7.81 (d, 1 H, 5-H); 7.21 (t, 1 H, Carbamat-NH); 5.73; 5.67 (2m, 2x 1 H. HC=CH); 4.20; 3.99 (2q, 2x 2 H, 2x Ethyl-CH₂); 3.86 (m, 1 H); 3.78 (m, 1 H); 3.56 (m, 2 H); 3.13 - 3.01 (m, 2 H); 2.89 (m, 1 H); 2.35 (m, 1 H); 2.18 (m, 2 H); 1.88 (m, 2 H); 1.26; 1.19 (2t, 2x 3H, 2x Ethyl-CH₃);1.02; 0.88 ppm (2x 2H, 4x Cyclopropyl-H).

### Beispiel 53:

### (1'RS,2'RS,6'RS)-7-(2'-Aminomethyl-8'azabicyclo[4.3.0]non-4'-en-8'-yl)-1-cyclopropyl-6-fluor-1,4-dihydro-4-oxo-1,8-naphthyridin-3-carbonsäure

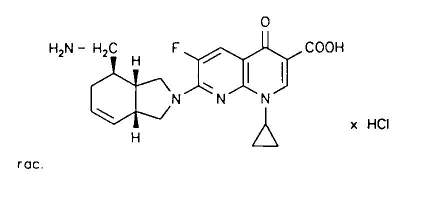

0.70 g des Produktes von Beispiel 52 werden in 7 ml 1,2-Ethandiol und 10 ml 10 %iger Kalilauge 4 h bei 130 °C gerührt. Nach dem Abkühlen wird mit Acetonitril verdünnt und mit verd. Salzsäure pH 2 eingestellt.Anschließend wird in vacuo auf ein Volumen von ca. 10 ml eingeengt und das Produkt durch Zugabe von Aceton ausgefällt. Die Kristalle werden bei 50 °C im Vakuum-Trockenschrank getrocknet.
- Ausbeute:: 0.30 g (47 % der Theorie), Hydrochlorid.
- Schmelzpunkt:: > 300 °C.

### Beispiel 54:

### 7-[(1'RS,2'RS,6'RS)-2'-Aminomethyl-8'-azabicyclo[4.3.0]non-4'-en-8'-yl]-6,8-difluor-1-[(1RS,2SR)-2-fluorcyclopropyl]-1,4-dihydro-4-oxo-)-chinolincarbonsäure-Hydrochlorid

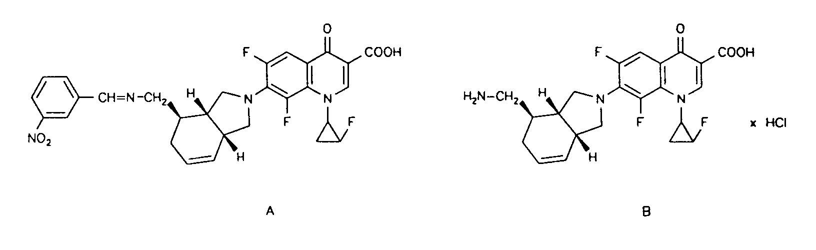

A. 380 mg (1 mmol) (1RS,2RS,6RS)-2-Aminomethyl-8-azabicyclo[4.3.0]non-4-en-Bis-trifluoracetat (Produkt aus Beispiel L) werden in 1 ml Acetonitril mit 112 mg (1 mmol) 1,4-Diazabicyclo[2.2.2]octan (Dabco) und einer Lösung von 155 mg 3-Nitrobenzaldehyd in 1 ml Acetonitril versetzt. Die Mischung wird 1 Stunde bei Raumtemperatur gerührt, mit 1 ml Dimethylformamid verdünnt, mit 224 mg (2 mmol) Dabco und 270 mg (0,9 mmol) 6,7,8-Trifluor-1-[(1RS,2SR)-2-fluorcyclopropyl]-1,4-dihydro-4-oxo-3-chinolincarbonsäure versetzt und 1 Stunde unter Rückfluß erhitzt. Man engt ein, verrührt mit Wasser, saugt ab und trocknet bei 80°C im Hochvakuum.
   Ausbeute: 480 mg 6,8-Difluor-1-[(1RS,2SR)-2-fluorcyclopropyl]-1,4-dihydro-7-[(1'RS,2'RS,6'RS)-2'-(3-nitrobenzylidenaminomethyl)-8-azabicyclo[4.3.0]non-4'-en-8'-yl]-4-oxo-3-chinolincarbonsäure,
   R_{f}-Wert (Kieselgel; Dichlormethan/Methanol/17%-Ammoniak = 30:8:1): 0,5.
B. 450 mg des Produktes aus der Stufe A werden in ca. 30 ml Dichlormethan gelöst und mit 3 ml 3n-Salzsäure versetzt. Die Mischung wird 30 Minuten bei Raumtemperatur gerührt, mit ca. 30 ml Wasser versetzt. Die wäßrige Phase wird abgetrennt, mit Dichlormethan gewaschen und lyophilisiert.
   Ausbeute: 170 mg 7-[(1'RS,2'RS,6'RS)-2'-Aminomethyl-8'-azabicyclo[4.3.0]non-4'-en-8'-yl]-6,8-difluor-1-[(1RS,2SR)-2-fluorcyclopropyl]-1,4-dihydro-4-oxo-3-chinolincarbonsäure-Hydrochlorid,
   R_{f}-Wert (Kieselgel; Dichlormethan/Methanol/17%-Ammoniak = 30:8:1): 0,06.
   ¹H-NMR (CF₃COOD): δ = 5,07 m und 5,2 ppm m (1H, CH-F).

### Beispiel 55:

### (1'SR,2'RS,6'RS)-1-Cyclopropyl-6,8-difluoro-1,4-dihydro-7-(2'-methylamino-8'-azabicyclo[4.3.0]non-4'-en-8'-yl)-4-oxo-3-chinolincarbonsäure

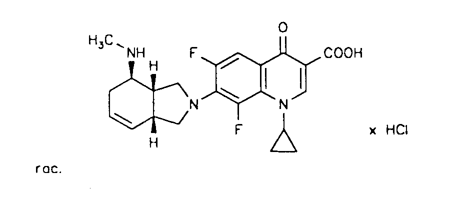

Die Titelverbindung wird erhalten, indem 1 -Cyclopropyl-6,7,8-trifluor-1,4-dihydro-4-oxo-3-chinolincarbonsäure mit der Titelverbindung aus Beispiel N umgesetzt wird, wie im Beispiel 22 beschrieben.
- Ausbeute:: 61 % der Theorie, Hydrochlorid.
- Schmelzpunkt:: > 300 °C.

### Beispiel 56:

### (1'SR,2'RS,6'RS)-1-Cyclopropyl-6-fluoro-1,4-dihydro-7-(2'-methylamino-8'-azabicyclo[4.3.0]non-4'-en-8'-yl)-4-oxo-3-chinolincarbonsäure

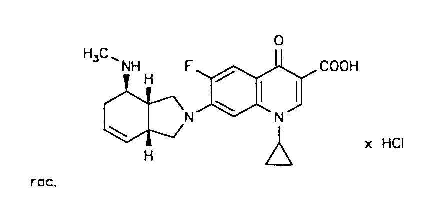

Die Titelverbindung wird erhalten, indem 1-Cyclopropyl-6,7-difluor-1,4-dihydro-4-oxo-3-chinolincarbonsäure mit der Titelverbindung aus Beispiel N umgesetzt wird, wie im Beispiel 22 beschrieben.
- Ausbeute:: 60 % der Theorie, Hydrochlorid.
- Schmelzpunkt:: > 300 °C.

### Beipiel 57:

### (1'SR,2'RS,6'RS)-8-Chlor-1-cyclopropyl-6-fluoro-1,4-dihydro-7-(2'-methylamino-8'-azabicyclo[4.3.0]non-4'-en-8'-yl)-4-oxo-3-chinolincarbonsäure

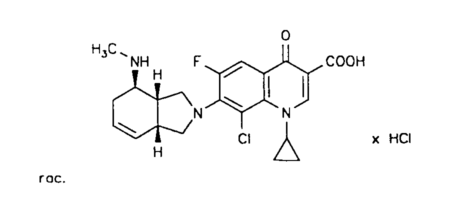

Die Titelverbindung wird erhalten, indem 8-Chlor-1-Cyclopropyl-6,7-difluor-1,4-dihydro-4-oxo-3-chinolincarbonsäure mit der Titelverbindung aus Beispiel N umgesetzt wird, wie im Beispiel 22 beschrieben.
- Ausbeute:: 70 % der Theorie, Hydrochlorid.
- Schmelzpunkt:: > 300 °C.

### Beispiel 58:

### (1'SR,2'SR,6'RS)-1-Cyclopropyl-6,8-difluoro-1,4-dihydro-7-(2'-methylamino-8'-azabicyclo[4.3.0]non-4'-en-8'-yl)-4-oxo-3-chinolincarbonsäure

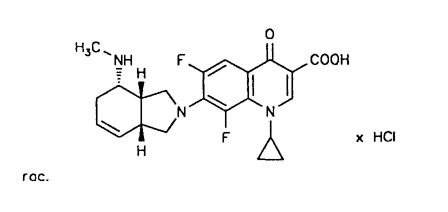

Die Titelverbindung wird erhalten, indem 1-Cyclopropyl-6,7,8-trifluor-1,4-dihydro-4-oxo-3-chinolincarbonsäure mit der Titelverbindung aus Beispiel O umgesetzt wird, wie im Beispiel 22 beschrieben.
- Ausbeute:: 35 % der Theorie, Hydrochlorid.
- Schmelzpunkt:: > 300 °C.

### Beispiel 59:

### (1'SR,2'SR,6'RS)-8-Chlor-1-cyclopropyl-6-fluoro-1,4-dihydro-7-(2'-methylamino-8'-azabicyclo[4.3.0]non-4'-en-8'-yl)-4-oxo-3-chinolincarbonsäure

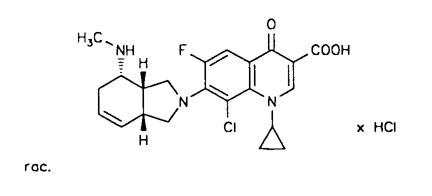

Die Titelverbindung wird erhalten, indem 8-Chlor-1-Cyclopropyl-6,7-difluor-1,4-dihydro-4-oxo-3-chinolincarbonsäure mit der Titelverbindung aus Beispiel O umgesetzt wird, wie im Beispiel 22 beschrieben.
- Ausbeute:: 78 % der Theorie, Hydrochlorid.
- Schmelzpunkt:: > 300 °C.

### Beispiel 60:

### (1'SR,2'SR,6'RS)-1-Cyclopropyl-6-fluoro-1,4-dihydro-7-(2'-methylamino-8'-azabicyclo[4.3.0]non-4'-en-8'-yl)-4-oxo-3-chinolincarbonsäure

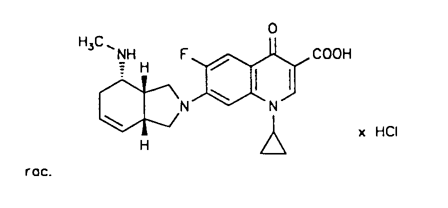

Die Titelverbindung wird erhalten, indem 1-Cyclopropyl-6,7-difluor-1,4-dihydro-4-oxo-3-chinolincarbonsäure mit der Titelverbindung aus Beispiel O umgesetzt wird, wie im Beispiel 22 beschrieben.
- Ausbeute:: 66 % der Theorie, Hydrochlorid.
- Schmelzpunkt:: > 300 °C.

### Beispiel 61:

### Ethyl-(1'SR,2'SR,6'RS)-1-cyclopropyl-6-fluor-1,4-dihydro-7-(2'-methylamino-8'-azabicyclo[4.3.0]non-4'-en-8'-yl)-4-oxo-1,8-naphthyridin-3-carboxylat

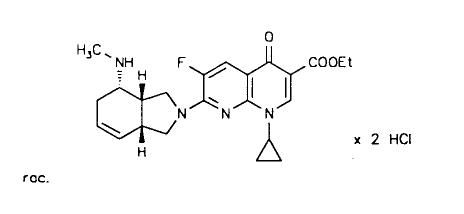

Die Titelverbindung wird erhalten, indem Ethyl-7-chlor-1-cyclopropyl-6-fluor-1,4-dihydro-4-oxo-1,8-naphthydridin-3-carboxylat mit der Titelverbindung aus Beispiel O umgesetzt wird, wie im Beispiel 52 beschrieben. Durch Behandlung mit wenig verd. Salzsäure erhält man das Hydrochlorid.
- Ausbeute:: 91 % der Theorie.
- RF =: 0.64 (Methanol/Dichlormethan/konz. Ammoniak 15:4:0.5).

### Beispiel 62:

### (1'SR,2'SR,6'RS)-1-Cyclopropyl-6-fluor-1,4-dihydro-7-(2'-methylamino-8'-azabicyclo[4.3.0]non-4'-en-8'-yl)-4-oxo-1,8-napthyridin-3-carbonsäure

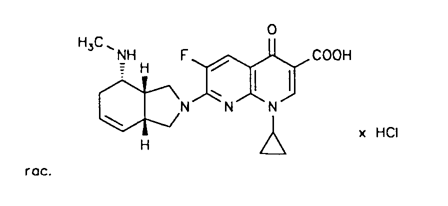

Das Produkt aus Beispiel 61 wird umgesetzt, wie im Beispiel 27 beschrieben.
- Ausbeute:: 89 der Theorie, Hydrochlorid.
- Schmelzpunkt:: > 300 °C.
- RF =: 0.32 (Methanol/Dichlromethan/konz. Ammoniak 15:5:0.5).

### Verwendete Abkürzungen:

- h: Stunde(n)
- min: Minute(n)
- konz.: konzentriert
- DMF: Dimethylformamid
- THF: Tetrahydrofuran
- MTBE: tert.-Butyl-methylether
- DIBAH: Diisobutylaluminiumhydrid
- DABCO: 1,4-Diaza-bicyclo[2.2.2]octan
- DMSO: Dimethylsulfoxid

## Patentansprüche

1. Verbindungen der Formel (I) 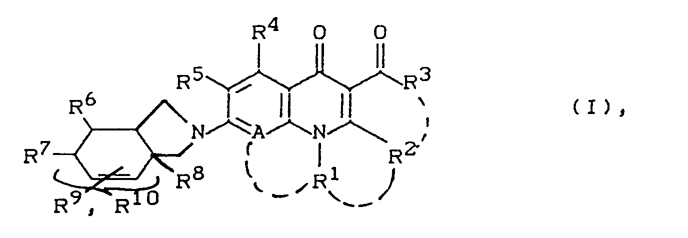 in welcher
R¹ für gegebenenfalls durch 1-3 Fluoratome substituiertes Alkyl mit 1 bis 4 Kohlenstoffatomen, Alkenyl mit 2 bis 4 Kohlenstoffatomen, gegebenenfalls durch 1 bis 2 Fluoratome substituiertes Cycloalkyl mit 3 bis 6 Kohlenstoffatomen, 2-Hydroxyethyl, Methoxy, Amino, Methylamino, Ethylamino, Dimethylamino, gegebenenfalls durch 1 oder 2 Fluoratome substituiertes Phenyl, 3-Oxetanyl, Bicyclo[1.1.1]pentyl,
R² für Wasserstoff steht oder auch gemeinsam mit R¹ eine Brücke bilden kann, so daß ein 4- oder 5-gliedriger Ring entsteht,
R³ für Hydroxy oder O-R¹¹ steht, worin
R¹¹ für Alkyl mit 1-4 C-Atomen steht oder
R³ gemeinsam mit R² eine Brücke bilden kann, so daß ein 5- oder 6-gliedriger Ring entsteht,
R⁴ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Halogen, Methyl, Ethyl oder Vinyl,
R⁵ für Wasserstoff, Halogen oder Methyl,
R⁶ für Wasserstoff, Alkyloxycarbonyl mit 1 bis 4
Kohlenstoffatomen, Hydroxymethyl, 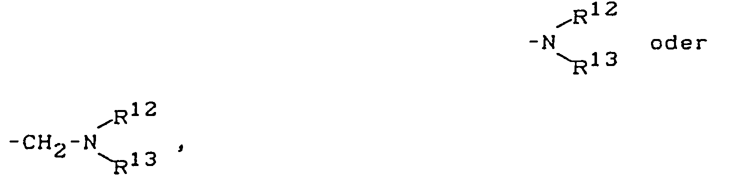 wobei
R¹² Wasserstoff, gegebenenfalls durch Hydroxy substituiertes Alkyl mit 1 bis 3 Kohlenstoffatomen, Alkyloxycarbonyl mit 1 bis 4 Kohlenstoffatomen im Alkylteil oder Acyl mit 1 bis 3 Kohlenstoffatomen und
R¹³ Wasserstoff oder Methyl bedeutet,
R⁷, R⁸, R⁹ und R¹⁰ für Wasserstoff oder Methyl steht oder R⁹ und R¹⁰ für Wasserstoff oder Methyl steht und R⁷ gemeinsam mit R⁸ eine Brücke der Struktur -O-, -CH₂- oder -CH₂-CH₂-bilden kann und
A für N oder C-R¹⁴ steht, worin
R¹⁴ für Wasserstoff, Halogen, gegebenenfalls durch 1 bis 3 Fluoratome substituiertes Methyl, Ethinyl, Vinyl, Hydroxy oder Methoxy steht, oder auch gemeinsam mit R¹ eine Brücke bilden kann, so daß ein 5- oder 6-gliedriger Ring entsteht, oder auch gemeinsam mit R¹ und R² eine Brücke bilden kann, so daß zwei 5- oder 6-gliedrige Ringe entstehen,
und deren Tautomere sowie deren pharmazeutisch verwendbare Hydrate und Säureadditionssalze.

2. Verbindungen der Formel (I) gemäß Anspruch 1, in welcher
R¹ für Cyclopropyl, 2-Fluorcyclopropyl, Ethyl, 2-Fluorethyl, gegebenenfalls durch 1, 2 oder 3 Fluoratome substituiertes tert.-Butyl oder gegebenenfalls durch 1 oder 2 Fluoratome substituiertes Phenyl, 3-Oxetanyl, Bicyclo[1.1.1]pentyl,
R² für Wasserstoff steht
oder auch gemeinsam mit R¹ eine Brücke der Struktur 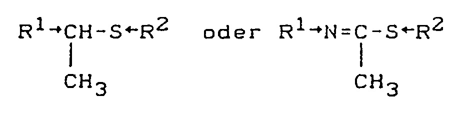 bilden kann,
R³ für Hydroxy oder O-R¹¹ steht, worin
R¹¹ für Alkyl mit 1-4 C-Atomen steht, oder
R³ gemeinsam mit R² eine Brücke der Struktur 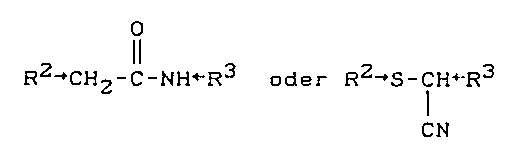 bilden kann,
R⁴ für Wasserstoff, Fluor, Chlor, Amino, Hydroxy, Methyl oder Vinyl,
R⁵ für Wasserstoff, Fluor, Chlor, Brom oder Methyl,
R⁶ für Amino, Methylamino, Ethoxycarbonylamino, Aminomethyl, Ethoxycarbonylaminomethyl, Hydroxymethyl, Ethoxycarbonyl oder Wasserstoff,
R⁷, R⁸, R⁹ und R¹⁰ für Wasserstoff oder Methyl steht oder R⁹ und R¹⁰ für Wasserstoff oder Methyl steht und R⁷ gemeinsam mit R⁸ eine Brücke der Struktur -O-, -CH₂ oder -CH₂-CH₂-bilden kann und
A für N oder C-R¹⁴ steht, worin
R¹⁴ für Wasserstoff, Fluor, Chlor, Brom, gegebenenfalls durch 1 bis 3 Fluoratome substituiertes Methyl, Ethinyl, Vinyl oder Methoxy steht oder auch gemeinsam mit R¹ eine Brücke der Struktur 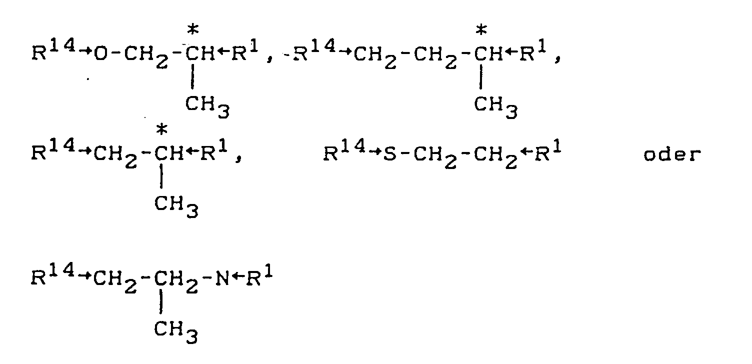 oder auch gemeinsam mit R¹ und R² eine Brücke der Struktur 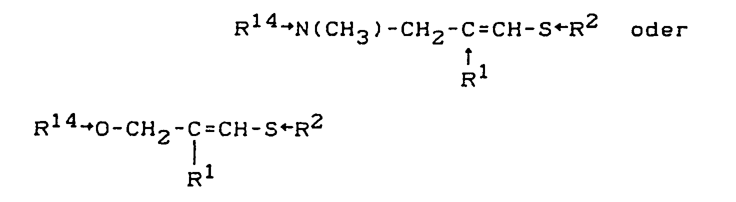
bilden kann.

3. Verbindungen der Formel (I) gemäß Anspruch 1, in welcher
R¹ für Cyclopropyl, cis-2-Fluorcyclopropyl, Ethyl, tert.-Butyl oder 2,4-Difluorphenyl,
R² für Wasserstoff,
R³ für Hydroxy oder Ethoxy,
R⁴ für Wasserstoff, Fluor oder Amino,
R⁵ für Wasserstoff oder Fluor,
R⁶ für Amino, Methylamino, Ethoxycarbonylamino, Aminomethyl, Ethoxycarbonylaminomethyl, Hydroxymethyl, Ethoxycarbonyl oder Wasserstoff,
R⁷, R⁸, R⁹ und R¹⁰ für Wasserstoff oder Methyl und
A für N oder C-R¹⁴ steht, worin
R¹⁴ für Wasserstoff, Fluor oder Chlor steht oder auch gemeinsam mit R¹ eine Brücke der Struktur 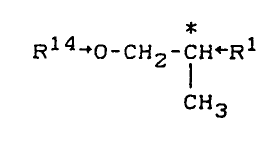 bilden kann.

4. Alkali-, Erdalkali-, Silber- und Guanidiniumsalze der zugrundeliegenden Carbonsäuren der Formel (I) gemäß den Ansprüchen 1 - 3.

5. Verfahren zur Herstellung von Verbindungen gemäß Ansprüchen 1 bis 3, Formel (I), dadurch gekennzeichnet, daß man eine Verbindung der Formel (II) 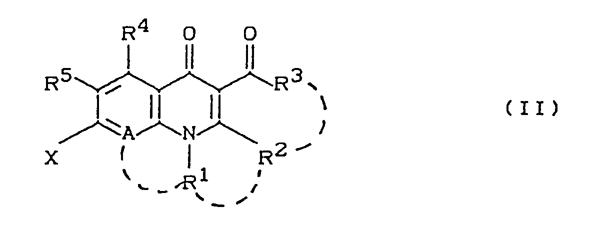 in welcher
R¹, R², R³, R⁴, R⁵ und A die in Ansprüchen 1 bis 3 angegebene Bedeutung haben und
X für Halogen, insbesondere Fluor oder Chlor steht,
mit Verbindungen der Formel (III) 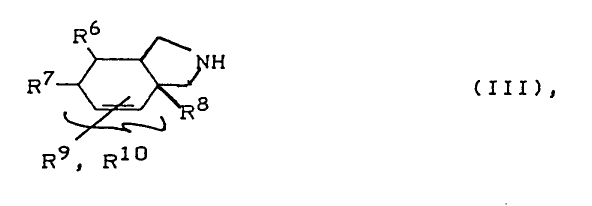 in welcher
R⁶, R⁷, R⁸, R⁹ und R¹⁰ die in Ansprüchen 1 bis 3 angegebene Bedeutung haben,
gegebenenfalls in Gegenwart von Säurebindern umsetzt und gegebenenfalls vorhandene Schutzgruppen durch alkalische oder saure Hydrolyse abspaltet und die erhaltenen Verbindungen gegebenenfalls in ihre Alkali-, Erdalkali-, Silber- oder Guanidiniumsalze überführt.

6. Isoindole der allgemeinen Formel (III) 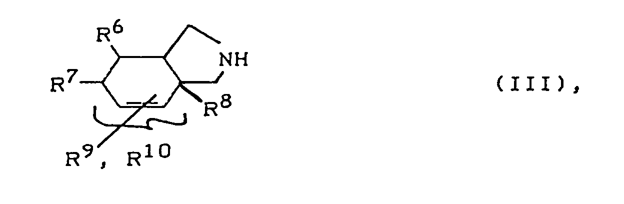 in welcher
R⁶ für Wasserstoff, Alkyloxycarbonyl mit 1 bis 4
Kohlenstoffatomen, Hydroxymethyl, 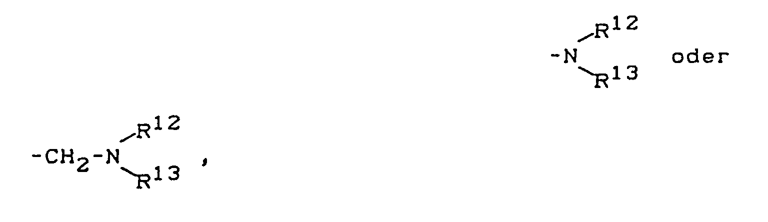 wobei
R¹² Wasserstoff, gegebenenfalls durch Hydroxy substituiertes Alkyl mit 1 bis 3 Kohlenstoffatomen, Alkyloxycarbonyl mit 1 bis 4 Kohlenstoffatomen im Alkylteil oder Acyl mit 1 bis 3 Kohlenstoffatomen und
R¹³ Wasserstoff oder Methyl bedeutet,
R⁷, R⁸, R⁹ und R¹⁰ für Wasserstoff oder Methyl steht oder R⁹ und R¹⁰ für Wasserstoff oder Methyl steht und R⁷ gemeinsam mit R⁸ eine Brücke der Struktur -O-, -CH₂- oder -CH₂-CH₂-bilden kann, mit Ausnahme der Verbindung 8-Azabicyclo[4.3.0]non-2-en.

7. Isoindole gemäß Anspruch 6 aus der Gruppe bestehend aus
8-Azabicyclo[4.3.0]non-4-en-2-carbonsäureethylester
2-Hydroxymethyl-8-azabicyclo[4.3.0]non-4-en
2-Amino-8-azabicyclo[4.3.0]non-4-en
2-Ethyloxycarbonylamino-8-azabicyclo[4.3.0]non-4-en
2-tert.-Butyloxycarbonylamino-8-azabicyclo[4.3.0]non-4-en
2-Aminomethyl-8-azabicyclo[4.3.0]non-4-en
2-Ethyloxycarbonylaminomethyl-8-azabicyclo[4.3.0]non-4-en
2-tert.-Butyloxycarbonylaminomethyl-8-azabicyclo[4.3.0]non-4-en
2-Methylamino-8-azabicyclo[4.3.0]non-4-en
2-Ethylamino-8-azabicyclo[4.3.0]non-4-en
2-Dimethylamino-8-azabicyclo[4.3.0]non-4-en
2-[(2-Hydroxyethyl)-amino]-8-azabicyclo[4.3.0]non-4-en
2-Amino-1-methyl-8-azabicyclo[4.3.0]non-4-en
2-Amino-2-methyl-8-azabicyclo[4.3.0]non-4-en
2-Amino-3-methyl-8-azabicyclo[4.3.0]non-4-en
2-Ethyloxycarbonylamino-3-methyl-8-azabicyclo-[4.3.0]non-4-en
2-tert.-Butyloxycarbonylamino-3-methyl-8-azabicyclo[4.3.0]non-4-en
2-Amino-4-methyl-8-azabicyclo[4.3.0]non-4-en
2-Amino-5-methyl-8-azabicyclo[4.3.0]non-4-en
2-Amino-6-methyl-8-azabicyclo[4.3.0]non-4-en
2-Amino-7-methyl-8-azabicyclo[4.3.0]non-4-en
2-Amino-9-methyl-8-azabicyclo[4.3.0]non-4-en
6-Amino-10-oxa-3-azatricyclo[5.2.1.0^{1,5}]dec-8-en
6-Ethyloxycarbonylamino-10-oxa-3-azatricyclo[5.2.1.0^{1,5}]dec-8-en
6-tert.-Butyloxycarbonylamino-10-oxa-3-azatricyclo[5.2.1.0^{1,5}] dec-8-en
6-Aminomethyl-10-oxa-3-azatricyclo[5.2.1.0^{1,5}]dec-8-en
6-Ethyloxycarbonylaminomethyl-10-oxa-3-azatricyclo[5.2.1.0^{1,5}] dec-8-en
6-tert.-Butyloxycarbonylaminomethyl-10-oxa-3-azatricyclo-[5.2.1.0^{1,5}]dec-8-en
6-Amino-3-azatricyclo[5.2.1.0^{1,5}]dec-8-en
6-Amino-3-azatricyclo[5.2.2.0^{1,5}]undec-8-en
sowie deren Racemate als auch deren enantiomeren- oder diastereomerenreine Verbindungen.

8. Verbindungen gemäß Ansprüchen 1 bis 4 zur Bekämpfung von Krankheiten.

9. Verbindungen gemäß Ansprüchen 1 bis 4 zur Bekämpfung von bakteriellen Infektionen.

10. Arzneimittel enthaltend Verbindungen gemäß Ansprüchen 1 bis 4.

11. Antibakterielle Mittel enthaltend Verbindungen gemäß Ansprüchen 1 bis 4.

## Claims

1. Compounds of the formula (I) 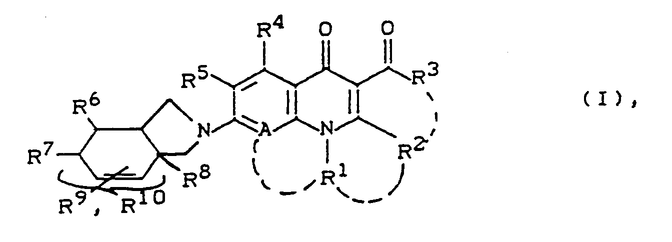 in which
R¹ represents alkyl having 1 to 4 carbon atoms, which is optionally substituted by 1-3 fluorine atoms, alkenyl having 2 to 4 carbon atoms, cycloalkyl having 3 to 6 carbon atoms, which is optionally substituted by 1 to 2 fluorine atoms, 2-hydroxyethyl, methoxy, amino, methylamino, ethylamino, dimethylamino, phenyl, which is optionally substituted by 1 or 2 fluorine atoms, 3-oxetanyl, bicyclo[1.1.1]pentyl,
R² represents hydrogen or else, together with R¹, can form a bridge, so that a 4- or 5-membered ring results,
R³ represents hydroxyl or O-R¹¹, wherein
R¹¹ represents alkyl having 1-4 C atoms or
R³, together with R², can form a bridge, so that a 5- or 6-membered ring results,
R⁴ represents hydrogen, amino, alkylamino having 1 to 4 carbon atoms, dialkylamino having 1 to 3 carbon atoms in each alkyl group, hydroxyl, alkoxy having 1 to 4 carbon atoms, mercapto, alkylthio having 1 to 4 carbon atoms, halogen, methyl, ethyl or vinyl,
R⁵ represents hydrogen, halogen or methyl,
R⁶ represents hydrogen, alkyloxycarbonyl having 1 to 4 carbon atoms, hydroxymethyl, 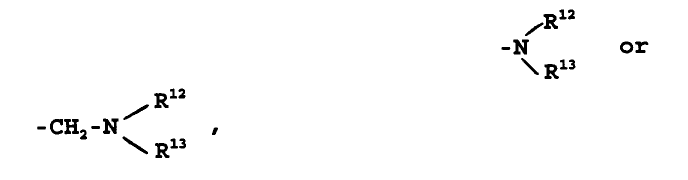 where
R¹² denotes hydrogen, alkyl having 1 to 3 carbon atoms, which is optionally substituted by hydroxyl, alkyloxycarbonyl having 1 to 4 carbon atoms in the alkyl moiety, or acyl having 1 to 3 carbon atoms, and
R¹³ denotes hydrogen or methyl,
R⁷, R⁸, R⁹ and R¹⁰ each represent hydrogen or methyl or R⁹ and R¹⁰ each represent hydrogen or methyl and R⁷, together with R⁸, can form a bridge of the structure -O-, -CH₂- or -CH₂-CH₂-, and
A represents N or C-R¹⁴, wherein
R¹⁴ represents hydrogen, halogen, methyl, which is optionally substituted by 1 to 3 fluorine atoms, ethinyl, vinyl, hydroxyl or methoxy, or else, together with R¹, can form a bridge, so that a 5-or 6-membered ring results, or else, together with R¹ and R², can form a bridge, so that two 5- or 6-membered rings result,
and their tautomers as well as their pharmaceutically utilisable hydrates and acid-addition salts.

2. Compounds of the formula (I) according to Claim 1, in which
R¹ represents cyclopropyl, 2-fluorocyclopropyl, ethyl, 2-fluoroethyl, tert-butyl, which is optionally substituted by 1, 2 or 3 fluorine atoms, or phenyl, which is optionally substituted by 1 or 2 fluorine atoms, 3-oxetanyl, bicyclo[1.1.1]pentyl,
R² represents hydrogen
or else, together with R¹, can form a bridge of the structure 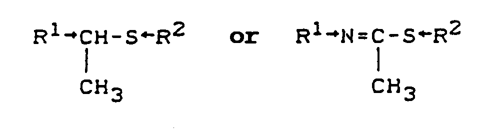
R³ represents hydroxyl or O-R¹¹, wherein
R¹¹ represents alkyl having 1-4 C atoms, or
R³, together with R², can form a bridge of the structure 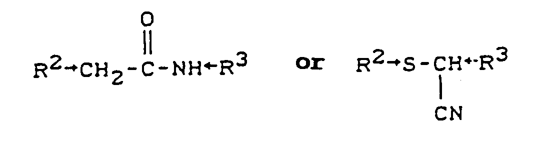
R⁴ represents hydrogen, fluorine, chlorine, amino, hydroxyl, methyl or vinyl,
R⁵ represents hydrogen, fluorine, chlorine, bromine or methyl,
R⁶ represents amino, methylamino, ethoxycarbonylamino, aminomethyl, ethoxycarbonylaminomethyl, hydroxymethyl, ethoxycarbonyl or hydrogen,
R⁷, R⁸, R⁹ and R¹⁰ each represent hydrogen or methyl or R⁹ and R¹⁰ each represent hydrogen or methyl and R⁷, together with R⁸, can form a bridge of the structure -O-, -CH₂ or -CH₂-CH₂-, and
A represents N or C-R¹⁴, wherein
R¹⁴ represents hydrogen, fluorine, chlorine, bromine, methyl, which is optionally substituted by 1 to 3 fluorine atoms, ethinyl, vinyl or methoxy, or else, together with R¹, can form a bridge of the structure 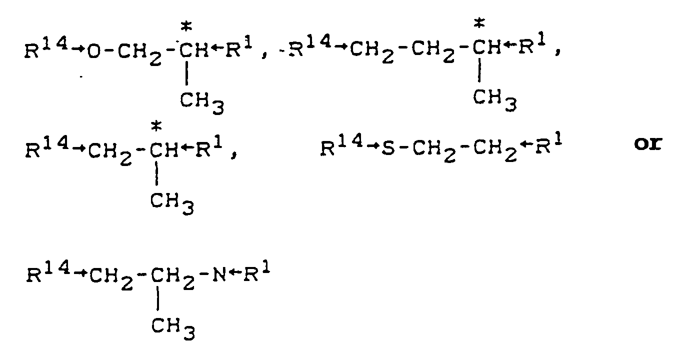 or else, together with R¹ and R², can form a bridge of the structure

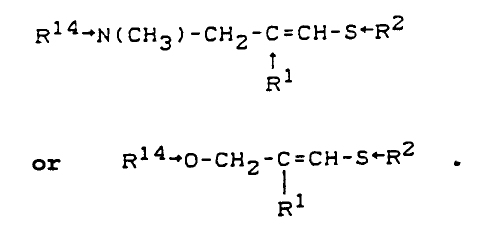

3. Compounds of the formula (I) according to Claim 1, in which
R¹ represents cyclopropyl, cis-2-fluorocyclopropyl, ethyl, tert-butyl or 2,4-difluorophenyl,
R² represents hydrogen,
R³ represents hydroxyl or ethoxy,
R⁴ represents hydrogen, fluorine or amino,
R⁵ represents hydrogen or fluorine,
R⁶ represents amino, methylamino, ethoxycarbonylamino, aminomethyl, ethoxycarbonylaminomethyl, hydroxymethyl, ethoxycarbonyl or hydrogen,
R⁷, R⁸, R⁹ and R¹⁰ each represent hydrogen or methyl, and
A represents N or C-R¹⁴, wherein
R¹⁴ represents hydrogen, fluorine or chlorine, or else, together with R¹, can form a bridge of the structure

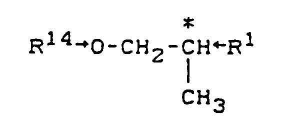

4. Alkali metal, alkaline earth metal, silver and guanidinium salts of the underlying carboxylic acids of the formula (I) according to Claims 1-3.

5. Process for preparing compounds according to Claims 1 to 3, formula (I), characterised in that a compound of the formula (II) 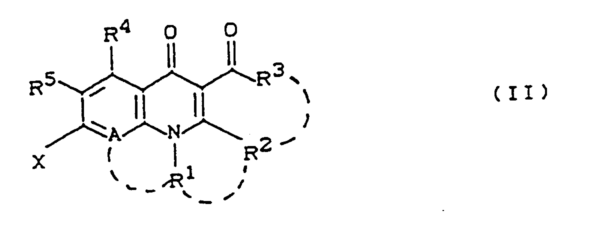 in which
R¹, R², R³, R⁴, R⁵ and A have the meaning given in Claims 1 to 3 and
X represents halogen, in particular fluorine or chlorine,
is reacted with compounds of the formula (III) 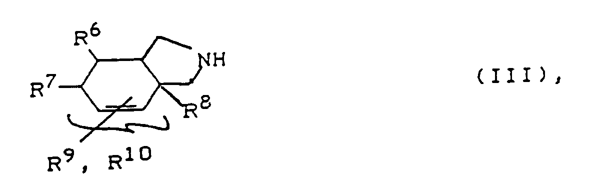 in which
R⁶, R⁷, R⁸, R⁹ and R¹⁰ have the meaning given in Claims 1 to 3,
optionally in the presence of acid binders, and protective groups which may optionally be present are eliminated by alkaline or acid hydrolysis, and the resulting compounds are optionally converted into their alkali metal, alkaline earth metal, silver or guanidinium salts.

6. Isoindoles of the general formula (III) 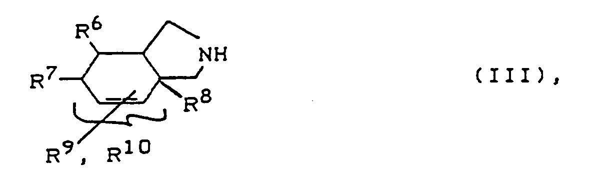 in which
R⁶ represents hydrogen, alkyloxycarbonyl having 1 to 4 carbon atoms, hydroxymethyl, 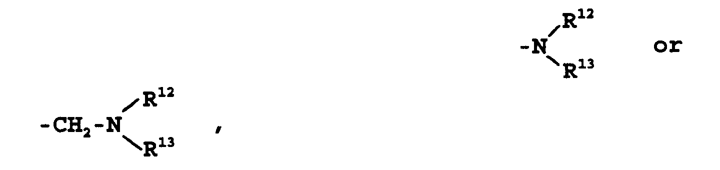 where
R¹² denotes hydrogen, alkyl having 1 to 3 carbon atoms, which is optionally substituted by hydroxyl, alkyloxycarbonyl having 1 to 4 carbon atoms in the alkyl moiety, or acyl having 1 to 3 carbon atoms, and
R¹³ denotes hydrogen or methyl,
R⁷, R⁸, R⁹ and R¹⁰ each represent hydrogen or methyl or R⁹ and R¹⁰ each represent hydrogen or methyl and R⁷, together with R⁸, can form a bridge of the structure -O-, -CH₂- or -CH₂-CH₂-, with the exception of the compound 8-azabicyclo[4.3.0]non-2-ene.

7. Isoindoles according to Claim 6 from the group comprising
Ethyl 8-azabicyclo[4.3.0]non-4-ene-2-carboxylate
2-Hydroxymethyl-8-azabicyclo[4.3.0]non-4-ene
2-Amino-8-azabicyclo[4.3.0]non-4-ene
2-Ethyloxycarbonylamino-8-azabicyclo[4.3.0]non-4-ene
2-tert-Butyloxycarbonylamino-8-azabicyclo[4.3.0]non-4-ene
2-Aminomethyl-8-azabicyclo[4.3.0]non-4-ene
2-Ethyloxycarbonylaminomethyl-8-azabicyclo[4.3.0]-non-4-ene
2-tert-Butyloxycarbonylaminomethyl-8-azabicyclo[4.3.0]non-4-ene
2-Methylamino-8-azabicyclo[4.3.0]non-4-ene
2-Ethylamino-8-azabicyclo[4.3.0]non-4-ene
2-Dimethylamino-8-azabicyclo[4.3.0]non-4-ene
2-[(2-Hydroxyethyl)-amino]-8-azabicyclo[4.3.0]non-4-ene
2-Amino-1-methyl-8-azabicyclo[4.3.0]non-4-ene
2-Amino-2-methyl-8-azabicyclo[4.3.0]non-4-ene
2-Amino-3-methyl-8-azabicyclo[4.3.0]non-4-ene
2-Ethyloxycarbonylamino-3-methyl-8-azabicyclo[4.3.0]non-4-ene
2-tert-Butyloxycarbonylamino-3-methyl-8-azabicyclo[4.3.0]non-4-ene
2-Amino-4-methyl-8-azabicyclo[4.3.0]non-4-ene
2-Amino-5-methyl-8-azabicyclo[4.3.0]non-4-ene
2-Amino-6-methyl-8-azabicyclo[4.3.0]non-4-ene
2-Amino-7-methyl-8-azabicyclo[4.3.0]non-4-ene
2-Amino-9-methyl-8-azabicyclo[4.3.0]non-4-ene
6-Amino-10-oxa-3-azatricyclo[5.2.1.0^{1,5}]dec-8-ene
6-Ethyloxycarbonylamino-10-oxa-3-azatricyclo[5.2.1.0^{1,5}]dec-8-ene
6-tert-Butyloxycarbonylamino-10-oxa-3-azatricyclo[5.2.1.0^{1,5}]dec-8-ene
6-Aminomethyl-10-oxa-3-azatricyclo[5.2.1.0^{1,5}]dec-8-ene 6-Ethyloxycarbonylaminomethyl-10-oxa-3-azatricyclo
[5.2.1.0^{1,5}]dec-8-ene
6-tert-Butyloxycarbonylaminomethyl-10-oxa-3-azatricyclo[5.2.1.0^{1,5}]dec-8-ene
6-Amino-3-azatricyclo[5.2.1.0^{1,5}]dec-8-ene
6-Amino-3-azatricyclo[5.2.2.0^{1,5}]undec-8-ene
as well as their racemates and also their enantiomerically or diastereomerically pure compounds.

8. Compounds according to Claims 1 to 4 for controlling diseases.

9. Compounds according to Claims 1 to 4 for controlling bacterial infections.

10. Medicaments containing compounds according to Claims 1 to 4.

11. Antibacterial agents containing compounds according to Claims 1 to 4.

## Revendications

1. Composés de formule (I) 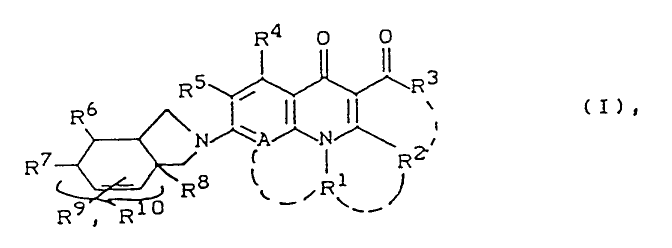 dans laquelle
R¹ représente un groupe alkyle ayant 1 à 4 atomes de carbone éventuellement substitué par 1 à 3 atomes de fluor, un groupe alcényle ayant 2 à 4 atomes de carbone, un groupe cycloalkyle de 3 à 6 atomes de carbone éventuellement substitué par 1 à 2 atomes de fluor, un groupe 2-hydroxyéthyle, méthoxy, amino, méthylamino, éthylamino, diméthylamino, un groupe phényle éventuellement substitué par 1 ou 2 atomes de fluor, un groupe 3-oxétanyle, bicyclo[1.1.1]-pentyle,
R² représente de l'hydrogène ou peut aussi former un pont conjointement avec R¹, en formant ainsi un noyau à 4 ou 5 membres,
R³ est un groupe hydroxy ou O-R¹¹, où
R¹¹ est un radical alkyle ayant 1 à 4 atomes de carbone, ou bien
R³ peut former un pont conjointement avec R², en formant ainsi un noyau pentagonal ou hexagonal,
R⁴ représente de l'hydrogène, un groupe amino, un groupe alkylamino ayant 1 à 4 atomes de carbone, dialkylamino ayant 1 à 3 atomes de carbone par groupe alkyle, un groupe hydroxy, un groupe alkoxy ayant 1 à 4 atomes de carbone, un groupe mercapto, un groupe alkylthio ayant 1 à 4 atomes de carbone, un halogène, un groupe méthyle, éthyle ou vinyle,
R⁵ représente de l'hydrogène, un halogène ou un groupe méthyle,
R⁶ est de l'hydrogène, un groupe alkyloxycarbonyle ayant 1 à 4 atomes de carbone, un groupe hydroxyméthyle, 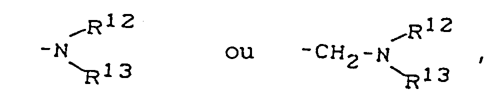 dans lequel
R¹² représente de l'hydrogène, un groupe alkyle ayant 1 à 3 atomes de carbone éventuellement substitué par un radical hydroxy, un groupe alkoxycarbonyle ayant 1 à 4 atomes de carbone dans la partie alkyle ou un groupe acyle ayant 1 à 3 atomes de carbone et
R¹³ est de l'hydrogène ou un groupe méthyle,
R⁷, R⁸, R⁹ et R¹⁰ représentent de l'hydrogène ou un groupe méthyle ou bien R⁹ et R¹⁰ représentent de l'hydrogène ou un groupe méthyle et R⁷ peut former conjointement avec R⁸ un pont de structure -O-, -CH₂- ou -CH₂-CH₂- et
A représente N ou un groupe C-R¹⁴, dans lequel
R¹⁴ représente de l'hydrogène, un halogène, un groupe méthyle éventuellement substitué par 1 à 3 atomes de fluor, un groupe éthynyle, vinyle, hydroxy ou méthoxy, ou bien il peut aussi former un pont conjointement avec R¹, en formant ainsi un noyau pentagonal ou hexagonal, ou bien il peut aussi former un pont conjointement avec R¹ et R², en formant ainsi deux ponts pentagonaux ou hexagonaux,
et leurs tautomères ainsi que leurs hydrates et leurs sels d'addition d'acides pharmaceutiquement utilisables.

2. Composés de formule (I) suivant la revendication 1, dans lesquels
R¹ est un groupe cyclopropyle, 2-fluorocyclopropyle, éthyle, 2-fluoréthyle, un groupe tertio-butyle éventuellement substitué par 1, 2 ou 3 atomes de fluor ou un groupe phényle éventuellement substitué par 1 ou 2 atomes de fluor, un groupe 3-oxétanyle, bicyclo[1.1.1]pentyle,
R² représente de l'hydrogène ou peut aussi former conjointement avec R¹ un pont de structure 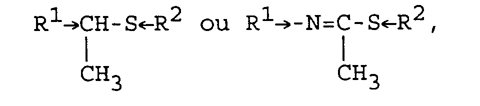
R³ est un groupe hydroxy ou un groupe O-R¹¹ dans lequel
R¹¹ est un groupe alkyle ayant 1 à 4 atomes de carbone, ou bien
R³ peut former conjointement avec R² un pont de structure 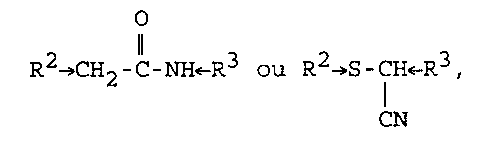
R⁴ représente de l'hydrogène, du fluor, du chlore, un groupe amino, hydroxy, méthyle ou vinyle,
R⁵ est de l'hydrogène, du fluor, du chlore, du brome ou un groupe méthyle,
R⁶ est un groupe amino, méthylamino, éthoxycarbonylamino, aminométhyle, éthoxycarbonylaminométhyle, hydroxyméthyle, éthoxycarbonyle ou de l'hydrogène,
R⁷, R⁸, R⁹ et R¹⁰ représentent de l'hydrogène ou un groupe méthyle ou bien R⁹ et R¹⁰ représentent de l'hydrogène ou un groupe méthyle et R⁷ peut former conjointement avec R⁸ un pont de structure -O-, -CH₂- ou -CH₂-CH₂- et
A représente N ou un groupe C-R¹⁴ dans lequel
R¹⁴ représente de l'hydrogène, du fluor, du chlore, du brome, un groupe méthyle éventuellement substitué par 1 à 3 atomes de fluor, un groupe éthynyle, vinyle, ou méthoxy, ou peut aussi former conjointement avec R¹ un pont de structure 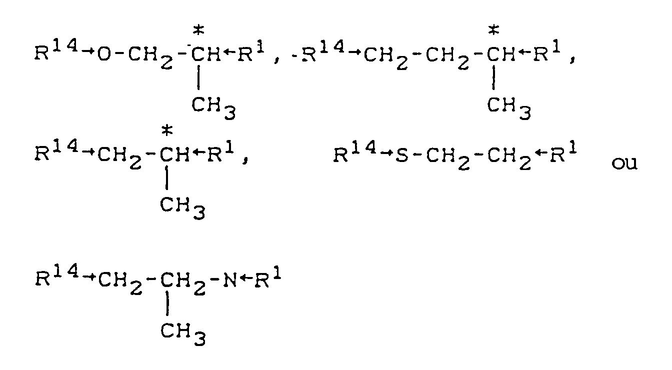 ou peut aussi former conjointement avec R¹ et R² un pont de structure

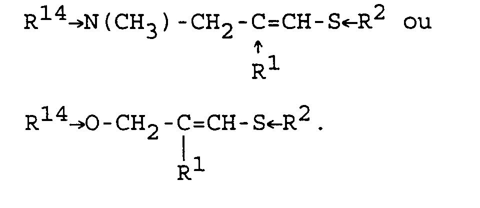

3. Composés de formule (I) suivant la revendication 1, dans lesquels
R¹ est un groupe cyclopropyle, cis-2-fluorocyclopropyle, éthyle, tertio-butyle ou 2,4-difluorophényle,
R² est de l'hydrogène,
R³ est un groupe hydroxy ou éthoxy,
R⁴ est de l'hydrogène, du fluor ou un groupe amino,
R⁵ est de l'hydrogène ou du fluor,
R⁶ est un groupe amino, méthylamino, éthoxycarbonylamino, aminométhyle, éthoxycarbonylaminométhyle, hydroxyméthyle, éthoxycarbonyle ou de l'hydrogène,
R⁷, R⁸, R⁹ et R¹⁰ représentent de l'hydrogène ou un groupe méthyle et
A représente N ou un groupe C-R¹⁴ dans lequel
R¹⁴ est de l'hydrogène, du fluor ou du chlore ou peut aussi former conjointement avec R¹ un pont de structure

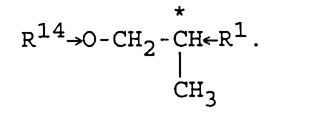

4. Sels de métaux alcalins, de métaux alcalino-terreux, d'argent et de guanidinium des acides carboxyliques de base de formule (I) suivant les revendications 1 à 3.

5. Procédé de production de composés suivant les revendications 1 à 3, de formule (I), caractérisé en ce qu'on fait réagir un composé de formule (II) 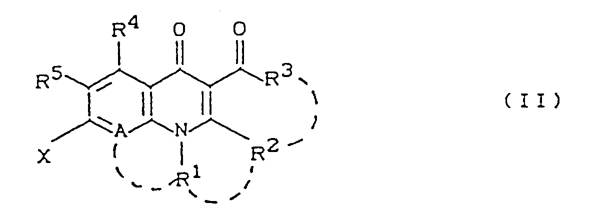 dans laquelle
R¹, R², R³, R⁴, R⁵ et A ont la définition indiquée dans les revendications 1 à 3 et
X représente un halogène, en particulier le fluor ou le chlore,
avec des composés de formule (III) 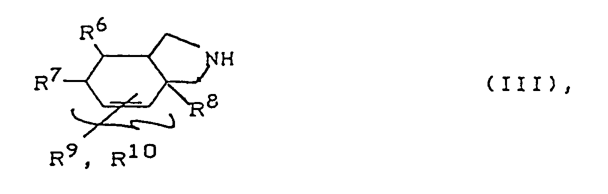 dans laquelle
R⁶, R⁷, R⁸, R⁹ et R¹⁰ ont la définition indiquée dans les revendications 1 à 3,
le cas échéant en présence d'accepteurs d'acides et on élimine les groupes protecteurs éventuellement présents par hydrolyse alcaline ou acide et on transforme éventuellement les composés obtenus en leurs sels de métaux alcalins, de métaux alcalino-terreux, d'argent et de guanidinium.

6. Iso-indoles de formule générale (III) 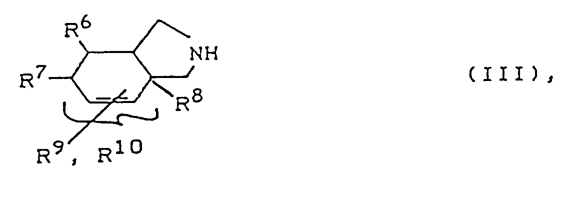 dans laquelle
R⁶ représente de l'hydrogène, un groupe alkyloxycarbonyle ayant 1 à 4 atomes de carbone, un groupe hydroxy méthyle, 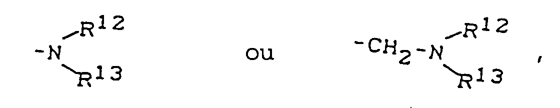 où
R¹² représente de l'hydrogène, un groupe alkyle ayant 1 à 3 atomes de carbone éventuellement substitué par un radical hydroxy, un groupe alkyloxycarbonyle ayant 1 à 4 atomes de carbone dans la partie alkyle ou un groupe acyle ayant 1 à 3 atomes de carbone et
R¹³ est de l'hydrogène ou un groupe méthyle,
R⁷, R⁸, R⁹ et R¹⁰ représentent de l'hydrogène ou un groupe méthyle ou bien R⁹ et R¹⁰ représentent de l'hydrogène ou un groupe méthyle et R⁷ peut former conjointement avec R⁸ un pont de structure -O-, -CH₂- ou -CH₂-CH₂-, à l'exception du composé 8-azabicyclo[4.3.0]non-2-ène.

7. Iso-indoles suivant la revendication 6, du groupe consistant en :
l'ester éthylique de l'acide 8-azabicyclo[4.3.0]non-4-ène-2-carboxylique,
le 2-hydroxyméthyl-8-azabicyclo[4.3.0]non-4-ène
le 2-amino-8-azabicyclo[4.3.0]non-4-ène
le 2-éthyloxycarbonylamino-8-azabicyclo[4.3.0]non-4-ène
le 2-tertio-butyloxycarbonylamino-8-azabicyclo[4.3.0]non-4-ène
le 2-aminométhyl-8-azabicyclo[4.3.0]non-4-ène
le 2-éthyloxycarbonylaminométhyl-8-azabicyclo[4.3.0]non-4-ène
le 2-tertio-butyloxycarbonylaminométhyl-8-azabicyclo[4.3.0]non-4-ène
le 2-méthylamino-8-azabicyclo[4.3.0]non-4-ène
le 2-éthylamino-8-azabicyclo[4.3.0]non-4-ène
le 2-diméthylamino-8-azabicyclo[4.3.0]non-4-ène
le 2-[(2-hydroxyéthyl)-amino]-8-azabicyclo[4.3.0]non-4-ène
le 2-amino-1-méthyl-8-azabicyclo[4.3.0]non-4-ène
le 2-amino-2-méthyl-8-azabicyclo[4.3.0]non-4-ène
le 2-amino-3-méthyl-8-azabicyclo[4.3.0]non-4-ène
le 2-éthyloxycarbonylamino-3-méthyl-8-azabicyclo[4.3.0]non-4-ène
le 2-tertio-butyloxycarbonylamino-3-méthyl-8-azabicyclo[4.3.0]non-4-ène
le 2-amino-4-méthyl-8-azabicyclo[4.3.0]non-4-ène
le 2-amino-5-méthyl-8-azabicyclo[4.3.0]non-4-ène
le 2-amino-6-méthyl-8-azabicyclo[4.3.0]non-4-ène
le 2-amino-7-méthyl-8-azabicyclo[4.3.0]non-4-ène
le 2-amino-9-méthyl-8-azabicyclo[4.3.0]non-4-ène
le 6-amino-10-oxa-3-azatricyclo[5.2.1.0^{1,5}]déc-8-ène
le 6-éthyloxycarbonylamino-10-oxa-3-azatricyclo[5.2.1.0^{1,5}]déc-8-ène
le 6-tertio-butyloxycarbonylamino-10-oxa-3-azatricyclo[5.2.1.0^{1,5}]déc-8-ène
le 6-aminométhyl-10-oxa-3-azatricyclo[5.2.1.0^{1,5}]déc-8-ène
le 6-éthyloxycarbonylaminométhyl-10-oxa-3-azatricyclo[5.2.1.0^{1,5}]déc-8-ène
le 6-tertio-butyloxycarbonylaminométhyl-10-oxa-3-azatricyclo[5.2.1.0^{1,5}]déc-8-ène
le 6-amino-3-azatricyclo[5.2.1.0^{1,5}]déc-8-ène
le 6-amino-3-azatricyclo[5.2.2.0^{1,5}]undéc-8-ène ainsi que leurs racémates de même que leurs composés de pureté énantiomérique ou diastéréo-isomérique.

8. Composés suivant les revendications 1 à 4, destinés à combattre des maladies.

9. Composés suivant les revendications 1 à 4, destinés à combattre des infections bactériennes.

10. Médicament contenant des composés suivant les revendications 1 à 4.

11. Agents antibactériens contenant des composés suivant les revendications 1 à 4.
